# EUROPEAN PATENT SPECIFICATION

(11) **EP 1 603 936 B1**
(45) Date of publication and mention of the grant of the patent: **11.04.2012**
(21) Application number: 04775814.9
(22) Date of filing: 27.02.2004
(51) Int. Cl.: C07K 1/00, A61K 39/39

(54) **USE OF LECTINS TO PROMOTE OLIGOMERIZATION OF GLYCOPROTEINS AND ANTIGENIC MOLECULES**
VERWENDUNG VON LEKTINEN ZUR FÖRDERUNG DER OLIGOMERISIERUNG VON GLYCOPROTEINEN UND ANTIGENEN MOLEKÜLEN
UTILISATION DE LECTINES EN VUE DE PROMOUVOIR L'OLIGOMERISATION DES GLYCOPROTEINES ET DES MOLECULES ANTIGENIQUES

(30) Priority: 28.02.2003 US 450721 P
(43) Date of publication of application: 14.12.2005
(73) Proprietor: Agenus Inc., Lexington MA 02421 (US)
(72) Inventor: ZABRECKY, James, R., Waltham, MA 02453 (US); MONKS, Stephen, A., Somerville, MA 02144 (US)
(74) Representative: Thomas, Philip John Duval
(86) International application number: PCT/US2004/006047
(87) International publication number: WO 2005/020936

(56) References cited:
- WO-A-01/34193
- MONKS STEPHEN A ET AL: "Potentiating role of Concanavalin A in heat shock protein, gp96, antigen cross-presentation and tumor rejection activity." PROCEEDINGS OF THE AMERICAN ASSOCIATION FOR CANCER RESEARCH ANNUAL MEETING, vol. 44, July 2003 (2003-07), page 560, XP008079931 & 94TH ANNUAL MEETING OF THE AMERICAN ASSOCIATION FOR CANCER RESEARCH; WASHINGTON, DC, USA; JULY 11-14, 2003 ISSN: 0197-016X
- ARNOLD-SCHILD: 'One-Step Single-Chain Fv Recombinant Antibody-based Purification of gp96 for Vaccine Development' CANCER RESEARCH vol. 60, no. 15, 01 August 2000, pages 4175 - 4178, XP002175252
- LIN H.Y. ET AL: 'The 170-kDa glucose-regulated stress protein is an endoplasmic reticulum protein that binds immunoglobulin.' MOLECULAR BIOLOGY OF THE CELL NOV 1993 LNKD- PUBMED:8305733 vol. 4, no. 11, November 1993, pages 1109 - 1119 ISSN: 1059-1524
- DENNING G.M. ET AL: 'Calreticulin biosynthesis and processing in human myeloid cells: demonstration of signal peptide cleavage and N-glycosylation.' BLOOD 1 JUL 1997 LNKD- PUBMED:9207473 vol. 90, no. 1, 01 July 1997, pages 372 - 381 ISSN: 0006-4971
- BASU S. ET AL: 'Calreticulin, a peptide-binding chaperone of the endoplasmic reticulum, elicits tumor- and peptide-specific immunity.' THE JOURNAL OF EXPERIMENTAL MEDICINE 1 MAR 1999 LNKD- PUBMED:10049943 vol. 189, no. 5, 01 March 1999, pages 797 - 802 ISSN: 0022-1007
- WANG X.Y. ET AL: 'Characterization of heat shock protein 110 and glucose-regulated protein 170 as cancer vaccines and the effect of fever-range hyperthermia on vaccine activity.' JOURNAL OF IMMUNOLOGY (BALTIMORE, MD. : 1950) 1 JAN 2001 LNKD- PUBMED:11123328 vol. 166, no. 1, 01 January 2001, pages 490 - 497 ISSN: 0022-1767
- WANG XIANG-YANG ET AL: "Development of cancer vaccines using autologous and recombinant high molecular weight stress proteins.", METHODS (SAN DIEGO, CALIF.) JAN 2004 LNKD- PUBMED:14624871, vol. 32, no. 1, January 2004 (2004-01), pages 13-20, ISSN: 1046-2023
- SURIANO ROBERT ET AL: "Differences in glycosylation patterns of heat shock protein, gp96: implications for prostate cancer prevention.", CANCER RESEARCH 15 JUL 2005 LNKD- PUBMED:16024652, vol. 65, no. 14, 15 July 2005 (2005-07-15) , pages 6466-6475, ISSN: 0008-5472
- INTERNET CITATION, [Online] Retrieved from the Internet: <URL:http://www.uniprot.org/uniprot/Q9JKR6>

## Description

### 1. INTRODUCTION

The present invention relates to the areas of biologic therapy, immunotherapy and stress protein-mediated immune modulation. More particularly, the present invention relates to compositions and methods of using molecular complexes comprising lectin or lectin-like molecules associated with immunologically and/or biologically active molecules to increase the prophylactic and/or therapeutic effects of the immunologically and/or biologically active molecules for the prevention or treatment of diseases, particularly for the prevention or treatment of cancer or infectious diseases.

### 2. BACKGROUND OF THE INVENTION

Citation or discussion of a reference herein shall not be construed as an admission that such is prior art to the present invention.

### 2.1. Immune Responses and Antigen Presentation

An organism's immune system reacts with two types of responses to pathogens or other harmful agents - humoral response and cell-mediated response *(see* Alberts, B. et al., 1994, Molecular Biology of the Cell, 1195-96). When resting B cells are activated by antigen to proliferate and mature into antibody-secreting cells, they produce and secrete antibodies with a unique antigen-binding site. This antibody-secreting reaction is known as the humoral response. On the other hand, the diverse responses of T cells are collectively called cell-mediated immune reactions. There are two main classes of T cells - cytotoxic T cells and helper T cells. Cytotoxic T cells directly kill cells that are infected with a virus or some other intracellular microorganism. Helper T cells, by contrast, help stimulate the responses of other cells: they help activate macrophages, dendritic cells and B cells, for example (*See* Alberts, B. *et al., supra,* at 1228). Both cytotoxic T cells and helper T cells recognize antigen in the form of peptide fragments that are generated by the degradation of foreign protein antigens inside the target cell, and both, therefore, depend on major histocompatibility complex (MHC) molecules, which bind these peptide fragments, carry them to the cell surface, and present them to the T cells. MHC molecules are typically found in abundance on antigen-presenting cells (APCs).

Antigen-presenting cells (APCs), such as macrophages and dendritic cells, are key components of innate and adaptive immune responses. Antigens are generally 'presented' to T cells or B cells on the surfaces of other cells, the APCs. APCs can trap lymph- and blood-borne antigens and, after internalization and degradation, present antigenic peptide fragments, bound to cell-surface molecules of the major histocompatibility complex (MHC), to T cells. APCs may then activate T cells (cell-mediated response) to clonal expansion, and these daughter cells may either develop into cytotoxic T cells or helper T cells, which in turn activate B (humoral response) cells with the same MHC-bound antigen to clonal expansion and specific antibody production (*see* Alberts, B. *et al., supra,* at 1238-45).

Two types of antigen-processing mechanisms have been recognized. The first type involves uptake of proteins through endocytosis by APCs, antigen fragmentation within vesicles, association with class II MHC molecules and expression on the cell surface. This complex is recognized by helper T cells expressing CD4. The other is employed for proteins, such as viral antigens, that are synthesized within the cell and appears to involve protein fragmentation in the cytoplasm. Peptides produced in this manner become associated with class I MHC molecules and are recognized by cytotoxic T cells expressing CD8 *(see* Alberts, B. *et al., supra.* at 1233-34).

Stimulation of T cells involves a number of accessory molecules expressed by both T cells and APCs. Co-stimulatory molecules are those accessory molecules that promote the growth and activation of the T cell. Upon stimulation, co-stimulatory molecules induce release of cytokines, such as interleukin 1 (IL-1) or interleukin 2 (IL-2), interferon, etc., which promote T cell growth and expression of surface receptors *(see* Paul, 1989, Fundamental Immunology, 109-10).

Normally, APCs are quiescent and require activation for their function. The identity of signals which activate APCs is a crucial and unresolved question *(see* Banchereau, et al., 1998, Nature, 392:245-252; Medzhitov, et al., 1998, Curr Opin Immunol., 10:12-15).

### 2.2. Heat Shock Proteins

Heat shock proteins (HSPs), also referred to as stress proteins, were first identified as proteins synthesized by cells in response to heat shock. Approximately ten families ofHSPs are known, and each family consists of from one to five closely related proteins. Srivastava, 2002, Annu. Rev. Immunol. 20:395-425. Many members of these families were found subsequently to be induced in response to other stressful stimuli including nutrient deprivation, metabolic disruption, oxygen radicals, and infection with intracellular pathogens *(see* Welch, May 1993, Scientific American, 56-64; Young, 1990, Annu. Rev. Immunol., 8:401-420; Craig, 1993, Science, 260:1902-1903; Gething et al., 1992, Nature, 355:33-45; and Lindquist et al., 1988, Annu. Rev. Genetics, 22:631-677).

Heat shock proteins are expressed in all cells in all forms of life and in a variety of intracellular locations, *i.e.*, they are expressed in the cytosol of prokaryotes and in the cytosol, nuclei, endoplasmic reticulum (ER), mitochondria, and chloroplasts of eukaryotes. Srivastava, 2002, Annu. Rev. Immunol. 20:395-425. The HSPs also constitute the single most abundant group of proteins inside cells. They are expressed in vast quantities under normal non-heat shocked conditions, and their expression can be powerfully induced to much higher levels as a result of heat shock or other forms of stress.

Heat shock proteins are among the most highly conserved proteins in existence. For example, DnaK, the Hsp70 from *E. coli* has about 50% amino acid sequence identity with Hsp70 proteins from excoriates (Bardwell et al., 1984, Proc. Natl. Acad. Sci., 81:848-852). The Hsp60 and Hsp90 families also show similarly high levels of intra-family conservation (Hickey et al., 1989, Mol. Cell. Biol., 9:2615-2626; Jindal, 1989, Mol. Cell. Biol., 9:2279-2283). In addition, it has been discovered that the Hsp60, Hsp70 and Hsp90 families are composed of proteins that are related to the stress proteins in sequence, for example, having greater than 35% amino acid identity, but whose expression levels are not altered by stress.

Studies on the cellular response to heat shock and other physiological stresses revealed that the HSPs possess functions such as folding and unfolding of proteins, degradation of proteins, assembly of multi-subunit complexes, thermotolerance, buffering of expression of mutations, and others. Srivastava, 2002, Annu. Rev. Immunol. 20:395-425. HSPs accomplish different kinds of chaperoning functions. For example, members of the Hsp70 family, located in the cell cytoplasm, nucleus, mitochondria, or endoplasmic reticulum (Lindquist et al., 1988, Ann. Rev. Genetics, 22:631-677), are involved in the presentation of antigens to the cells of the immune system, and are also involved in the transfer, folding and assembly of proteins in normal cells. HSPs are capable of binding proteins or peptides, and releasing the bound proteins or peptides in the presence of adenosine triphosphate (ATP) or low pH.

### 2.3. Immunogenicity of HSP-Peptide Complexes

Srivastava *et al.* demonstrated immune response to methylcholanthrene-induced sarcomas of inbred mice (1988, Immunol. Today, 9:78-83). In these studies, it was found that the molecules responsible for the individually distinct immunogenicity of these tumors were glycoproteins of 96kDa (gp96) and intracellular proteins of 84 to 86kDa (Srivastava et al., 1986, Proc. Natl. Acad. Sci. USA, 83:3407-3411; Ullrich et al., 1986, Proc. Natl. Acad. Sci. USA, 83:3121-3125). Immunization of mice with gp96 or p84/86 isolated from a particular tumor rendered the mice immune to that particular tumor, but not to antigenically distinct tumors. Isolation and characterization of genes encoding gp96 and p84/86 revealed significant homology between them, and showed that gp96 and p84/86 were, respectively, the endoplasmic reticular and cytosolic counterparts of the same heat shock proteins (Srivastava et al., 1988, Immunogenetics, 28:205-207; Srivastava et al., 1991, Curr. Top. Microbiol. Immunol., 167:109-123). Further, Hsp70 was shown to elicit immunity to the tumor from which it was isolated but not to antigenically distinct tumors. However, Hsp70 depleted of peptides was found to lose its immunogenic activity (Udono and Srivastava, 1993, J. Exp. Med., 178:1391-1396). These observations suggested that the heat shock proteins are not immunogenic *per se,* but form noncovalent complexes with antigenic peptides, and the complexes can elicit specific immunity to the antigenic peptides (Srivastava, 1993, Adv. Cancer Res., 62:153-177; Udono et al., 1994, J. Immunol., 152:5398-5403; Suto et al., 1995, Science, 269:1585-1588).

The heat shock protein gp96 chaperones a wide array of peptides, depending upon the source from which gp96 is isolated (for review, *see* Srivastava et al., 1998, Immunity, 8:657-665). Tumor-derived gp96 carries tumor-antigenic peptides (Ishii et al., 1999, J. Immunology, 162:1303-1309); gp96 preparations from virus-infected cells carry viral epitopes (Suto and Srivastava, 1995, Science, 269:1585-1588; Nieland et al., 1996, Proc. Natl. Acad. Sci. USA, 95:1800-1805), and gp96 preparations from cells transfected with model antigens such as ovalbumin or β-galactosidase are associated with the corresponding epitopes (Arnold et al., 1995, J. Exp. Med.,182:885-889; Breloer et al., 1998, Eur. J. Immunol., 28:1016-1021). The association of gp96 with peptides occurs *in vivo* (Menoret and Srivastava, 1999, Biochem. Biophys. Research Commun., 262:813-818). Gp96-peptide complexes, whether isolated from cells (Tamura et al., 1997, Science, 278:117-120), or reconstituted *in vitro* (Blachere et al., 1997, J. Exp. Med., 186:1183-1406) are excellent immunogens and have been used extensively to elicit CD8+ T cell responses specific for the gp96-chaperoned antigenic peptides.

The capacity of gp96-peptide complexes to elicit an immune response is dependent upon the transfer of the peptide to MHC class I molecules of antigen-presenting cells (Suto and Srivastava, 1995, *supra*)*.* Endogenously synthesized antigens chaperoned by gp96 in the endoplasmic reticulum (ER) can prime antigen-specific CD8+ T cells (or MHC I-restricted CTLs) *in vivo;* this priming of CD8+ T cells requires macrophages. Although exogenous antigens are typically routed through the MHC II-presentation pathway and elicit CD4+ responses, exogenously introduced gp96-peptide complexes can elicit CD8+ T cell response. Suto and Srivastava, 1995, *supra;* Blachere et al., 1997, J. Exp. Med., 186:1315-22.

In view of the extremely small quantity of gp96-chaperoned antigenic peptides required for immunization (Blachere *et al.,* 1997, *supra*), and the strict dependence of immunogenicity of gp96-peptide complexes on functional antigen presenting cells (APCs) (Udono et al., 1994, Proc. Natl. Acad. Sci. U.S.A., 91:3077-3081), APCs had been proposed to possess receptors for gp96 (Srivastava et al., 1994, Immunogenetics, 39:93-98). The proposal was confirmed when it was shown that α2 macroglobulin (α2M) receptor CD91 binds to gp96 and α2M as well as antibodies to CD91 completely inhibit the representation of gp96-chaperoned peptides by APCs. Binder et al., 2000, Nat. Immunol. 1(2):151-55; Srivastava, 2002, Annu. Rev. Immunol. 20:395-425. Later, it was demonstrated that CD91 acted as the receptor not only for gp96 but also for hsp90, hsp70, and calreticulin. Basu et al., 2001, Immunity 14(3):301-13.

It has been demonstrated that the HSP-chaperoned peptides can be re-presented by the MHC II molecules of the APCs, in addition to re-presented by MHC I molecules. Srivastava, 2002, Annu. Rev. Immunol. 20:395-425. The re-presentation by MHC II molecules also occurs through the CD91 receptor. Thus, it has been suggested that once an HSP-peptide complex is taken up through CD91, it may enter one or more of several trafficking and processing pathways. Srivastava, *supra.*

HSPs have also been implicated in innate immunity. Exposure of APCs to gp96 (or other HSPs) leads to secretion of low levels of TNFα by the APCs, regardless of whether or not the gp96 molecules are associated with antigenic peptides. Suto and Srivastava, 1995, Science 269:1585-88. Later it was shown that the interaction of HSPs, *e.g.,* gp96, hsp90, hsp 70 and hsp60, with APCs can lead to a series of events associated with innate immunity, such as secretion of inflammatory cytokines TNFα, IL-1β, IL-12, and GM-CSF by macrophages; secretion of chemokines, *e.g.,* MCP-1, MIP-2, and RANTES, by macrophages; induction of inducible nitric oxide synthase and production of nitric oxide by macrophages and DCs; maturation of DCs as measured by enhanced expression of MHC II, B7-2, and CD40 molecules on CD11c+ cells; migration of vast numbers of DCs (presumably Langerhans cells) from site of injection of gp96 to the draining lymph nodes; and translocation of NFκB into the nuclei of macrophages and DCs. Srivastava, 2002, Annu. Rev. Immunol. 20:395-425. There is little evidence that CD91 is the receptor involved in these phenomena, and it has been suggested that other receptors are involved. Ohashi et al., 2000, J. Immunol. 164(2):558-61; Panjwani et al., 2000, Cell Stress Chaperones 5:391; Srivastava, 2002, Annu. Rev. Immunol. 20:395-425.

Noncovalent complexes of HSPs and peptide, purified from cancer cells, can be used for the treatment and prevention of cancer and have been described in PCT publications WO 96/10411, dated April 11,1996, and WO 97/10001, dated March 20, 1997 (U.S. Patent No. 5,750,119 issued April 12, 1998, and U.S. Patent No. 5,837,251 issued November 17, 1998, respectively ). The isolation and purification of stress protein-antigen complexes has been described, for example, from pathogen-infected cells, and used for the treatment and prevention of infection caused by the pathogen, such as viruses, and other intracellular pathogens, including bacteria, protozoa, fungi and parasites (see, for example, PCT Publication WO 95/24923, dated September 21, 1995). Immunogenic stress protein-antigen complexes can also be prepared by *in vitro* complexing of stress protein and antigenic peptides, and the uses of such complexes for the treatment and prevention of cancer and infectious diseases has been described in PCT publication WO 97/10000, dated March 20, 1997 (U.S. Patent No. 6,030,618 issued February 29, 2000).
US-A-6 143 299 discloses complexes of heat shock protein gp96 noncovalently bound to an antigenic molecule and the use of this complex for inducing an immune response against the antigenic molecule and in this way treating or preventing an infectious disease. The use of stress protein- antigen complexes for sensitizing antigen presenting cells *in vitro* for use in adoptive immunotherapy is described in PCT publication WO 97/10002, dated March 20, 1997 (see also U.S. Patent No. 5,985,270 issued November 16, 1999).

The identification and characterization of specific molecules or methods that may increase the immunogenicity of HSP-mediated antigen presentation of peptides could provide useful reagents and techniques for eliciting specific immunity by HSP and HSP-peptide complexes, and for developing novel diagnostic and therapeutic methods.

### 2.4. Lectins

Lectins are a group of proteins found in plants, animals, fungi, algae, and bacteria that share the property of binding to specific carbohydrate groups (see Sharon et al., 1972, Science, 177:949). They are a structurally diverse class of proteins, and their only common features are the ability to bind carbohydrates specifically and reversibly, and to agglutinate cells by forming cross links between the oligosaccharide groups on cell surfaces. Sharon, 1993, Trends Biochem Sci 18(6):221-6. Lectins are widely used for diagnosis and experimental purposes, *e.g.* to identify mutant cells in cell cultures, to determine blood groups by triggering agglutination of red blood cells, or in mapping the surface of cell membranes. Lectins are also used for protein purification because of their ability to bind carbohydrates specifically and reversibly.

Some lectins can be grouped together into distinct families, such as those of the legumes or the cereals that are structurally similar, or the c-type (Ca²⁺-dependent) animal lectins that contain homologous carbohydrate recognition domains. Sharon, *supra.* Legumes lectins are strikingly similar in their primary, secondary and tertiary structures. Srinivas et al., 2001, Biochim. Biophy. Acta 1527:102-111. For all the legumes lectins known so far, the tertiary structure is made up of two anti-parallel β sheets, a six-stranded flat "back" and a seven-stranded curved "front" β sheet. Srinivas *et al., supra.* These sheets are in turn connected to form a so called "jelly roll" motif. Despite their similarities at the primary, secondary and tertiary structural levels, legumes lectins show considerable differences in their quaternary associations and modes of monomer organizations in the dimeric/tetrameric assemblage. Srinivas *et al., supra.*

Concanavalin A (Con A) from Jack bean was the first lectin of the legumes family whose structure became known. Con A consists of 237 amino acids and has two metal binding sites. Becker et al., 1975, J. Biol. Chem. 250:1513. At pH 4.5-5.6, Con A exists as a single dimer. McKenzie et al., 1972, Biochim. Biophys. Acta, 263:283. Above pH 7, it is predominantly tetrameric. Wang et al., 1975, J. Biol. Chem. 250: 1490. Con A reacts with non-reducing D-glucose and D-mannose. Smith et al., 1967, Arch. Biochem. Biophys., 121:88. In such reactions, α-methyl-D-glucopyranoside may act as a competitive inhibitor. Smith *et al., supra.*

### 3. SUMMARY OF THE INVENTION

The present invention provides one or more noncovalent complexes, each complex comprising a heat shock protein, an antigenic molecule, and a lectin, wherein said heat shock protein is glycosylated, and wherein the amount of lectin present in said complexes relative to the amount of heat shock protein is greater than or equal to 50 nanograms lectin per microgram of heat shock protein or less than or equal to 5 nanogram lectin per microgram of heat shock-protein. As used herein, the term "Antigenic Molecule" refers to a molecule that displays one or more antigenic determinants against which an immune response is desired in a subject (e.g., for therapeutic purposes). Non-limiting examples of Antigenic Molecules are given in Section 5.2.
In a preferred embodiment, the immunologically and/or biologically active molecule is a therapeutic. Methods of making such compositions and the compositions for use in prevention or treatment of a disease (*e.g.*, cancer, an infectious disease, anemia, growth hormone deficiency disease, enzyme deficiency disease, a condition of immune suppression) and for use in stimulating an immune response in a subject in need thereof are also provided. While not bound by any theory, the invention is based, in part, on the applicants' discovery that lectin promotes oligomerization of glycoproteins (including glycopeptides and glycopolypeptides) or an immunologically and/or biologically active complex comprising one or more glycoproteins, and that the oligomerized complex shows increased biological activity (both *in vitro* and *in vivo*) over that of the un-oligomerized molecules.

Disclosed herein are one or more noncovalent molecular complexes or a composition comprising one or more noncovalent molecular complexes, wherein each molecular complex comprises a lectin associated with an immunologically and/or biologically active glycoprotein, and wherein the amount of lectin present in the composition relative to the amount of glycoprotein is equal to or greater than 1fg, 100fg, 500fg, 1pg, 100pg, 500pg, 1ng, 2ng, 5ng, 10ng, 20ng, 30ng, 40ng, 50ng, 75ng, 100ng, or 200ng per microgram of glycoprotein. In a specific embodiment, the amount of lectin present in the composition relative to the amount of glycoprotein is 40ng to 1000ng, 50ng to 1000ng, 50ng to 500ng, 50ng to 250ng, or 100ng to 500ng lectin per microgram of glycoprotein. In another embodiment, lectin is in molar excess with respect to the glycoprotein. In one embodiment, the glycoprotein is an Antigenic Molecule. In a specific embodiment, it is disclosed herein that the molecular complex comprises a lectin, a glycoprotein that is an Antigenic Molecule, and another molecule, such as a heat shock protein ("HSP"), that may or may not be glycosylated. In another embodiment, the glycoprotein is not an Antigenic Molecule. In a specific embodiment, the glycoprotein is a glycosylated heat shock protein. In yet another embodiment, the molecular complex comprises a lectin, a glycoprotein that is not an Antigenic Molecule, and an Antigenic Molecule (which may or may not be a glycoprotein). In a specific embodiment, the Antigenic Molecule is a protein (including peptide and polypeptide) that displays the antigenicity of an antigen of a type of cancer or of an agent of an infectious disease.

The present invention provides one or more molecular complexes or a composition comprising one or more molecular complexes, each complex comprising a heat shock protein, an Antigenic Molecule, and a lectin, wherein the heat shock protein is glycosylated, and wherein the amount of lectin present in the complexes relative to the amount of heat shock protein is equal to or greater than 50ng, 75ng, 100ng, or 200ng per microgram of heat shock protein. Preferably, the amount of lectin present in the complexes relative to the amount of heat shock protein is 50ng to 1000ng, 50ng to 500ng, 100ng to 250ng, or 150ng to 200ng lectin per microgram of heat shock protein. In some embodiments, the amount of lectin present in the complexes relative to the amount of heat shock protein is equal to or less than 5ng per microgram of heat shock protein. Preferably, the amount of lectin present in the composition relative to the amount of heat shock protein is between 0.1ng to 5ng, 0.2ng to 4ng, 0.3ng to 3ng, 0.5ng to 2ng, or 0.1ng to 1 ng lectin per microgram of heat shock protein.

Disclosed herein is a pharmaceutical composition comprising a pharmaceutically acceptable carrier and one or more molecular complexes, wherein each molecular complex comprises a lectin and an immunologically and/or biologically active glycoprotein, and wherein said glycoprotein forms oligomers in said complex in the presence of lectin. In one embodiment, the glycoprotein is an Antigenic Molecule. In a specific embodiment, the molecular complex comprises a lectin, a glycoprotein that is an Antigenic Molecule, and another molecule, such as a heat shock protein ("HSP"), that may or may not be glycosylated. In another embodiment, the glycoprotein is not an Antigenic Molecule. In a specific embodiment, the glycoprotein is a glycosylated heat shock protein. In yet another embodiment, the molecular complex comprises a lectin, a glycoprotein that is not an Antigenic Molecule, and an Antigenic Molecule (which may or may not be a glycoprotein). In a preferred embodiment, the Antigenic Molecule is a protein (including peptide and polypeptide) that displays the antigenicity of an antigen of a type of cancer or of an agent of an infectious disease. In some embodiments, the glycoprotein is a heat shock protein, and the amount of lectin present in the composition relative to the amount of heat shock protein is equal to or greater than 5ng, 10ng, 20ng, 30ng, 40ng, 50ng, 75ng, 100ng, or 200ng per microgram of glycoprotein. Preferably, the amount of lectin present in the composition relative to the amount of heat shock protein is 40ng to 1000ng, 50ng to 1000ng, 50ng to 500ng, 50ng to 250ng, or 100ng to 500ng lectin per microgram of HSP. In some embodiments, the amount of lectin present in the composition relative to the amount of heat shock protein is equal to or less than 5ng per microgram of HSP. Preferably, the amount of lectin present in the composition relative to the amount of HSP is between 0.1ng to 5ng, 0.1ng to 4ng, 0.1ng to 3ng, 0.1ng to 2ng, 0.1ng to 1ng, 0.5ng to 5ng, 0.5ng to 3ng, or 1ng to 4ng lectin per microgram of glycoprotein. In some embodiments, the glycoprotein is not a heat shock protein, and the amount of lectin present in the composition relative to the amount of glycoprotein is equal to or greater than 1fg, 100fg, 500fg, 1pg, 100pg, 500pg, 1ng, 2ng, 5ng, 10ng, 20ng, 30ng, 40ng, 50ng, 75ng, 100ng, or 200ng per microgram of glycoprotein.

The molecular complex of the invention comprises a lectin associated with a glycosylated heat shock protein complexed to an Antigenic Molecule (e.g., an antigenic protein (including antigenic peptide and polypeptide)). Some heat shock proteins are naturally glycosylated, including but are not limited to, gp96, GRP170, calreticulin, and Bip (GRP78). Heat shock proteins that are not naturally glycosylated can also be converted into a glycoprotein by adding one or more glycosylation sites that are not present in the native amino acid sequences comprising the heat shock protein followed by addition of carbohydrate groups, or by engineering HSP to contain peptide sequences that bind Con A (see Scott et al., PNAS (1992) 89:5398-5402), or covalently attaching HSP to Con A using coupling chemistry known in the art. It is disclosed herein that the molecular complexes comprise a lectin associated with an immunologically and/or biologically active glycoprotein that is not a heat shock protein. In yet some other embodiments, the molecular complexes comprise a lectin associated with an immunologically and/or biologically active glycoprotein complexed to a heat shock protein (which may or may not be glycosylated). In some embodiments, the molecular complex of the invention is a noncovalent complex. It is disclosed that the molecular complex of the invention is a covalent complex.

In a preferred embodiment, the lectin in the molecular complexes of the invention is a mannose-binding lectin. In a specific embodiment, the lectin in the molecular complexes of the invention is Concanavalin A (Con A). In a preferred embodiment, the heat shock protein in the molecular complexes of the invention is gp96. In a specific embodiment, the molecular complexes of the invention are purified.

The present invention also provides methods of making the molecular complexes of the invention. In some embodiment, lectin is added after a glycoprotein or a complex of a glycoprotein with another molecule (*e.g.*, glycosylated HSP associated with an Antigenic Molecule) is purified to promote the oligomerization of the glycoprotein. In some embodiment, lectin is added during the process of purifying a glycoprotein or a complex of a glycoprotein with another molecule (*e.g.*, glycosylated HSP with an Antigenic Molecule) to promote the oligomerization of the glycoprotein. In one embodiment, the present invention provides a method of making one or more noncovalent complexes, wherein each complex comprises a heat shock protein, an Antigenic Molecule, and a lectin, and wherein said heat shock proteins are glycosylated, said method comprising the steps of ; (a) binding said lectin to said heat shock protein; and (b) complexing said heat shock protein to the Antigenic Molecule. In another embodiment, the present invention provides a method of making one or more noncovalent complexes, wherein each complex comprises a heat shock protein, an Antigenic Molecule, and a lectin, and wherein said heat shock protein and/or Antigenic Molecule are glycosylated, said method comprising binding a lectin to one or more complexes, each complex comprising a heat shock protein and an Antigenic Molecule, wherein said lectin is not bound to a solid phase. In a specific embodiment, the method comprises isolating the complex of heat shock protein and Antigenic Molecule by lectin-based affinity chromatography prior to binding the complex to a lectin. In another specific embodiment, the method comprises isolating said complex of heat shock protein and Antigenic Molecule by non-lectin based protein purification method, such as antibody-based affinity chromatography, prior to binding the complex to a lectin. The invention further provides compositions made by the described methods.

Disclosed herein is a method of preventing or treating a disease (e.g., cancer, infectious diseases, anemia, growth hormone deficiencies, enzyme deficiency diseases, conditions of immune suppression, etc.) comprising administering to a subject in need thereof a prophylactically or therapeutically effective amount of a composition comprising one or more noncovalent complexes, wherein each complex comprises a lectin associated with an immunologically and/or biologically active glycoprotein. In one embodiment, the glycoprotein is an Antigenic Molecule. In a specific embodiment, the complex comprises a lectin, a glycoprotein that is an Antigenic Molecule, and another molecule, such as a heat shock protein ("HSP"), that may or may not be glycosylated. In another embodiment, the glycoprotein is not an Antigenic Molecule. In a specific embodiment, the glycoprotein is a glycosylated heat shock protein. In yet another embodiment, the molecular complex comprises a lectin, a glycoprotein that is not an Antigenic Molecule, and an Antigenic Molecule (which may or may not be a glycoprotein). In a specific embodiment, the Antigenic Molecule is a protein (including peptide and polypeptide) that displays the antigenicity of an antigen of a type of cancer or of an agent of an infectious disease. The composition may further comprise a pharmaceutically acceptable carrier.

Disclosed herein is a method of preventing or treating a disease (*e.g.*, cancer, infectious diseases, anemia, growth hormone deficiencies, enzyme deficiency diseases, conditions of immune suppression, etc.) comprising administering to a subject in need thereof a prophylactically or therapeutically effective amount of a composition comprising one or more noncovalent complexes, wherein each complex comprises a lectin, a heat shock protein, and an Antigenic Molecule, wherein said heat shock protein and/or Antigenic Molecule is/are glycosylated, and wherein the amount of lectin present in the composition relative to the amount ofHsp is equal to or greater than 5ng, 10ng, 20ng, 30ng, 40ng, 50ng, 75ng, 100ng, or 200ng per microgram of heat shock protein. Preferably, the amount of lectin present in the composition relative to the amount ofHsps is 40ng to 1000ng, 50ng to 1000ng, 50ng to 500ng, 50ng to 250ng, or 100ng to 500ng lectin per microgram of heat shock protein. In some embodiments, the amount of lectin present in the composition relative to the amount of Hsp is equal to or less than 5ng per microgram of heat shock protein. Preferably, the amount of lectin present in the composition relative to the amount of heat shock protein is between 0.1ng to 5ng, 0.1ng to 4ng, 0.1ng to 3ng, 0.1ng to 2ng, 0.1ng to 1ng, 0.5ng to 5ng, 0.5ng to 3ng, or 1ng to 4ng lectin per microgram of heat shock protein. The composition may further comprise a pharmaceutically acceptable carrier. In a specific embodiment, the Antigenic Molecule is a protein that displays the antigenicity of an antigen of a type of cancer or of antigen of an agent of an infectious disease.

In a preferred embodiment, the immunologically and/or biologically active glycoprotein of the molecular complex, *e.g.* a heat shock protein complexed to a peptide that displays the antigenicity of an antigen of a type of cancer or of an antigen of an agent of an infectious disease, is autologous to the subject; that is, it is isolated from the cells of the subject himself (*e.g.*, prepared from tumor biopsies of the patient when the treatment of cancer is desired). Alternatively, the molecular complex can be allogeneic to the subject to whom a composition of the molecular complex of the invention is administered. Alternatively, the molecular complex is prepared *in vitro*, *e.g.*, from cultured cells that recombinantly express a heat shock protein.

Disclosed herein are methods and compositions for prevention and treatment of primary and metastatic neoplastic diseases. In addition to cancer therapy, the compositions of the invention can be utilized for the prevention of a variety of cancers, *e.g.*, in subjects who are predisposed as a result of familial history or in subjects with an enhanced risk to cancer due to environmental factors.

Disclosed herein is a method of preventing or treating a disease (*e.g.*, cancer, infectious diseases, anemia, growth hormone deficiencies, enzyme deficiency diseases, conditions of immune suppression, etc.) comprising administering to a subject in need thereof a prophylactically or therapeutically effective amount of a composition comprising one or more molecular complexes of the invention in combination with one or more prophylactic or therapeutic agents other than the molecular complexes of the invention. Disclosed herein is a method of preventing or treating a disease (*e.g.*, cancer, infectious diseases, anemia, growth hormone deficiencies, enzyme deficiency diseases, conditions of immune suppression, etc.) comprising administering to a subject in need thereof a prophylactically or therapeutically effective amount of a composition comprising one or more molecular complexes of the invention, and one or more immune response enhancers or biological response modifiers, including but not limited to, cytokines, agonists or antagonists of various ligands, receptors and signal transduction molecules, immunostimulatory nucleic acids, and adjuvants. In accordance with this aspect , the compositions of the invention are administered in combination therapy with one or more of these immune response enhancers or biological response modifiers. In another embodiment, the compositions of the invention are administered in combination with radiotherapy or one or more chemotherapeutic agents for the treatment of cancer. In yet another embodiment, the compositions of the invention are administered in combination with anti-viral, anti-bacterial, anti-fungal, anti-parasitic agents for treating or preventing an infectiour disease.

Disclosed herein are methods of delivering one or more glycoproteins or one or more complexes comprising glycoproteins to a desirable site or a desirable cell type in a subject, said method comprising administering to the subject one or more molecular complexes, wherein each molecular complex comprises said glycoprotein and a lectin. In a specific embodiment, the glycoprotein is an Antigenic Molecule.

The present invention further provides kits comprising a plurality of containers each comprising a pharmaceutical formulation or composition comprising a dose of molecular complexes of the invention sufficient for a single prophylactic or therapeutic administration. The invention also provides kits comprising a container comprising an immunologically and/or biologically active glycoprotein or a complex thereof, and a container comprising lectin. In a specific embodiment, the present invention provides a kit comprising: (a) a first container containing a composition comprising a population of noncovalent complexes, each complex comprising a heat shock protein and an Antigenic Molecule, wherein the heat shock protein and/or Antigenic Molecule are glycosylated; and (b) a second container containing purified lectin. Optionally, instructions for formulating the oligomerized complex according to the methods of the invention can be included in the kits.

Specific therapeutic regimens and pharmaceutical compositions are also provided by the invention.

### 4. BRIEF DESCRIPTION OF THE FIGURES

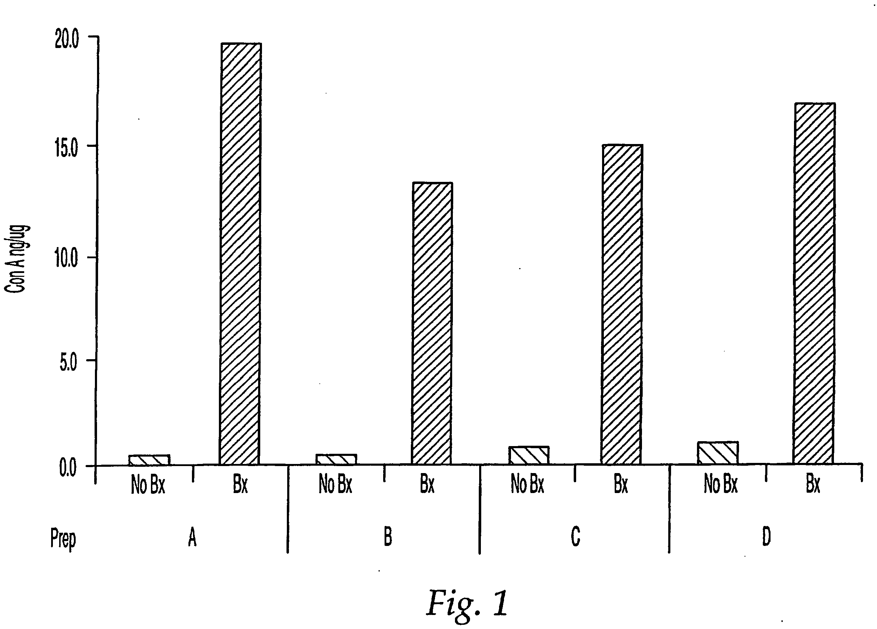

Figure 1. Consistently elevated levels of Con A in human gp96-peptide complexes lots. Tissue homogenates from four independent human renal tumor samples (A through D) were prepared and processed through Con A column chromatography. The Con A eluate was divided and half of the material set aside. The remaining sample was buffer exchanged into PBS (PD-10 column) and then both samples further purified over separate DEAE columns producing two homogenate-matched final products - one produced without buffer exchange (no Bx) between Con A and DEAE columns and one produced with a buffer exchange step (Bx) between the two columns. A sensitive ELISA to detect Con A was then used to determine the Con A concentration in these separate gp96-peptide complex samples. gp96 purified from a common homogenate using a process including the buffer exchange step had higher levels of Con A than did the corresponding homogenate-matched gp96 sample produced with the omission of the buffer exchange step.

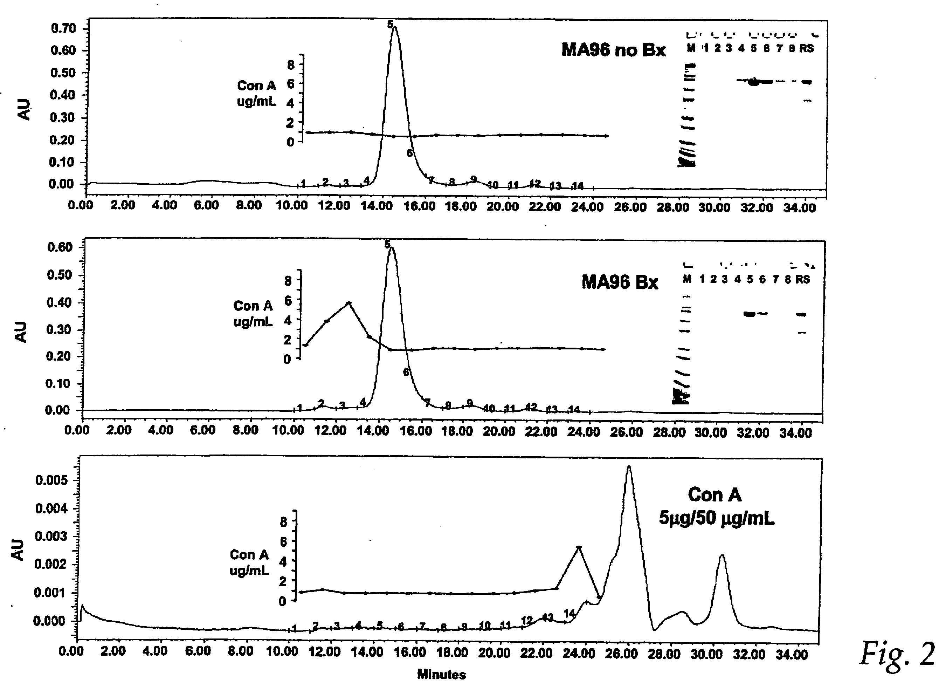

Figure 2. Con A is present in an oligomerized molecular complex. A common homogenate from chemically induced murine fibrosarcoma (Meth A) tissue was prepared and processed through Con A column chromatography. The Con A eluate was divided and half of the material set aside. The remaining sample was buffer exchanged into PBS and then both samples purified over separate DEAE columns producing two homogenate-matched final products - one produced without buffer exchange (no Bx) and one produced with a buffer exchange step (Bx) between the Con A and DEAE columns. Both samples, along with a sample of free Con A (5ug, 50ug/mL) were fractionated by SEC on a Superdex 200 column (Upper, middle, lower respectively). Collected fractions were analyzed for gp96 by SDS-PAGE (Fractions 1 through 8; inset) and the Con A content in the individual fractions evaluated by a direct ELISA against Con A (fractions 1 through 14; overlay). Little Con A was found in the no Bx-gp96 preparation while the gp96 produced with Bx was shown to have Con A in fractions 1 through 5. Free Con A eluted much later suggesting the Con A present in the Bx-gp96 sample was not free, but associated with a higher molecular weight species.

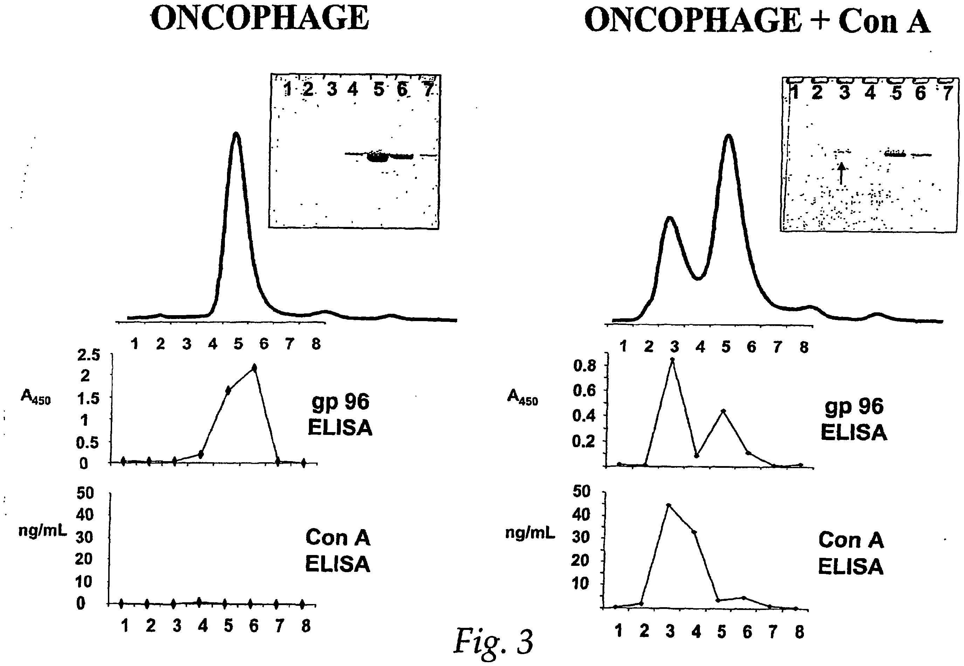

Figure 3. Con A mediates a shift in the elution position of gp96. A common homogenate from Meth A-induced murine fibrosarcoma tissue was prepared and processed using a process that included Con A and DEAE column chromatography. The final purified gp96 preparation was divided in two. To one half of the material, exogenous Con A was added to a final concentration of 50ug/mL; buffer alone was added to the other as a control, both samples incubated at 37°C for 2hr and then fractionated by SEC (Superdex 200). Individual fractions were analyzed by SDS-PAGE, and by gp96- and Con A-specific ELISA. Analytical data for material produced without the Buffer exchange step is shown in the left panel; that to which Con A was added to the right. In the left panel the peak of gp96 is in fraction 5 and con A levels as detected by specific ELISA are low. In the right panel (con A added) two peaks of gp96 are evident as shown by SDS-PAGE (inset; peak fractions 3 (arrow) and 5) and gp96 ELISA (fractions 3 and 5) as well as a distinct peak of Con A centered on fraction 3. Con A mediated a shift in the elution position of gp96.

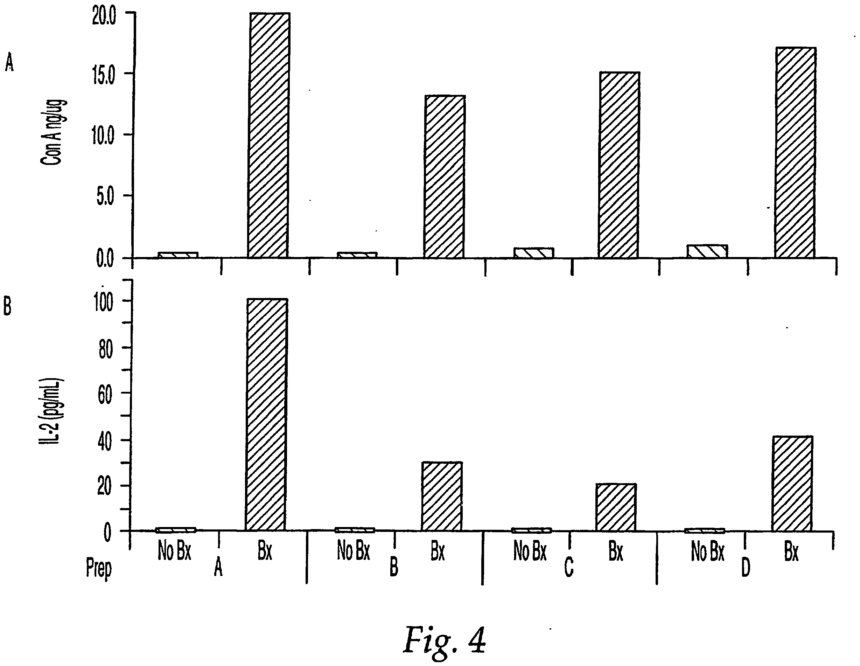

Figure 4. Con A content correlates with *in vitro* potency for human gp96 samples. The gp96 samples from four independent human renal tumor samples (A through D) were prepared as described above (See. Fig. 1) generating four paired samples differing only in the inclusion or omission of a buffer exchange step between Con A and DEAE columns. All eight samples were assayed for Con A content (Panel A; also see Fig. 1) along with *in vitro* antigen representation using the CD71 system (Panel B). In each case, material prepared by the process including the buffer exchange step (and containing increased levels of con A) had higher *in vitro* representation activity than a sample generated from the matching tumor homogenate and prepared by a step in which the buffer exchange step was omitted.

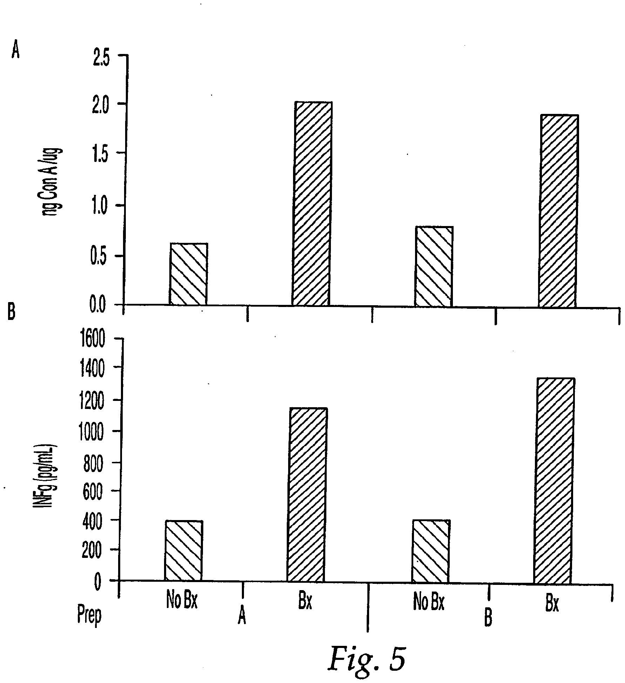

Figure 5. Con A correlates with *in vitro* potency in the murine CT26 system. The gp96 samples from two independent murine CT26 tumor samples (Preps A and B) were prepared as described above for human tumor derived samples (See. Fig. 1) All four samples were assayed for Con A content (Panel A) and *in vitro* antigen representation using the CT26 system (Panel B). In each case, material prepared by the process including the buffer exchange step (and having an increased amount of Con A) had higher *in vitro* representation activity than a sample generated from the matching tumor homogenate and prepared by a step in which the buffer exchange step was omitted.

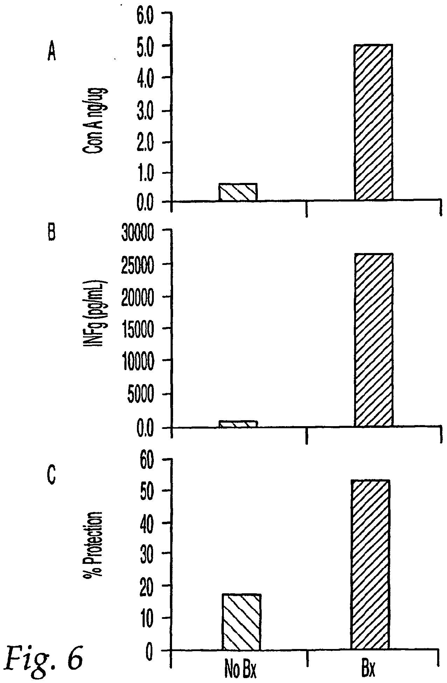

Figure 6. Con A correlates with both *in vitro* in the Meth A representation assay and *in vivo* potency in the murine Meth A tumor protection model. Two separate Meth A gp96 preparations were prepared from a common tumor homogenate (described above in Figure 1) generating a paired sample differing only in the inclusion or omission of a buffer exchange step between Con A and DEAE columns. These samples were assayed by Con A ELISA for Con A content (Panel A), *in vitro* activity in the Meth A representation assay (Panel B) and *in vivo* in the meth A tumor protection assay at a dose of 10µg (Panel C). Meth A gp96 prepared by a process including a buffer exchange step between Con A and DEAE columns had increased Con A content, higher *in vitro* antigen representation and higher *in vivo* tumor protection activity over that prepared by a process in which the buffer exchange step was omitted.

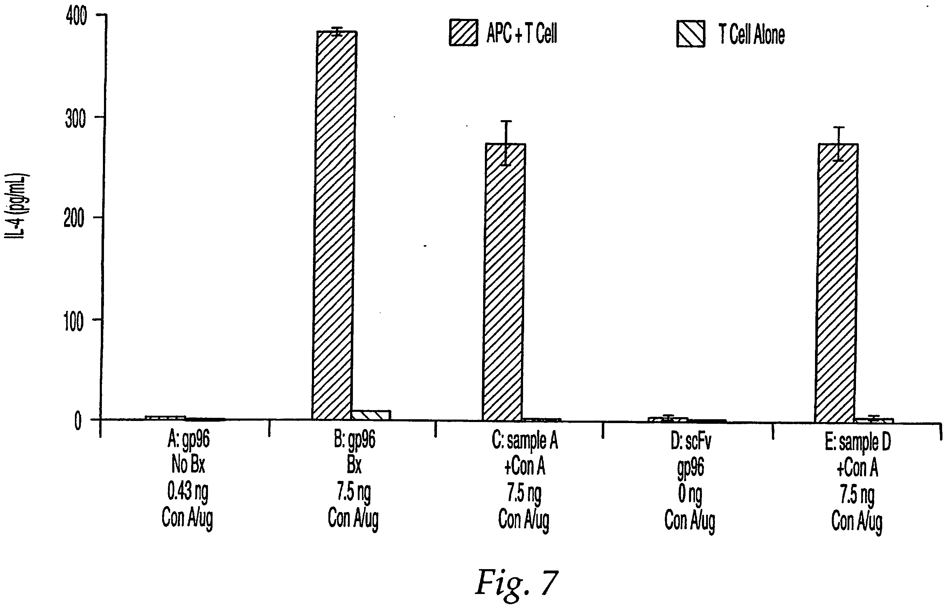

Figure 7. Exogenous Con A increases gp96 activity in the CD71 *in vitro* representation assay. Human liver tissue was homogenized and centrifuged producing an 11K supernatant that was divided into 3 identical samples and processed by different methods. Two lots were processed through Con A column chromatography, the Con A eluate was divided and half of the material set aside. The remaining sample was buffer exchanged into PBS and then both samples purified over separate DEAE columns producing two homogenate-matched final products - one produced without buffer exchange (NO Bx - sample A) and one produced with a buffer exchange step (Bx - sample B) between the Con A and DEAE columns. The gp96 from the remaining 11K supernatant was purified using a gp96-specific scFv column as described (Arnold-Schild et al., Cancer Research, 2000, 60(15):4175-4178) and the eluate concentrated producing sample D. All samples were analyzed for Con A concentration by a Con A specific ELISA and for *in vitro* antigen representation in the CD71 system ata protein concentration of 75µg/mL. For matched sample pairs, material produced by the process including the buffer exchange step (sample #1; Con A content 7.5ng/µg total protein) was more active *in vitro* than material produced by a process in which the buffer exchange step was omitted (sample A; con A content 0.43ng/µg total protein). Material produced by a single-step method that did not utilize a Con A column purification step (scFv gp96; 0 ng/µg) had low *in vitro* activity similar to sample A. Addition of exogenous Con A to samples A and C to levels equivalent to that in sample B (7.5ng con A/µg total protein), increased the specific *in vitro* antigen representation activity to a level similar to that present in sample B. This level of Con A had no effect on T cells alone.

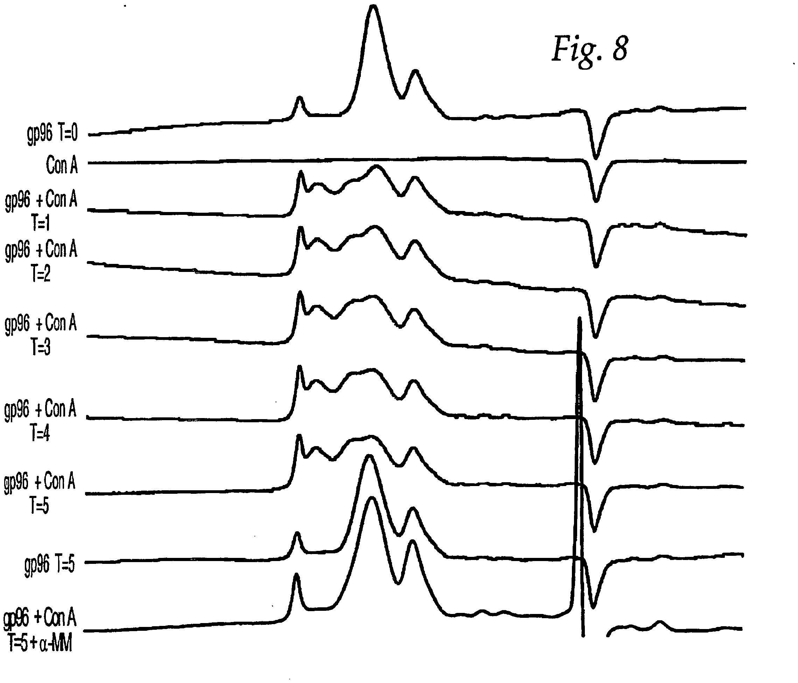

Figure 8. The oligomeric species is Methyl α-D-Mannopyranoside (α-MM) sensitive. A meth A gp96 sample was purified by the standard purification process including Con A and DEAE chromatography (without buffer exchange) and the protein analyzed by analytical SEC using a superose 6 column (Pharmacia) which showed the protein preparation contained primarily dimeric gp96 (gp96 T=0). Con A was added (50µg/mL final) to an aliquot of this gp96 sample (concentration 500µg/ml), the sample incubated at RT and hourly samples were taken (T=1 through T=5) and analyzed by SEC. A sample comprising Con A alone was also run. The addition of con A mediated a shift in the elution position of the gp96 dimer peak which changed only slightly following the first time point. gp96 alone did not change over this time period (gp96 T=5). Following the final time point, two separate aliquots of the final 5hr sample were taken and either an equal volume of PBS or PBS containing 10% α-MM added. Each sample was then re-analyzed by SEC. No change was evident in the sample to which PBS was added (not shown). The addition of α-MM dissociated the high molecular weight complex (gp96 +con A T=5 + α-MM) resulting in the SEC profile resembling that of the original gp96 sample (gp96 T=0 or T=5).

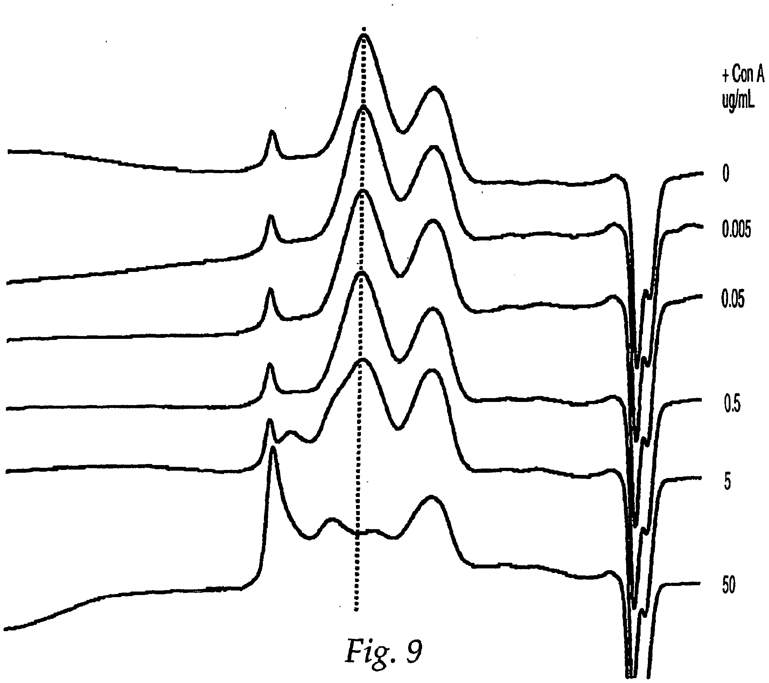

Figure 9. Low Con A:gp96 stoichiometries mediate an SEC sensitive shift in the gp96 elution position. Human renal tumor gp96 was purified by the standard purification process including Con A and DEAE chromatography and the protein analyzed by analytical SEC using a superose 6 column (Pharmacia). Con A was added to final concentration of 0.005 - 50µg/mL to gp96 (180ug/mL), the sample incubated at room temperature for one hour and analyzed by SEC. Stiochiometries of ∼ 1 Con A : 10 gp96 are able to generate an SEC sensitive shift in gp96 elution position.

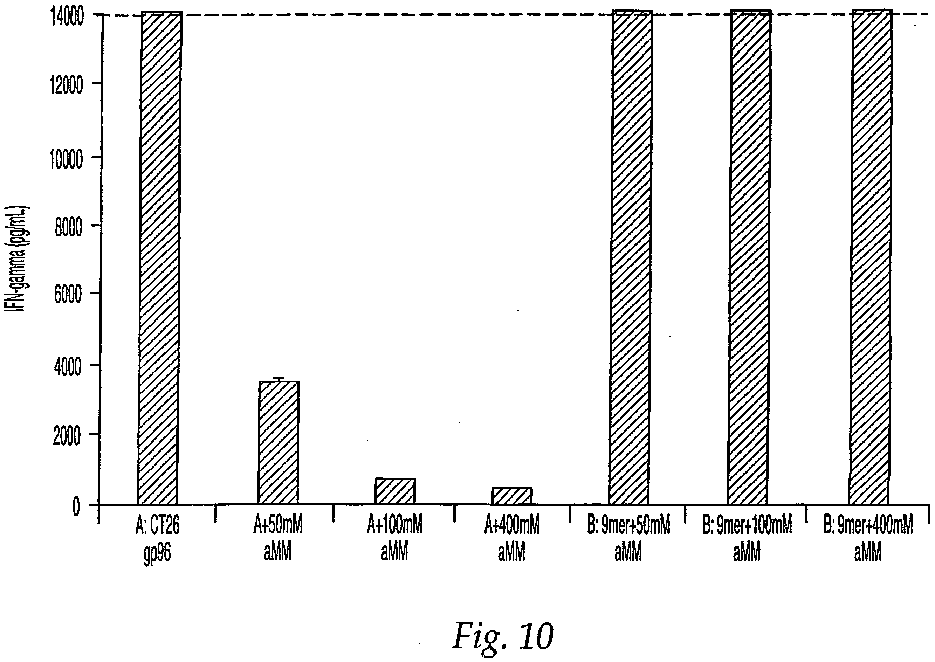

Figure 10. Addition of Methyl α-D-Mannopyranoside causes a concentration dependent decrease in CT26 *in vitro* antigen representation activity. A sample of CT26-derived gp96 (prepared by the Bx process) along with a positive control 9mer peptide (SPSYVYHQF) were incubated for 30 minutes in the presence of 50, 100 or 400 mM α-MM prior to being diluted (1 in 5) into a microtiter plate well containing RAW264.7 APC cells and AH1 specific T-cells. The samples were incubated overnight and the resulting supernatants analyzed by an INF-γ specific ELISA. α-MM caused a dose-dependent decrease in CT26 antigen representation and was without effect on T-cell recognition of the positive control 9mer peptide.

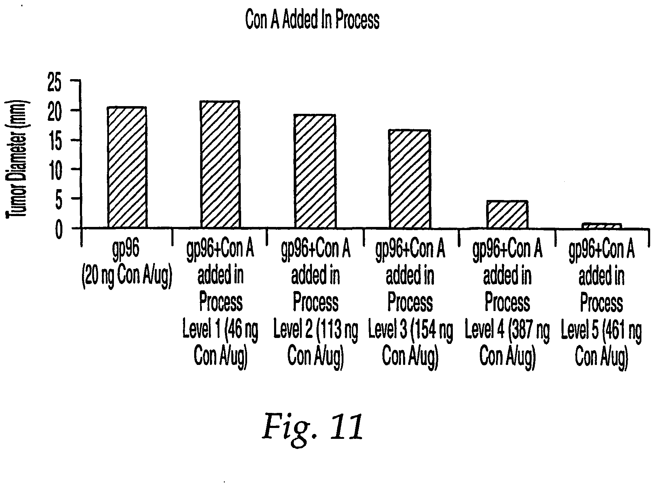

Figure 11. Addition of Con A during the purification of Hsp peptide causes a titratable increase in the tumor rejection activity of HSPPC-96.

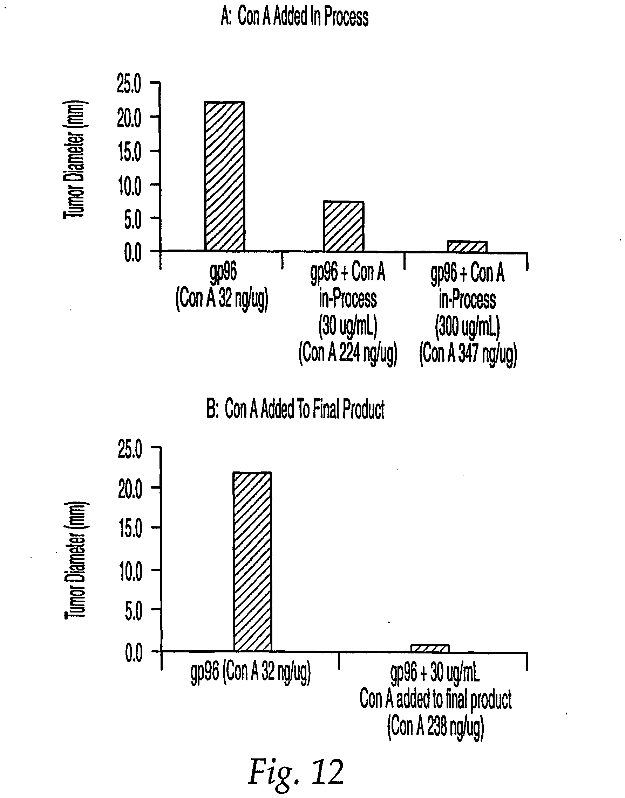

Figure 12. Can A increases the tumor rejection activity of gp96. (A) Con A added during the purification of Hsp-peptide. (B) Con A added to final product.

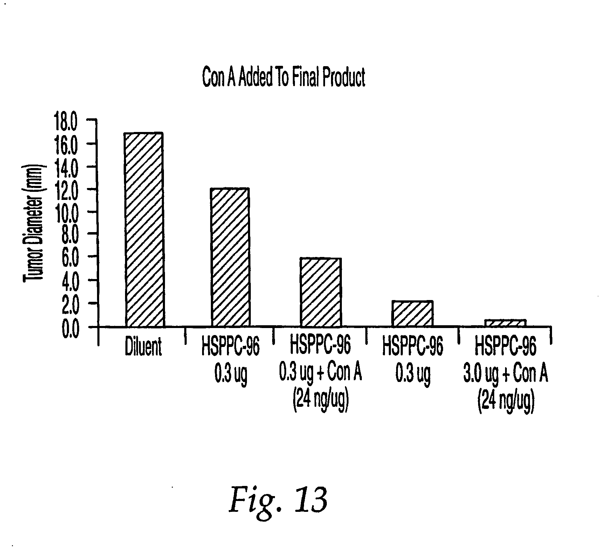

Figure 13. Con A increases the tumor rejection activity of HSPPC-96 that was purified by immunoaffinity column.

### 5. DETAILED DESCRIPTION OF THE INVENTION

The present invention provides one or more noncovalent complexes, each complex comprising a heat shock protein, an antigenic molecule, and a lectin, wherein said heat shock protein is glycosylated, and wherein the amount of lectin present in said complexes relative to the amount of heat shock protein is greater than or equal to 50 nanograms lectin per microgram of heat shock protein or less than or equal to 5 nanogram lectin per microgram of heat shock protein.

As will be apparent to a person skilled in the art, "a" lectin, "a" glycoprotein, or "a" any other molecule, when used in the context of a complex (*i.e.*, as a component of a complex), refers to "at least one" lectin, "at least one" glycoprotein, or "at least one" any other molecule, respectively, unless expressly indicated otherwise. Methods of making the molecular complexes, and the molecular complexes or compositions comprising such molecular complexes for use in the prevention and treatment of various diseases (*e.g.*, cancer, infectious diseases, anemia, growth hormone deficiencies, enzyme deficiency diseases, conditions of immune suppression, etc.) and for use in eliciting an immune response in a subject in need thereof, are also provided. The invention is useful in various situations, including but not limited to, where it is desirable to modulate the biological potency of an immunotherapeutic and/or biologically active molecule; to improve vaccine delivery into antigen presenting cells by specific and/or alternate receptor or non-receptor mediated events; to improve delivery of an immunotherapeutic and/or biologically active moiety to a target of interest; to improve the adjuvant capabilities of an immunotherapeutic moiety; or to capture secondary immunotherapeutic/immunoactive moieties and deliver them into an antigen presenting cell via specific receptor-mediated uptake.

As used herein, unless otherwise indicated, the terms "molecule", "complex", "molecular complex", "glycoprotein", "glycopeptide", "heat shock protein", "glycosylated heat shock protein", and "lectin" when used in singular, also encompasses a plurality of the molecules, and may refer to a population of the referred molecules.

As used herein, unless otherwise indicated, the term "glycoprotein" refers to a molecule comprising a protein and one or more carbohydrate moieties. A glycoprotein can be either a naturally occurring glycoprotein, or formed by a synthetic glycosylation process, i. e., the process in which a carbohydrate is joined to the protein molecule. A glycoprotein can be either a glycopeptide or a glycopolypeptide. Non-limiting examples of naturally occurring glycoproteins including, but are not limited to, human growth hormones, erythropoietin (EPO), antibodies, tissue plasminogen activator (tPA), granulocyte colony-stimulating factor (G-CSF), granulocyte-macrophage colony-stimulating factor (GM-CSF), and glucocerebrosidase (Cerezyme™ from Genzyme). In a preferred embodiment, a glycoprotein is a heat shock protein. In another preferred embodiment, a glycoprotein is an immunoactive heat shock protein. As used herein, "immunoactive heat shock protein" refers to heat shock proteins that have the ability to modulate, preferably enhance, an immune response, preferably an immune response directed against an Antigenic Molecule to which the heat shock protein is complexed. As used herein, the term "Antigenic Molecule" refers to a molecule that displays one or more antigenic determinants against which an immune response is desired in a subject (*e.g.*, for therapeutic purposes). Non-limiting examples of Antigenic Molecules are given in Section 5.2. In a preferred embodiment, a glycoprotein is a glycosylated heat shock protein including, but not limited to, gp96, GRP170, Calreticulin, and Bip (GRP78). Preferably, the glycosylated heat shock protein is gp96. In certain embodiments, a glycoprotein can also be an Antigenic Molecule, which may be a naturally occurring glycoprotein or an Antigenic Molecule that is engineered to be a glycoprotein. Non-limiting examples of Antigenic Molecules are described in Section 5.2. In certain embodiments, the molecular complex of the invention comprises a lectin and a glycoprotein that is not denatured.

A protein (including peptide and polypeptide) can be genetically engineered into a glycoprotein by adding one or more glycosylation sites that are not present in the native amino acid sequence and recombinantly expressing the protein in a host cell that can glycosylate proteins. Glycosylation of a protein is typically either N-linked or O-linked. The term "N-linked" refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. The term "O-linked glycosylation" refers to the attachment of one of the sugars such as N-aceylgalactosamine, galactose, or xylose to a hydroxylamino acid, most commonly serine or threonine, although 5-hydroxyproline or 5-hydroxylysine may also be used.

Addition of glycosylation sites may be accomplished by various means. For example, the amino acid sequence of a protein or peptide of interest can be altered such that it contains one or more of the above-described tripeptide sequences (for N-linked glycosylation sites). The alteration may also be made by the addition of, or substitution by, one or more serine or threonine residues to the native sequence (for O-linked glycosylation sites). The amino acid sequence may optionally be altered through changes at the DNA level, particularly by mutating the DNA encoding a protein of interest at pre-selected bases such that codons are generated that will translate into the desired amino acids. The DNA mutation(s) may be made, for example, using methods described in U.S. Pat. No. 5,364,934.

Another means of increasing the number of carbohydrate moieties on a protein (including peptide and polypeptide) is by chemical or enzymatic coupling of glycosides to the protein. Depending on the coupling mode used, the sugar(s) may be attached to (a) arginine and histidine, (b) free carboxyl groups, (c) free sulfhydryl groups such as those of cysteine, (d) free hydroxyl groups such as those of serine, threonine, or hydroxyproline, (e) aromatic residues such as those of phenylalanine, tyrosine, or tryptophan, or (f) the amide group of glutamine. Any methods known in the art, such as the methods described in the International Publication No. WO 87/05330, and in Aplin and Wriston, CRC Crit. Rev. Biochem., pp. 259-306 (1981), can be used. Once one or more carbohydrates are added to a protein, lectin can form a complex with the protein by binding to the carbohydrate units.

In some embodiments of the invention, the glycoprotein displays the antigenicity of an antigen of a type of cancer or of an antigen of an agent of an infectious disease. In some other embodiments of the invention, the glycoprotein does not display the antigenicity of an antigen of a type of cancer or of an antigen of an agent of an infectious disease. In some embodiments, the glycoprotein is a heat shock protein, and the protein that the heat shock protein chaperones (*in vivo*) or complexed with displays the antigenicity of an antigen of a type of cancer or of an antigen of an agent of an infectious disease.

As used herein, the term "antigenicity" refers to the ability of a molecule to bind antibody or MHC molecules. As used herein, "a type of cancer" refers to the cell type of the tissue of origin, *e.g.*, breast, lung, ovarian. In one embodiment, the Antigenic Molecule displays the antigenicity of an antigen of an infectious agent. In another embodiment, the Antigenic Molecule displays the antigenicity of an antigen overexpressed in a cancer cell relative to its expression in a noncancerous cell of said cell type. In another embodiment, the Antigenic Molecule is a tumor specific antigen or a tumor-associated antigen. In another embodiment, the molecular complex is purified. In yet another embodiment, the purified molecular complex comprises lectin associated with a heat shock protein and an Antigenic Molecule of an infectious agent or an antigen overexpressed in a cancer cell relative to its expression in a noncancerous cell of said cell type.

Many lectins are known in the art. The term "lectin" refers to a group of proteins that share the property of binding to specific carbohydrate groups. A lectin can be purified from a natural source, such as from plants, animals, fungi, algae, and bacteria, or it can be synthesized chemically. In certain embodiments, lectins are cross linked to each other by any method known in the art to form a dimer or oligomer for use in the present invention. In a preferred embodiment, the lectin molecule is a mannose-binding lectin, which can be, but is not limited to, those listed in Table 1. Lectins are commercially available from many commercial vendors, such as Sigma (see Sigma's website sigmaaldrich.com/Brands/Sigma/Enzyme_Explorer_Home/Lectin_for_life_Science_html). In a preferred embodiment, the lectin is Concanavalin A (Con A).

**Table 1. Examples of Lectins**

| Sigma Prodcut No.: | LECTIN: | Mol. Wt. (kDa): | Subunits: | Specificity Blood Group: | Specificity Sugar: | Mitogenic Activity: |
|---|---|---|---|---|---|---|
| L 5640 | Agaricus bisporus | 58.5 | - | - | ß-gal(1->3)gaINAc | |
| L 4141 | Anguilla anguilla | 40 | 2 | H | α-L-Fuc | |
| L 0881 | Arachis hypogaea | 120 | 4 | T | ß-gal(1->3)gaINAc | |
| L 7759 | conjugate | | | | | |
| L 6135 | conjugate | | | | | |
| L 7381 | conjugate | | | | | |
| L 3766 | conjugate | | | | | |
| L 6646 | conjugate | | | | | |
| L 3515 | Artocarpus integrifolia | 42 | 4 | T | α-gal->OMe | (+) |
| L 4650 | conjugate | | | | | |
| L 5147 | conjugate | | | | | |

| Bandeiraea Simplicifolia | | | | | | |
|---|---|---|---|---|---|---|
| L 2380 | BS-I | 114 | 4 | A, B | α-gal, α-galNAc | |
| L 3759 | conjugate | | | | | |
| L 9381 | conjugate | | | | | |
| L 5264 | conjugate | | | | | |
| L 1509 | BS-I-A4 | 114 | 4 | A | α-galNAc | |
| L 0890 | conjugate | | | | | |
| L 3019 | BS-I-B4 | 114 | 4 | B | α-gal | |
| L 5391 | conjugate | | | | | |
| L 2140 | conjugate | | | | | |
| L 2895 | conjugate | | | | | |
| L 6013 | Bauhinia purpurea | 195 | 4 | - | ß-gal(1->3)galNAc | (+) |
| L 9637 | Caragana arborescens | 60;120 (c) | 2;4 | - | galNAc | |
| L 3141 | Cicer arietinum | 44 | 2 | - | fetuin | |
| L 2638 | Codium fragile | 60 | 4 | - | galNAc | |
| L 7275 | Concanavalin A | 102 | 4 | | α-man, α-glc | (+) |
| L 6397 | conjugate | | | | | |
| C 2272 | conjugate | | | | | |
| C 7642 | conjugate | | | | | |
| C 6904 | conjugate | | | | | |
| C 9017 | conjugate | | | | | |
| L 5021 | conjugate | | | | | |
| L 3642 | conjugate | | | | | |
| C 7898 | conjugate | | | | | |
| L 3885 | Succinyl-Concanavalin A | 51 | 2 | - | α-man, α-glc | (+) (d) |
| L 9385 | conjugate | | | | | |
| L 2766 | Datura stramonium | 86 | 2(α&ß)a | - | (glcNAc)2 | |
| L 2785 | Dolichos biflorus | 140 | 4 | A1 | α-galNAc | |
| L 1287 | conjugate | | | | | |
| L 6533 | conjugate | | | | | |
| L 9142 | conjugate | | | | | |
| L 9658 | conjugate | | | | | |
| L 9894 | conjugate | | | | | |
| L 2142 | Erythrina corallodendron | 60 | 2 | - | ß-gal(1->4)glcNAc | (+) |
| L 5390 | Erythrina cristagalli | 56.8 | 2(α&ß)a | - | ß-gal(1->4)glcNAc | |
| L 7400 | Euonymus europaeus | 166 | 4(α&ß)a | B, H | α-gal(1->3)gal | (+) |
| L 8725 | Galanthus nivalis | 52 | 4 | (h) | non-reduc. D-man | |
| L 8775 | conjugate | | | | | |
| L 1395 | Glycine max | 110 | 4 | - | galNAc | (+) (b) |
| L 2650 | conjugate | | | | | |
| L 4511 | conjugate | | | | | |
| L 1105 | conjugate | | | | | |
| L 6635 | Helix aspersa | 79 | - | A | galNAc | |
| L 8764 | conjugate | | | | | |
| L 3382 | Helix pomatia | 79 | 6 | A | galNAc | |
| L 6387 | conjugate | | | | | |
| L 6512 | conjugate | | | | | |
| L 1034 | conjugate | | | | | |
| L 1261 | conjugate | | | | | |
| L 9267 | Lens culinaris | 49 | 2 | - | α-man | (+) |
| L 4143 | conjugate | | | | | |
| L 9262 | conjugate | | | | | |
| L 0511 | conjugate | | | | | |
| L 2263 | Limulus polyphemus | 400 | 18 | - | NeuNAc | |
| L 2886 | Lycopersicon esculentum | 71 | - | - | (glcNAc)3 | (+) (e) |
| L 0651 | conjugate | | | | | |
| L 0401 | conjugate | | | | | |
| L 8025 | Maackia amurensis | 130 | 2(α&β) | O | sialic acid | (+) |
| L 6141 | Maclura pomifera | 40-43 | 2(α&β)a | - | α-gal,α-galNAc | |
| L 4401 | conjugate | | | | | |
| L 2013 | conjugate | | | | | |
| L 5650 | Narcissus pseudonarcissus | 26 | 2 | (h) | α-D-man | |
| L 3138 | Phaseolus coccineus | 112 | 4 | - | - | |
| L 4389 | conjugate | | | | | |

| Phaseolus Vulgaris | | | | | | |
|---|---|---|---|---|---|---|
| L 8629 | PHA-E | 128 | 4 | - | oligosaccharide | (+) |
| L 6139 | conjugate | | | | | |
| L 2769 | PHA-L | 126 | 4 | - | oligosaccharide | (+) |
| L 8754 | PHA-P | | | | | |
| L 2646 | PHA-M | | | | | |
| L 9379 | Phytolacca americana | 32f | - | - | (glcNAc)3 | (+) |
| L 2882 | conjugate | | | | | |
| L 5380 | Pisum sativum | 49 | 4(α&β)a | - | α-man | (+) |
| L 0770 | conjugate | | | | | |
| L 9895 | Pseudomonas aeruginosa (PA-1) | 13-13.7 | - | - | gal | |
| L 2138 | Psophocarpus tetragonolobus | 35 | 1 | - | - galNAc, gal galNAc, gal | |
| L 3139 | conjugate | | | | | |
| L 3014 | conjugate | | | | | |
| L 3264 | conjugate | | | | | |

| Ricinus Communis | | | | | | |
|---|---|---|---|---|---|---|
| L 7886 | Agglutinin, RCA₁₂₀ | 120 | 4 | - | ß-gal | |
| L 2390 | conjugate | | | | | |
| L 2758 | conjugate | | | | | |
| L 9514 | Ricin, A chain | | | | | |
| L 4022 | Ricin, A chain, deglycosylated | | | | | |
| L 9639 | Ricin, B chain | | | | | |
| L 6890 | Sambucus nigra | | | | | |
| L 4266 | Solanum tuberosum | | | | | |
| L 9254 | Tetragonolobus purpureas | 120(A), 58(B), 117(C) | 4;2;4 | H | α-L-fuc | |
| L 5759 | conjugate | | | | | |
| L 3134 | conjugate | | | | | |
| L 5644 | conjugate | | | | | |
| L 9640 | Triticum vulgaris | 36 | 2 | - | (glcNAc)2, NeuNAc | (+) |
| L 3892 | conjugate | | | | | |
| L 0390 | conjugate | | | | | |
| L 5142 | conjugate | | | | | |
| L 4895 | conjugate | | | | | |
| L 9884 | conjugate | | | | | |
| L 1894 | conjugate | | | | | |
| L 1882 | conjugate | | | | | |

| Ulex Europaeus | | | | | | |
|---|---|---|---|---|---|---|
| L 5505 | UEA | 68 | - | H | α-L-fuc | |
| L 8146 | conjugate | | | | | |
| L 8262 | conjugate | | | | | |
| L 9006 | conjugate | | | | | |
| L 4889 | conjugate | | | | | |
| L 6263 | Vicia faba | 50 | 4(α&β)a | - | man, glc | (+) |
| L 4011 | Vicia villosa | 139 | 4a | A₁+Tₙ | galNAc | |
| L 9388 | conjugate | | | | | |
| L 7513 | Isolectin B4 | 143 | 4 | Tₙ | galNAc | |
| L 7888 | conjugate | | | | | |
| L 2662 | Viscum album | 115g | 4(α&β)a | - | ß-gal | |
| | Wisteria floribunda | 68 | 2 | - | galNAc | |
| L 1516 | conjugate | | | | | |
| L 2016 | Reduced | 34 | 1 | - | galNAc | |
| L 1766 | conjugate | | | | | |

Disclosed herein are complexes comprising a lection and a glycoprotein (which may or may not be an Antigenic Molecule), and molecular complexes comprising a lectin, a glycoprotein, and also a molecule (e.g., an Antigenic Molecule) that is not a glycoprotein. Each molecular complex of the invention may comprise one or more molecules of a glycoprotein, one or more molecules of a lectin, and one or more Antigenic Molecules if present in the complex. Preferably, the complex comprises more than one molecule of glycoprotein which form oligomers in the presence of lectin. When the molecular complexes comprise more than one glycoprotein, the glycoproteins do not need to be homogenous, *i.e*., some of the glycoproteins in the population are Antigenic Molecules, and some of the glycoproteins are not Antigenic Molecules. The stoichiometry of the components of a molecular complex of the invention can be represented by the ratios (g) : (l) : (a) where (g), (l), and (a) can be any integer and (g) is the number of glycoprotein molecules (which may or may not be an Antigenic Molecule) in the complex, (1) is the number of lectin molecules in the complex, and (a) is the number of other molecules (*e.g*., Antigenic Molecules that are not glycoproteins) in the complex. In complexes where there is no other molecules (*e.g.*, no Antigenic Molecules that are not glycoproteins), a = 0. For example, a molecular complex may comprise one glycoprotein (which may or may not be an Antigenic Molecule) and one lectin. In another example, a molecular complex may comprise one lectin, two heat shock proteins, and two Antigenic Molecules. There are many more combinations that are encompassed by the present invention. In a preferred embodiment, 1 is greater than g, *i.e.*, the number of the lectins present in a molecular complex is more than the numbers of glycoproteins that are present in the molecular complex. In some embodiments, the molar ratio between glycoprotein and lectin is 3:1,2:1, or 1:1. In a preferred embodiment, lectin is Con A in a form of a tetramer, and for each tetramer Con A molecule, there are three, two, or one glycoprotein(s) attached.

Accordingly, in one embodiment, the invention provides a homogenous population of complexes wherein the stoichiometric ratios of (g) : (1) : (a) for all the complexes are identical or approximately the same. In another embodiment, the invention provides a population of complexes wherein the complexes display more than one ratio of (g) : (l) : (a) or the ratios are not known for all the complexes in the population, *i.e*., the stoichiometry of the components of a molecular complex of the invention may vary among the molecular complexes in a population. In embodiments of the invention where the stoichiometric ratios of a complex or a population of complexes have not been determined, the mass ratios of each component can be used in most cases to characterize the complex or population of complexes. For example, a population of complexes can be characterized and thus distinguished from other populations by the relative amounts of lectins and glycoproteins (including heat shock proteins). Examples of such complexes and methods for determining the mass ratios are provided in Section 5.4 hereinbelow. In some embodiments, a complex comprising a heat shock protein, a lectin and an Antigenic Molecule, wherein the amount of lectin relative to heat shock protein is greater than or equal to 50 ng, 75 ng, 100 ng, or 200ng per microgram of heat shock protein, is preferred. In other embodiments, a complex comprising a heat shock protein, a lectin and an Antigenic Molecule, wherein the amount of lectin relative to heat shock protein is less than or equal to 5 ng, 4ng, 3ng, 2ng, or 1 ng, is preferred.

In a preferred embodiment, a molecular complex comprises a lectin, a glycosylated heat shock protein, and a protein that displays the antigenicity of an antigen of a type of cancer or of an antigen of an agent of an infectious disease. The molecular complex comprises a lectin and an immunologically and/or biologically active glycoprotein, wherein said immunologically and/or biologically active glycoprotein can be, but is not limited to, human growth hormones, erythropoietin (EPO), antibody therapeutics, tissue plasminogen activator (tPA), granulocyte colony-stimulating factor (G-CSF), granulocyte-macrophage colony-stimulating factor (GM-CSF), and Cerezyme™ (Genzyme). In another preferred embodiment, the molecular complex comprises a lectin and an immunologically and/or biologically active glycoprotein, wherein said immunologically and/or biologically active glycoprotein is an Antigenic Molecule.

Disclosed herein are one or more molecular complexes, each complex comprising a lectin and an immunologically and/or biologically active glycoprotein, wherein the lectin forms oligomers with the glycoprotein, and wherein the amount of lectin present in the complex relative to the amount of glycoprotein is equal to or greater than 1fg, 100fg, 500fg, 1pg, 100pg, 500pg, 1ng, 2ng, 5ng, 10ng, 20ng, 30ng, 40ng, 50ng, 75ng, 100ng, or 200ng per microgram of glycoprotein. Preferably, the amount of lectin present in the complex relative to the amount of glycoprotein is 40ng to 1000ng, 50ng to 1000ng, 50ng to 500ng, 100ng to 250ng, or 150ng to 200ng lectin per microgram of glycoprotein. In some embodiments, the amount of lectin present in the complex relative to the amount of glycoprotein is equal to or less than 5ng per microgram of glycoprotein. Preferably, the amount of lectin present in the complex relative to the amount of glycoprotein is between 0.1ng to 5ng, 0.2ng to 4ng, 0.3ng to 3ng, 0.5ng to 2ng, or 0.1ng to 1ng lectin per microgram of glycoprotein. The molecular complex may further comprise one or more other molecules, preferably proteins (including peptides and polypeptides), that display the antigenicity of an antigen of a type of cancer or of an antigen of an agent of an infectious disease. In some embodiments, the glycoprotein is a heat shock protein. In some embodiments, the glycoprotein is not a heat shock protein. In some embodiments, the glycoprotein is an Antigenic molecule. In some embodiments, the glycoprotein is not an Antigenic molecule.

The present invention provides one or more molecular complexes, each complex comprises a heat shock protein, an Antigenic Molecule, and a lectin, wherein the amount of lectin present in the complex relative to the amount of heat shock protein is equal to or greater than 50ng, 75ng, 100ng or 200ng per microgram of heat shock protein. Preferably, the amount of lectin present in the complex relative to the amount of heat shock protein is 50ng to 1000ng, 50ng to 500ng, 100ng to 250ng, or 150ng to 200ng lectin per microgram of heat shock protein. In some embodiments, the amount of lectin present in the complex relative to the amount of heat shock protein is equal to or less than 5ng per microgram of heat shock protein. Preferably, the amount of lectin present in the complex relative to the amount of heat shock protein is between 0.1ng to 5ng, 0.2ng to 4ng, 0.3ng to 3ng, 0.5ng to 2ng, or 0.1ng to 1ng lectin per microgram of heat shock protein.

Disclosed herein is a pharmaceutical composition comprising one or more molecular complexes and a pharmaceutically acceptable carrier, wherein each molecular complex is a noncovalent complex comprising a lectin and an immunologically and/or biological active glycoprotein. In some embodiments, the glycoprotein is a heat shock protein, and the amount of lectin present in the complex relative to the amount of heat shock protein is equal to or greater than 5ng, 10ng, 20ng, 30ng, 40ng, 50ng, 75ng, 100ng, or 200ng per microgram of heat shock protein. Preferably, the amount of lectin present in the complex relative to the amount of heat shock protein is 40ng to 1000ng, 50ng to 1000ng, 50ng to 500ng, 100ng to 250ng, or 150ng to 200ng lectin per microgram of heat shock protein. In some embodiments, the amount of lectin present in the complex relative to the amount of heat shock protein is equal to or less than 5ng per microgram of heat shock protein. Preferably, the amount of lectin present in the complex relative to the amount of heat shock protein is between 0.1ng to 5ng, 0.2ng to 4ng, 0.3ng to 3ng, 0.5ng to 2ng, or 0.1ng to 1ng lectin per microgram of heat shock protein. The molecular complex may further comprise one or more molecules, preferably proteins that display antigenicity of an antigen of a type of cancer or of an antigen of an agent of an infectious disease.

Disclosed herein is a purified population of molecular complexes in which each complex comprises an immunologically and/or biologically active glycoprotein and a lectin. In some embodiments, the glycoprotein is a heat shock protein, and the amount of lectin present in the population relative to the amount of heat shock protein is equal to or greater than 5ng, 10ng, 20ng, 30ng, 40ng, 50ng, 75ng, 100ng, or 200ng per microgram of heat shock protein. Preferably, the amount of lectin present in the population relative to the amount of heat shock protein is 40ng to 1000ng, 50ng to 1000ng, 50ng to 500ng, 100ng to 250ng, or 150ng to 200ng lectin per-microgram of heat shock protein. In some embodiments, the amount of lectin present in the population relative to the amount of heat shock protein is equal to or less than 5ng per microgram of heat shock protein. Preferably, the amount of lectin present in the population relative to the amount of heat shock protein is between 0.1ng to 5ng, 0.2ng to 4ng, 0.3ng to 3ng, 0.5ng to 2ng, or 0.1ng to 1ng lectin per microgram of heat shock protein. The molecular complex may further comprise one or more molecules, e.g., proteins (including peptides and polypeptides), that display antigenicity of an antigen of a type of cancer or of an antigen of an agent of an infectious disease. In one embodiment, the molecular complexes of the population comprise the same Antigenic Molecule. In another embodiment, the molecular complexes of the population comprise different Antigenic Molecule. Also provided by the invention is a purified population of molecular complexes comprising lectin associated with a complex purified from a recombinant cell in which each complex comprises a glycoprotein associated with a protein that displays antigenicity of an antigen of a type of cancer or of an antigen of an agent of an infectious disease.

The present invention also provides methods of making a composition, which is preferably immunogenic against a disease (*e.g.*, against a type of cancer or an agent of infectious disease), comprising a step of adding lectin molecules to promote oligomerization of a molecular complex, wherein said molecular complex is prepared by a method described in section 5.1-5.4 of the application or a method know in the art. In one embodiment, the oligomerized molecular complex comprises a lectin and an immunologically and/or biologically active glycoprotein (which may or may not be an Antigenic Molecule). In another embodiment, the oligomerized molecular complex comprises a lectin, a glycoprotein, and an Antigenic Molecule, wherein said Antigenic Molecule displays the antigenicity of an antigen of a type of cancer or of an antigen of an agent of an infectious disease. In another embodiment, the oligomerized molecular complex comprises a lectin, a heat shock protein, and an immunologically and/or biologically active glycoprotein. In yet another embodiment, the molecular complex comprises a lectin, a glycosylated heat shock protein, and an Antigenic Molecule, and wherein said Antigenic Molecule displays the antigenicity of an antigen of a type of cancer or of an antigen of an agent of an infectious disease. The invention further provides compositions made by the described methods.

In one embodiment, the steps of preparation of the immunologically and/or biologically active glycoprotein or a complex comprising an immunologically and/or biologically active glycoprotein associated with one or more other molecules that are not lectins do not involve the use of lectins, such as lectins bound to a solid phase, typically used in column chromatography. Lectin is added to the immunologically and/or biologically active glycoprotein preparation or the complex preparation to promote oligomerization of the glycoproteins. In another embodiment, adding lectin is one of the steps or part of a step in the preparation of the immunologically and/or biologically active glycoprotein (or glycoprotein associated with one or more other molecules that are not lectin), wherein the amount of lectin added is sufficient to promote the oligomerization of the preparation, and to produce a final product or products. In some embodiments, the molecular complex comprises a heat shock protein and a lectin, and lectin is added so that the amount of lectin present in the final product(s) relative to the amount of heat shock protein is equal to or greater than 5ng, 10ng, 20ng, 30ng, 40ng, 50ng, 75ng, 100ng, or 200ng per microgram of heat shock protein. Preferably, the amount of lectin present in the final product(s) relative to the amount of heat shock protein is 40ng to 1000ng, 50ng to 1000ng, 50ng to 500ng, 100ng to 250ng, or 150ng to 200ng lectin per microgram of heat shock protein. In some embodiments, the amount of lectin added is sufficient to promote oligomerization of the preparation and to produce a final product or products wherein the amount of lectin present in final product(s) relative to the amount of heat shock protein is equal to or less than 5ng per microgram of heat shock protein. Preferably, the amount of lectin present in the final product(s) relative to the amount of heat shock protein is between 0.1ng to 5ng, 0.2ng to 4ng, 0.3ng to 3ng, 0.5ng to 2ng, or 0.1ng to 1ng lectin per microgram of heat shock protein. In a preferred embodiment, lectin is Con A.

In a preferred embodiment, said glycoprotein is a glycosylated heat shock protein (Hsp), including both naturally occurring glycosylated heat shock proteins (*e.g.*, gp96, GRP170, Calreticulin, Bip (GRP78), or a combination thereof) and heat shock proteins that are not naturally glycosylated are converted into a glycoprotein by adding one or more glycosylation sites that are not present in the native sequences encoding the heat shock protein followed by addition of carbohydrate groups. The immunologically and/or biologically active complex may further comprise a molecule that displays the antigenicity of an antigen of a type of cancer or of an antigen of an agent of an infectious disease. In the embodiments wherein the Antigenic Molecules are proteins complexed to Hsps *in vivo*, the complexes can be isolated from cells (see Section 5.1). Alternatively, an Hsp-antigen complex can be produced *in vitro* from purified preparations of Hsps and Antigenic Molecules (see Section 5.3). In this embodiment, antigens of cancers or infectious agents can be obtained by purification from natural sources, by chemical synthesis, or recombinantly. Lectins can form oligomers with both *in vivo* and *in vitro* produced Hsp-antigen complex through *in vitro* procedures such as those described in Section 5.1 to 5.4. In a preferred embodiment, said lectin is a mannose-bind lectin molecule. More preferably, the mannose-binding lectin molecule is Concanavalin A (Con A).

The compositions and methods of the present invention can be used in various situations. For example, the composition of the invention can be used to enhance the immunogenicity of a molecular complex and/or to elicit an immune response in a subject in whom the treatment or prevention of a disease (*e.g.*, cancer, an infectious disease, anemia, growth hormone deficiency disease, enzyme deficiency disease, or a condition of immune suppression). As used herein, the term "subject" refers to an animal, preferably a mammal, and more preferably a human, having the disease or prone to have the disease.

In accordance with the invention, administration of oligomerized immunologically and/or biologically active complexes to a subject results in eliciting, stimulating, modulating (including enhancing and down regulating), and/or sustaining an immune response and/or biological activity in the subject, particularly against antigenic proteins specific to an antigen source of interest. The oligomerized complex may be administered as a single dose or multiple doses. The prophylactic or therapeutic dose may differ for different subjects and different therapeutic or prophylactic applications.

In one embodiment, the invention provides for a method of inducing an immune response by a sub-immunogenic amount of a vaccine composition, wherein the oligomerization facilitates the induction of an immune response by an amount of vaccine composition which is otherwise insufficient for inducing the immune response when used without oligomerization.

The present invention can also be used to increase biological activity of an immunotherapeutic and/or biologically active moiety by adding lectin to form an oligomer. As used herein, the term "immunotherapeutic moiety" refers to a molecule that is part of an immunotherapeutic complex. As used herein, the term "oligomer" refers to a complex of two or more units (*e.g.*, a complex comprising one lectin molecule and one HSP molecule, or a complex comprising one lectin molecule, and two HSP molecules, etc.). In one embodiment, an oligomer of the invention is a dimer comprising immunotherapeutic and/or biologically active moieties and lectin. In another embodiment, the immunotherapeutic and/or biologically active moieties form higher-order species, *i.e.*, an oligomer with more than two subunits. According to the invention, oligomerization of an immunotherapeutic and/or biologically active moiety increases its biological activities. In a preferred embodiment, the oligomer of the invention comprises a lectin molecule and gp96.

The present invention can also be used to increase vaccine uptake into antigen presenting cells (APCs) by receptor mediated events. While not limited by any theory, one of the possible explanations of the increased vaccine uptake into APCs by receptor mediated events is that more subunits of an oligomer in the immunologically and/or biologically active complex increases the interactions with the receptors on the antigen presenting cells compared to non-oligomerized complex wherein only one subunit interacts with the receptor. In a specific embodiment, the receptor is CD91.

The present invention can also be used to increase vaccine uptake into antigen presenting cells by non-receptor mediated events. Some immunologically and/or biologically active complexes comprising glycoproteins do not function through receptor-mediated events. Moreover, even when an immunologically and/or biologically active complex exerts some of its functions through receptor-mediated events, it may still exert the same or some other functions through non-receptor mediated events. For example, heat shock protein associated antigenic peptides can be taken up by antigen presenting cells by non-receptor mediated events including, but not limited to, pinocytosis, phagocytosis, and non-specific interactions with cell surface components and subsequent insertion and/or translocation ofHsp-peptide complexes across the cell membrane. According to the invention, oligomerization will increase such uptake. Oligomerization of biologically active moieties also increases their delivery to a target site.

In a specific embodiment, the present invention provides a method of delivering an Antigenic Molecule to an immune system of a subject comprising administering a molecular complex comprising a lectin and said Antigenic Molecule, wherein the Antigenic Molecule is either a naturally occurring glycoprotein or a protein that has been engineered to be a glycoprotein.

The present invention can further be used to improve the adjuvant capabilities of an immunotherapeutic moiety. As used herein, the term "adjuvant capabilities" refers to the ability of a nonantigenic substance that, in combination with an antigen, enhances immune response by, *e.g.*, inducing an inflammatory response, which leads to a local influx of immunoactive cells, such as antibody-forming cells and T lymphocytes. Immunotherapeutic moieties with adjuvant capabilities are used therapeutically in the preparation of vaccines, since they increase the production of antibodies against small quantities of antigen and lengthen the period of antibody production and/or the level of cellular immune response (*e.g.*, T lymphocytes). While not bound by any theory, oligomerization of the immunotherapeutic and/or biologically active moieties will increase their adjuvant capabilities. As used herein, the immunotherapeutic and/or biologically active moiety refers to a glycoprotein (including naturally occurring glycoprotein and chemically synthesized glycoproteins). In a preferred embodiment, the glycoprotein is a glycosylated heat shock protein including, but not limited to, gp96, GRP 170, Calreticulin, Bip (GRP78) or combinations thereof

The present invention can also be used to improve delivery of an immunotherapeutic and/or biologically active moiety. Not limited by any theory, oligomerization of an immunotherapeutic and/or biologically active moiety may enable the complex to exploit alternative and more effective pathways into target sites (*e.g.*, organs such as kidney, lung, liver, heart; tissues; or cells such as antigen presenting cells, red blood cells ("RBCs"), macrophages, lymphocytes, or cells that are involved in a particular signal transduction pathway).

Disclosed herein is a method of capturing secondary immunotherapeutic moieties and delivering said moiety into an antigen presenting cell via receptor mediated uptake in a subject, comprising administering to the subject a composition of a molecular complex, wherein said molecular complex comprises a first glycoprotein oligomerized with a second glycoprotein in the presence of lectin molecules, the amount of lectin present in the composition relative to the amount of glycoprotein is equal to or greater than 5ng, 10ng, 20ng, 30ng, 40ng, 50ng, 75ng, 100ng, or 200ng per microgram of glycoprotein. Preferably, the amount of lectin present in the composition relative to the amount of glycoprotein is 40ng to 1000ng, 50ng to 1000ng, 50ng to 500ng, 100ng to 250ng, or 150ng to 200ng lectin per microgram of glycoprotein. In some embodiments, the amount of lectin present in the composition relative to the amount of glycoprotein is equal to or less than 5ng per microgram of glycoprotein. Preferably, the amount of lectin present in the composition relative to the amount of glycoprotein is between 0.1ng to 5ng, 0.2ng to 4ng, 0.3ng to 3ng, 0.5ng to 2ng, or 0.1ng to 1ng lectin per microgram of glycoprotein. The molecular complex may further comprise one or more molecules, preferably peptides, that display antigenicity of an antigen of a type of cancer or of an antigen of an agent of an infectious disease. In one embodiment, the second glycoprotein is different from the first glycoprotein. In another embodiment, the first glycoprotein can be taken up by an antigen presenting cell, and the second glycoprotein normally will not be able to be taken up by an antigen presenting cell or other target cells as described previously, and the oligomerization of the first glycoprotein to the second glycoprotein enables the second glycoprotein being taken up by an antigen presenting cell.

In a specific embodiment, the first glycoprotein is a member of glycosylated heat shock protein, and the second glycoprotein is selected from the same group but is a different member. In another embodiment, the first glycoprotein is a member of glycosylated heat shock protein, and the second glycoprotein is not a heat shock protein. In another embodiment, the first glycoprotein is not a heat shock protein and the second glycoprotein is a heat shock protein. In yet another embodiment, neither the first nor the second glycoprotein is a heat shock protein. In a preferred embodiment, the first glycoprotein is further associated with an Antigenic Molecule that displays the antigenicity of an antigen of a type of cancer or of an antigen of an agent of an infectious disease. In another preferred embodiment, the second glycoprotein is further associated with an Antigenic Molecule that displays antigenicity of an antigen of a type of cancer or of an antigen of an agent of an infectious disease.

Disclosed herein is a method of treating or preventing a disease (*e.g.*, cancer, infectious disease, anemia, immunosuppressive conditions, enzyme deficiencies or hormone deficiencies) comprising administering to a subject in need thereof a therapeutically or prophylactically effective amount of a composition of the invention. In one embodiment, the composition comprises a purified noncovalent complex comprising a heat shock protein, an Antigenic Molecule, and a lectin molecule, wherein the heat shock protein and/or the Antigenic Molecule are glycosylated, wherein the Antigenic Molecule displays antigenicity of an antigen of said type of cancer or of an antigen of an agent of said infectious disease, and wherein the amount of lectin present in the composition relative to the amount of heat shock protein is equal to or greater than 5ng, 10ng, 20ng, 30ng, 40ng, 50ng, 75ng, 100ng, or 200ng per microgram of heat shock protein. Preferably, the amount of lectin present in the composition relative to the amount of heat shock protein is 40ng to 1000ng, 50ng to 1000ng, 50ng to 500ng, 100ng to 250ng, or 150ng to 200ng lectin per microgram of heat shock protein. In some embodiments, the amount of lectin present in the composition relative to the amount of heat shock protein is equal to or less than 5ng per microgram of heat shock protein. Preferably, the amount of lectin present in the composition relative to the amount of heat shock protein is between 0.1 ng to 5ng, 0.2ng to 4ng, 0.3ng to 3ng, 0.5ng to 2ng, or 0.1ng to 1ng lectin per microgram of heat shock protein.

In another embodiment, the composition is a pharmaceutical composition comprising a molecular complex and a pharmaceutically acceptable carrier, wherein the molecular complex comprises a heat shock protein, an Antigenic Molecule, and a lectin molecule, wherein the heat shock protein and/or the Antigenic Molecule are glycosylated, wherein the Antigenic Molecule displays antigenicity of an antigen of said type of cancer or of an antigen of an agent of said infectious disease, and wherein the amount of lectin present in the composition relative to the amount of heat shock protein is equal to or greater than 5ng; 10ng, 20ng, 30ng, 40ng, 50ng, 75ng, 100ng, or 200ng per microgram of heat shock protein. Preferably, the amount of lectin present in the composition relative to the amount of heat shock protein is 40ng to 1000ng, 50ng to 1000ng, 50ng to 500ng, 100ng to 250ng, or 150ng to 200ng lectin per microgram of heat shock protein. In some embodiments, the amount of lectin present in the composition relative to the amount of heat shock protein is equal to or less than 5ng per microgram of heat shock protein. Preferably, the amount of lectin present in the composition relative to the amount of heat shock protein is between 0.1ng to 5ng, 0.2ng to 4ng, 0.3ng to 3ng, 0.5ng to 2ng, or 0.1ng to 1ng lectin per microgram of heat shock protein.

Disclosed herein is a method of treating or preventing a disease (*e.g.*, cancer, infectious disease, anemia, immunosuppressive conditions, enzyme deficiencies or hormone deficiencies) comprising administering to a subject in need thereof (a) one or more molecular complexes of a glycoprotein (e.g., a glycosylated Hsp), a lectin, and a first Antigenic Molecule, wherein the first Antigenic Molecule displays the antigenicity of an antigen of a type of cancer or of an antigen of an agent of an infectious disease, and wherein the Antigenic Molecule may or may not be glycosylated, and wherein the amount of lectin present in the complexes relative to the amount of glycoprotein is equal to or greater than 5ng, 10ng, 20ng, 30ng, 40ng, 50ng, 75ng, 100ng, or 200ng per microgram of glycoprotein. Preferably, the amount of lectin present in the complexes relative to the amount of glycoprotein is 40ng to 1000ng, 50ng to 1000ng, 50ng to 500ng, 100ng to 250ng, or 150ng to 200ng lectin per microgram of glycoprotein. In some embodiments, the amount of lectin present in the complexes relative to the amount of glycoprotein is equal to or less than 5ng per microgram of glycoprotein. Preferably, the amount of lectin present in the complexes relative to the amount of glycoprotein is between 0.1ng to 5ng, 0.2ng to 4ng, 0.3ng to 3ng, 0.5ng to 2ng, or 0.1ng to 1ng lectin per microgram of glycoprotein; and (b) before, concurrently, or after administration of the molecular complex, administering to the subj ect a composition comprising antigen presenting cells sensitized *in vitro* with a sensitizing amount of a second molecular complex of glycoprotein associated with a lectin molecule and a second Antigenic Molecule, wherein said second Antigenic Molecule displays the antigenicity of a second antigen of said type of cancer or of a second antigen of an agent of said infectious disease. The APC can be selected from among those antigen presenting cells known in the art, including but not limited to macrophages, dendritic cells, B lymphocytes, and a combination thereof, and are preferably macrophages. In one embodiment, the first molecular complex is the same as the second molecule complex used to sensitize the APCs. In another embodiment, the first molecular complex is different from the second molecular complex used to sensitize the APCs. In a specific embodiment wherein the APCs and the compositions of the invention are administered concurrently, the APCs and composition of the invention can be present in the same composition (comprising APCs and the molecular complexes) or different composition. Adoptive immunotherapy (using sensitized APCs) according to the invention allows activation of immune antigen presenting cells by incubation with oligomerized molecule complexes. Preferably, prior to use of the cells *in vivo*, measurement of reactivity against the tumor or infectious agent *in vitro* is done. This *in vitro* boost followed by clonal selection and/or expansion, and patient administration constitutes a useful therapeutic/prophylactic strategy. In a preferred embodiment, the glycoprotein is a glycosylated heat shock protein.

In a preferred embodiment, the immunologically and/or biologically active moiety of the molecular complex, *e.g.* a heat shock protein complexed to a protein that displays antigenicity of an antigen of a type of cancer or of an antigen of an agent of an infectious disease, is autologous to the subject; that is, it is isolated from the cells of the subject himself (*e.g.*, prepared from tumor biopsies of the patient when the treatment of cancer is desired). Alternatively, the molecular complex can be allogeneic to the subject to whom a composition of the molecular complex of the invention is administered. The molecular complex can be prepared *in vitro*, *e.g*., from cultured cells that recombinantly express a heat shock protein. The heat shock protein can be a naturally glycosylated heat shock protein (*e.g.*, gp96, GRP170, Calreticulin, Bip (GRP78), or a combination thereof), a non-naturally glycosylated heat shock protein that is converted into a glycoprotein, or a combination thereof.

Exogenous antigens and fragments and derivatives thereof for use in complexing with glycoproteins to generate the specific complexes can be selected from among those known in the art, as well as those readily identified by standard immunoassays known in the art by the ability to bind antibody or MHC molecules (antigenicity) or generate immune response (immunogenicity). Non-limiting examples of exogenous antigens include, but are not limited to, cancer specific antigens, cancer associated antigens, antigens expressed by a cell line or a subject that is infected with a pathogen or transfected with a gene encoding a tumor specific antigen, a tumor associated antigen, or an antigen of an agent of a pathogen. Specific complexes of glycoproteins and Antigenic Molecules can be isolated from cancer or precancerous tissue of a patient, or from a cancer cell line, or can be produced *in vitro* (as is necessary in the embodiment in which an exogenous antigen is used as the Antigenic Molecule).

The present invention further provides kits comprising a plurality of containers each comprising a pharmaceutical formulation or composition comprising a dose of molecular complexes of the invention sufficient for a single immunogenic administration. The invention also provides kits comprising a container comprising an immunoactive glycoprotein or a complex thereof, and a container comprising lectin. Optionally, instructions for formulating the oligomerized complexes according to the methods of the invention can be included in the kits.

In a specific embodiment, the present invention relates to compositions for use in prevention and treatment of primary and metastatic neoplastic diseases.

The therapeutic regimens and pharmaceutical compositions of the invention may be used with another therapeutic or prophylactic therapy, such as other immune response enhancers or biological response modifiers including, but not limited to, cytokines, agonists or antagonists of various ligands, receptors and signal transduction molecules, immunostimulatory nucleic acids, and adjuvants. In accordance with this aspect of the invention, the compositions of the invention are administered in combination therapy with one or more of these immune response enhancers or biological response modifiers. In another embodiment, the compositions of the invention are administered with radiotherapy or one or more chemotherapeutic agents for the treatment of cancer.

In addition to cancer therapy, the compositions of the invention can be utilized for the prevention of a variety of cancers, *e.g.*, in subjects who are predisposed as a result of familial history or in subjects with an enhanced risk to cancer due to environmental factors.

Specific therapeutic regimens, pharmaceutical compositions, and kits are provided by the invention.

### 5.1. Heat Shock Protein Preparations

The molecular complex of the invention comprises a lectin associated with a glycosylated heat shock protein complexed to an Antigenic Molecule (*e.g.*, one or more proteins (including peptides and polypeptides) that display antigenicity of an antigen of a type of cancer or of an antigen of an agent of an infectious disease). Heat shock protein or Hsp-Antigenic Molecule complexes can be prepared separately, and lectin added as an additional step to promote the oligomerization of the Hsps or Hsp-Antigenic Molecule complexes.

Heat shock proteins (Hsps) are referred to interchangeably herein as stress proteins and can be selected from among any cellular protein that satisfies the following criteria: it is a protein whose intracellular concentration increases when a cell is exposed to a stressful stimuli; it is capable of binding other proteins; it is capable of releasing the bound proteins in the presence of adenosine triphosphate (ATP) or low pH (*e.g.*, pH of 1, 2, 3, 4, 5, or 6); and it is a protein showing at least 35% homology with any cellular protein having any of the above properties. Non-limiting examples of heat shock proteins are described in Srivastava, Nature Reviews (Immunology) 2:185-194 (2002) . In some embodiments, only naturally glycosylated heat shock proteins are used, which include but not limited to, gp96, calreticulin, GRP 170, Bip (GRP78) or noncovalent or covalent complexes thereof In some embodiments of the invention, a heat shock protein is converted into a glycosylated heat shock protein by adding one or more glycosylation sites that are not present in the native sequences of the heat shock protein followed by the addition of carbohydrates.

In accordance with the methods described herein, each specific Hsp-antigenic complex (Hsp-protein complex) employed in a composition of the invention is preferably purified in the range of 60 to 100 percent of the total mg protein, or at least 20%, 30%, 40%, 50%, 60%, 70%, 80% or 90% of the total mg protein. In another embodiment, each specific Hsp-Antigenic Molecule complex is purified to apparent homogeneity, as assayed by sodium dodecyl sulfate-polyacrylamide gel electrophoresis.

In a preferred embodiment, non-covalent complexes of Hsps (*e.g.*, hsp70, hsp90 and gp96) with proteins are purified and prepared postoperatively from tumor cells obtained from the cancer patient for use as specific complexes in the compositions of the invention.

In accordance with the methods described herein, immunogenic or antigenic proteins (including peptides and polypeptides) that are endogenously complexed to Hsps or MHC antigens can be used as specific Antigenic Molecules. For example, such proteins may be prepared which stimulate cytotoxic T cell responses against different tumor antigens (*e.g.*, tyrosinase, gp100, melan-A, gp75, mucins, etc.) and viral proteins including, but not limited to, proteins of immunodeficiency virus type I (HIV-I), human immunodeficiency virus type II (HIV-II), hepatitis type A, hepatitis type B, hepatitis type C, influenza, Varicella, adenovirus, herpes simplex type I (HSV-I), herpes simplex type II (HSV-II), rinderpest, rhinovirus, echovirus, rotavirus, respiratory syncytial virus, papilloma virus, papova virus, cytomegalovirus, echinovirus, arbovirus, huntavirus, coxsackie virus, mumps virus, measles virus, rubella virus and polio virus. In the embodiment wherein the Antigenic Molecules are proteins complexed to Hsps *in vivo*, the complexes can be isolated from cells. Alternatively, the complexes can be produced *in vitro* from purified preparations each of Hsps and Antigenic Molecules. In some embodiments, the Antigenic Molecules are exogenous antigens and fragments and derivatives thereof.

In the embodiments wherein one wishes to use Antigenic Molecules by complexing to Hsps *in vitro,* Hsps can be purified for such use from the endogenous Hsp-protein complexes in the presence of ATP or low pH (pH 1, 2, 3, 4, 5, or 6). Hsps can also be chemically synthesized or recombinantly produced. The protocols described herein may be used to isolate specific Hsp-protein complexes or the Hsps alone, from any eukaryotic cells, for example, tissues, isolated cells, or immortalized eukaryote cell lines infected with a pre-selected intracellular pathogen, tumor cells or tumor cell lines.

### 5.1.1 Preparation and Purification of Hsp 70 or Hsp70-protein Complexes

The purification of Hsp70-protein complexes has been described previously, see, for example, Udono et al., 1993, J. Exp. Med. 178:1391-1396. A procedure that may be used, presented by way of example but not limitation, is described below.

Initially, tumor cells are suspended in 3 volumes of 1X Lysis buffer consisting of 30mM sodium bicarbonate pH7.5, 1mM PMSF. Then, the pellet is sonicated, on ice, until >99% cells are lysed as determined by microscopic examination. As an alternative to sonication, the cells may be lysed by mechanical shearing by homogenizing the cells in a Dounce homogenizer until >95% cells are lysed.

Then the lysate is centrifuged at 1,000g for 10 minutes to remove unbroken cells, nuclei and other cellular debris. The resulting supernatant is recentrifuged at 100,000g for 90minutes, the supernatant harvested and then mixed with Con A Sepharose equilibrated with phosphate buffered saline (PBS) containing 2mM Ca2+ and 2mM Mg2+. When the cells are lysed by mechanical shearing the supernatant is diluted with an equal volume of 2X lysis buffer prior to mixing with Con A Sepharose. The supernatant is then allowed to bind to the Con A Sepharose for 2-3 hours at 4°C. The material that fails to bind is harvested and dialyzed for 36 hours (three times, 100 volumes each time) against 10mM Tris-Acetate pH.7.5, 0.1 mM EDTA, 10mM NaCl, 1mM PMSF. Then the dialyzate is centrifuged at 17,000 rpm (Sorval1 SS34 rotor) for 20 minutes. Then the resulting supernatant is harvested and applied to a Mono Q FPLC column equilibrated in 20mM Tris-Acetate pH 7.5, 20mM NaCl, 0.1mM EDTA and 15mM 2-mercaptoethanol. The column is then developed with a 20mM to 500mM NaCl gradient and then eluted fractions fractionated by sodium dodecyl sulfate-polyacrylamide gel electrophoresis (SDS-PAGE) and characterized by immunoblotting using an appropriate anti-HSP70 antibody (such as from clone N27F3-4, from StressGen).

Fractions strongly immunoreactive with the anti-HSP70 antibody are pooled and the HSP70-protein complexes precipitated with ammonium sulfate; specifically with a 50%-70% ammonium sulfate cut. The resulting precipitate is then harvested by centrifugation at 17,000 rpm (SS34 Sorvall rotor) and washed with 70% ammonium sulfate. The washed precipitate is then solubilized and any residual ammonium sulfate removed by gel filtration on a SephadexR G25 column (Pharmacia). If necessary the HSP70 preparation thus obtained can be repurified through the Mono Q FPLC Column as described above.

The Hsp70-protein complex can be purified to apparent homogeneity using this method. Typically 1mg of Hsp70-protein complex can be purified from 1g of cells/tissue.

An improved method for purification of HSP70 comprises contacting cellular proteins with ATP or a nonhydrolyzable analog of ATP affixed to a solid substrate, such that HSP70 in the lysate can bind to the ATP or nonhydrolyzable ATP analog, and eluting the bound HSP70. A preferred method uses column chromatography with ATP affixed to a solid substratum (*e.g.*, ATP-agarose). The resulting HSP70 preparations are higher in purity and devoid of contaminating proteins. The HSP70 yields are also increased significantly by about more than 10 fold.

Alternatively, chromatography with nonhydrolyzable analogs of ADP, instead of ATP, can be used for purification of HSP70-protein complexes. See Peng et al., Journal of Immunological Methods 204:13-21 (1997) . By way of example but not limitation, purification of HSP70 free of proteins by ATP-agarose chromatography can be carried out as follows: Meth A sarcoma cells (500 million cells) are homogenized in hypotonic buffer and the lysate is centrifuged at 100,000 g for 90 minutes at 4°C. The supernatant is applied to an ATP-agarose column. The column is washed in buffer and is eluted with 5 column volumes of 3 mM ATP. The HSP70 elutes in fractions 2 through 10 of the total 15 fractions which elute. The eluted fractions are analyzed by SDS-PAGE. The HSP70 can be purified to apparent homogeneity using this procedure.

Alternatively, Hsp70 or Hsp70-protein can be purified by using immunoaffinity purification methods known in the art. For example, Hsp70-specific scFv column can be used. (See Arnold-Schild et al., Cancer Research, 2000, 60(15):4175-4178 . Although Arnold-Schild describes a gp96-specific scFv column, a Hsp70-specific scFv column can be produced by the equivalent method). By way of example but not limitation, the purification using Hsp70-specific scFv column can be carried out as follows: scFv anti-Hsp70 are coupled to CNBr-activated Sepharose. The samples containing Hsp70 or Hsp70-protein complex are applied to the scFv anti-Hsp70 column. After extensive washing with PBS, Hsp70 or Hsp70-protein can be eluted with PBS, 1.3 M NaCl, or 10mM sodium phosphate (pH 7.2).

Separation of the protein from an hsp70-protein complex can be performed in the presence of ATP or low pH. These two methods may be used to elute the protein from an hsp70-protein complex. The first approach involves incubating an hsp70-protein complex preparation in the presence of ATP. The other approach involves incubating an hsp70-protein complex preparation in a low pH buffer (*e.g.*, pH is 1, 2, 3, 4, 5, or 6). These methods and any others known in the art may be applied to separate the HSP and protein from an Hsp-protein complex.

### 5.1.2 Preparation and Purification of Hsp90 or Hsp90-protein Complexes

A procedure that can be used, presented by way of example and not limitation, is as follows:

Initially, human or mammalian cells are suspended in 3 volumes of 1X Lysis buffer consisting of 5mM sodium phosphate buffer (pH 7), 150mM NaCl, 2mM CaCl₂, 2mM MgCl₂ and 1mM phenyl methyl sulfonyl fluoride (PMSF). Then, the pellet is sonicated, on ice, until >99% cells are lysed as determined by microscopic examination. As an alternative to sonication, the cells may be lysed by mechanical shearing and in this approach the cells typically are resuspended in 30mM sodium bicarbonate (pH 7.5), 1mM PMSF, incubated on ice for 20 minutes and then homogenized in a Dounce homogenizer until >95% cells are lysed.

Then the lysate is centrifuged at 1,000g for 10 minutes to remove unbroken cells, nuclei and other cellular debris. The resulting supernatant is recentrifuged at 100,000g for 90 minutes, the supernatant harvested and then mixed with Con A Sepharose™ equilibrated with PBS containing 2mM Ca²⁺ and 2mM Mg²⁺. When the cells are lysed by mechanical shearing the supernatant is diluted with an equal volume of 2X Lysis buffer prior to mixing with Con A Sepharose™. The supernatant is then allowed to bind to the Con A Sepharose™ for 2-3 hours at 4°C. The material that fails to bind is harvested and dialyzed for 36 hours (three times, 100 volumes each time) against 20mM Sodium Phosphate (pH 7.4), 1mM EDTA, 250mM NaCl, 1mM PMSF. Then the dialyzate is centrifuged at 17,000 rpm (Sorvall SS34 rotor) for 20 minutes. Then the resulting supernatant is harvested and applied to a Mono Q FPLC™ ion exchange chromatographic column (Pharmacia) equilibrated with dialysis buffer. The proteins are then eluted with a salt gradient of 200mM to 600mM NaCl.

The eluted fractions are fractionated by SDS-PAGE and fractions containing the hsp90-protein complexes identified by immunoblotting using an anti-hsp90 antibody such as 3G3 (Affinity Bioreagents). Hsp90-protein complexes can be purified to apparent homogeneity using this procedure. Typically, 150-200 µg of hsp90-protein complex can be purified from 1g of cells/tissue.

Alternatively, Hsp90 or Hsp90-protein can be purified by using any immunoaffinity purification methods known in the art. For example, Hsp90-specific scfv column can be used. (See Arnold-Schild, incorporates herein by its entirety. Although Arnold-Schild describes a gp96-specific scFv column, a Hsp90-specific scFv column can be produced by the equivalent method). By way of example but not limitation, the purification using Hsp90-specific scFv column can be carried out as follows: scFv anti-Hsp90 are coupled to CNBr-activated Sepharose. The samples containing Hsp90 or Hsp90-protein complex are applied to the scFv anti-Hsp70 column. After extensive washing with PBS, Hsp90 or Hsp90-protein can be eluted with PBS, 1.3 M NaCl, or 10mM sodium phosphate (pH 7.2).

Separation of the protein from an hsp90-protein complex can be performed in the presence of ATP or low pH. These two methods may be used to elute the protein from an hsp90-protein complex. The first approach involves incubating an hsp90-protein complex preparation in the presence of ATP. The other approach involves incubating an hsp90-protein complex preparation in a low pH buffer (*e.g.*, pH is 1, 2, 3, 4, 5, or 6). These methods and any others known in the art may be applied to separate the HSP and protein from an Hsp-protein complex.

### 5.1.3 Preparation and Purification of Gp96 or Gp96-protein Complexes

A procedure that can be used, presented by way of example and not limitation, is as follows:

A pellet of human or mammalian cells is resuspended in 3 volumes of buffer consisting of 30mM sodium bicarbonate buffer (pH 7.5) and 1mM PMSF and the cells allowed to swell on ice 20 minutes. The cell pellet is then homogenized in a Dounce homogenizer (the appropriate clearance of the homogenizer will vary according to each cell type) on ice until >95% cells are lysed.

The lysate is centrifuged at 1,000g for 10 minutes to remove unbroken cells, nuclei and other debris. The supernatant from this centrifugation step is then recentrifuged at 100,000g for 90 minutes. The gp96-protein complex can be purified either from the 100,000 pellet or from the supernatant.

When purified from the supernatant, the supernatant is diluted with equal volume of 2X lysis buffer and the supernatant mixed for 2-3 hours at 4°C with Con A Sepharose™ equilibrated with PBS containing 2mM Ca²⁺ and 2mM Mg²⁺. Then, the slurry is packed into a column and washed with 1X lysis buffer until the OD₂₈₀ drops to baseline. Then, the column is washed with 1/3 column bed volume of 10% α-methyl mannoside (α-MM) dissolved in PBS containing 2mM Ca²⁺ and 2mM Mg²⁺, the column sealed with a piece of parafilm, and incubated at 37°C for 15 minutes. Then the column is cooled to room temperature and the parafilm removed from the bottom of the column. Five column volumes of the α-MM buffer are applied to the column and the eluate analyzed by SDS-PAGE. Typically the resulting material is about 60-95% pure, however this depends upon the cell type and the tissue-to-lysis buffer ratio used. Then the sample is applied to a Mono Q FPLC™ ion exchange chromatographic column (Pharmacia) equilibrated with a buffer containing 5mM sodium phosphate (pH 7). The proteins are then eluted from the column with a 0-1M NaCl gradient and the gp96 fraction elutes between 400mM and 550mM NaCl.

The procedure, however, may be modified by two additional steps, used either alone or in combination, to consistently produce apparently homogeneous gp96-protein complexes. One optional step involves an ammonium sulfate precipitation prior to the Con A purification step and the other optional step involves DEAE-Sepharose™ purification after the Con A purification step and in lieu of the Mono Q FPLC™ step.

In the first optional step, described by way of example as follows: the supernatant resulting from the 100,000g centrifugation step is brought to a final concentration of 50% ammonium sulfate by the addition of ammonium sulfate. The ammonium sulfate is added slowly while gently stirring the solution in a beaker placed in a tray of ice water. The solution is stirred from about ½ to 12 hours at 4°C and the resulting solution centrifuged at 6,000 rpm (Sorvall SS34 rotor). The supernatant resulting from this step is removed, brought to 70% ammonium sulfate saturation by the addition of ammonium sulfate solution, and centrifuged at 6,000 rpm (Sorvall SS34 rotor). The resulting pellet from this step is harvested and suspended in PBS containing 70% ammonium sulfate in order to rinse the pellet. This mixture is centrifuged at 6,000 rpm (Sorvall SS34 rotor) and the pellet dissolved in PBS containing 2mM Ca²⁺ and Mg²⁺. Undissolved material is removed by a brief centrifugation at 15,000 rpm (Sorvall SS34 rotor). Then, the solution is mixed with Con A Sepharose™ and the procedure followed as before.

In the second optional step, described by way of example as follows: the gp96 containing fractions eluted from the Con A column are pooled and the buffer exchanged for 5mM sodium phosphate buffer (pH 7), 300mM NaCl by dialysis, or preferably by buffer exchange on a Sephadex G25 column. After buffer exchange, the solution is mixed with DEAE-Sepharose™ previously equilibrated with 5mM sodium phosphate buffer (pH 7), 300mM NaCl. The protein solution and the beads are mixed gently for 1 hour and poured into a column. Then, the column is washed with 5mM sodium phosphate buffer (pH 7), 300mM NaCl, until the absorbance at 280nm drops to baseline. Then, the bound protein is eluted from the column with five volumes of 5mM sodium phosphate buffer (pH 7), 700mM NaCl. Protein containing fractions are pooled and diluted with 5mM sodium phosphate buffer (pH 7) in order to lower the salt concentration to 175mM. The resulting material then is optionally applied to the Mono Q FPLC™ ion exchange chromatographic column (Pharmacia) equilibrated with 5mM sodium phosphate buffer (pH 7) and the protein that binds to the Mono Q FPLC™ ion exchange chromatographic column (Pharmacia) are then eluted from the column with a 0-1M NaCl gradient. The gp96 fraction elutes between 400mM and 550mM NaCl.

It is appreciated, however, that one skilled in the art may assess, by routine experimentation, the benefit of incorporating the second optional step into the purification protocol. In addition, it is appreciated also that the benefit of adding each of the optional steps will depend upon the source of the starting material.

When the gp96 fraction is isolated from the 100,000g pellet, the pellet is suspended in 5 volumes of PBS containing either 1% sodium deoxycholate or 1% octyl glucopyranoside (but without the Mg²⁺ and Ca²⁺) and incubated on ice for 1 hour. The suspension is centrifuged at 20,000g for 30 minutes and the resulting supernatant dialyzed against several changes of PBS (also without the Mg²⁺ and Ca²⁺) to remove the detergent. The dialysate is centrifuged at 100,000g for 90 minutes, the supernatant harvested, and calcium and magnesium are added to the supernatant to give final concentrations of 2mM, respectively. Then the sample is purified by either the unmodified or the modified method for isolating gp96-protein complex from the 100,000g supernatant, see above.

The gp96-protein complexes can be purified to apparent homogeneity using this procedure. About 10-20µg of gp96 can be isolated from 1g cells/tissue.

Alternatively, gp96 or gp96-protein can be purified by using any immunoaffinity purification methods known in the art. For example, Hsp96-specific scFv column can be used. (See Arnold-Schild, Cancer Research, 2000, 60(15):4175-4178 ).

Separation of the protein from a gp96-protein complex can be performed in the presence of ATP or low pH (e.g., pH 1, 2, 3, 4, 5, or 6). These two methods may be used to elute the protein from a gp96-protein complex. The first approach involves incubating a gp96-protein complex preparation in the presence of ATP. The other approach involves incubating a gp96-protein complex preparation in a low pH buffer. These methods and any others known in the art may be applied to separate the HSP and protein from an Hsp-protein complex.

### 5.1.4 Preparation and Purification of Hsp 110-protein Complexes

A procedure, described by Wang et al., 2001, J. Immunol. 16G(1):490-7, that can be used, presented by way of example and not limitation, is as follows:

A pellet (40-60 ml) of cell or tissue, e.g., tumor cell tissue, is homogenized in 5 vol of hypotonic buffer (30 mN sodium bicarbonate, pH7.2, and protease inhibitors) by Dounce homogenization. The lysate is centrifuged at 4,500 x g and then 100,000 x g for 2 hours. If the cells or tissues are of hepatic origin, the resulting supernatant is was first applied to a blue Sepharose column (Pharmacia) to remove albumin. Otherwise, the resulting supernatant is applied to a Con A-Sepharose column (Pharmacia Biotech, Piscataway, NJ) previously equilibrated with binding buffer (20mM Tris-HCl, pH 7.5; 100mM NaCl; 1mM MgCl₂; 1 mM CaCl₂; 1 mM MnCl₂; and 15 mM 2-ME). The bound proteins are eluted with binding buffer containing 15% α-D-o-methylmannoside (Sigma, St. Louis, MO).

Con A-Sepharose unbound material is first dialyzed against a solution of 20 mM Tris-HCl, pH 7.5; 100 mM NaCl; and 15 mM 2-ME, and then applied to a DEAE-Sepharose column and eluted by salt gradient from 100 to 500 mM NaCl. Fractions containing hsp110 are collected, dialyzed, and loaded onto a Mono Q (Pharmacia) 10/10 column equilibrated with 20mM Tris-HCl, pH 7.5; 200 mM NaCl; and 15 mM 2-ME. The bound proteins are eluted with a 200-500 mM NaCl gradient. Fractions are analyzed by SDS-PAGE followed by immunoblotting with an Ab for Hsp110, as described by Wang et al., 1999, J. Immunol. 162:3378. Pooled fractions containing Hsp110 are concentrated by Centriplus (Amicon, Beverly, MA) and applied to a Superose 12 column (Pharmacia). Proteins are eluted by 40 mM Tris-HCl, pH 8.0; 150 mM NaCl; and 15 mM 2-ME with a flow rate of 0.2 ml/min.

Alternatively, Hsp110 or Hsp110-protein can be purified by using any immunoaffinity purification methods known in the art. For example, Hsp 110-specific scFv column can be used. (See Arnold-Schild et al., Cancer Research, 2000, 60(15):4175-4178, Although Arnold-Schild describes a gp96-specific scFv column, a Hsp110-specific scFv column can be produced by the equivalent method). By way of example but not limitation, the purification using Hsp110-specific scFv column can be carried out as follows: scFv anti-Hsp110 are coupled to CNBr-activated Sepharose. The samples containing Hsp110 or Hsp110-protein complex are applied to the scFv anti-Hsp110 column. After extensive washing with PBS, Hsp110 or Hsp110-protein can be eluted with PBS, 1.3 M NaCl, or 10mM sodium phosphate (pH 7.2).

### 5.1.5 Preparation and Purification of Grp 170-protein Complexes

A procedure, described by Wang et al., 2001, J. Immunol. 166(1):490-7, that can be used, presented by way of example and not limitation, is as follows:

A pellet (40-60 ml) of cell or tissue, e.g., tumor cell tissue, is homogenized in 5 vol of hypotonic buffer (30 mN sodium bicarbonate, pH7.2, and protease inhibitors) by Dounce homogenization. The lysate is centrifuged at 4,500 x g and then 100,000 x g for 2 hours. If the cells or tissues are of hepatic origin, the resulting supernatant is was first applied to a blue Sepharose column (Pharmacia) to remove albumin. Otherwise, the resulting supernatant is applied to a Con A-Sepharose column (Pharmacia Biotech, Piscataway, NJ) previously equilibrated with binding buffer (20mM Tris-HCl, pH 7.5; 100mM NaCl; 1mM MgCl₂; 1 mM CaCl₂; 1 mM MnCl₂; and 15 mM 2-ME). The bound proteins are eluted with binding buffer containing 15% α-D-o-methylmannoside (Sigma, St. Louis, MO).

Con A-Sepharose-bound material is first dialyzed against 20 mM Tris-HCl, pH 7.5, and 150 mM NaCl and then applied to a Mono Q column and eluted by a 150 to 400 mM NaCl gradient. Pooled fractions are concentrated and applied on the Superose 12 column (Pharmacia). Fractions containing homogeneous grp170 are collected.

Alternatively, GRP170 or GRP170-protein can be purified by using any immunoaffinity purification methods known in the art. For example, GRP170-specific scFv column can be used. (See Arnold-Schild et al., Cancer Research, 2000, 60(15):4175-4178. Although Arnold-Schild describes a gp96-specific scFv column, a Hsp170-specific scFv column can be produced by the equivalent method). By way of example but not limitation, the purification using Hsp170-specific scFv column can be carried out as follows: scFv anti-Hsp170 are coupled to CNBr-activated S epharose. The samples containing Hsp170 or Hsp170-protein complex are applied to the scFv anti-Hsp170 column. After extensive washing with PBS, Hsp170 or Hsp170-protein can be eluted with PBS, 1.3 M NaCl, or 10mM sodium phosphate (pH 7.2).

### 5.1.6 Recombinant Expression of Hsps

Methods known in the art can be utilized to recombinantly produce Hsps. A nucleic acid sequence encoding a heat shock protein can be inserted into an expression vector for propagation and expression in host cells.

An expression construct, as used herein, refers to a nucleotide sequence encoding an HSP operably associated with one or more regulatory regions which enables expression of the HSP in an appropriate host cell. "Operably-associated" refers to an association in which the regulatory regions and the HSP sequence to be expressed are joined and positioned in such a way as to permit transcription, and ultimately, translation.

The regulatory regions necessary for transcription of the HSP can be provided by the expression vector. A translation initiation codon (ATG) may also be provided if the HSP gene sequence lacking its cognate initiation codon is to be expressed. In a compatible host-construct system, cellular transcriptional factors, such as RNA polymerase, will bind to the regulatory regions on the expression construct to effect transcription of the modified HSP sequence in the host organism. The precise nature of the regulatory regions needed for gene expression may vary from host cell to host cell. Generally, a promoter is required which is capable of binding RNA polymerase and promoting the transcription of an operably-associated nucleic acid sequence. Such regulatory regions may include those 5' non-coding sequences involved with initiation of transcription and translation, such as the TATA box, capping sequence, CAAT sequence, and the like. The non-coding region 3' to the coding sequence may contain transcriptional termination regulatory sequences, such as terminators and polyadenylation sites.

In order to attach DNA sequences with regulatory functions, such as promoters, to the HSP gene sequence or to insert the HSP gene sequence into the cloning site of a vector, linkers or adapters providing the appropriate compatible restriction sites may be ligated to the ends of the cDNAs by techniques well known in the art (Wu et al., 1987, Methods in Enzymol, 152:343-349). Cleavage with a restriction enzyme can be followed by modification to create blunt ends by digesting back or filling in single-stranded DNA termini before ligation. Alternatively, a desired restriction enzyme site can be introduced into a fragment of DNA by amplification of the DNA by use of PCR with primers containing the desired restriction enzyme site.

An expression construct comprising an HSP sequence operably associated with regulatory regions can be directly introduced into appropriate host cells for expression and production of Hsp-peptide complexes without further cloning. *See,* for example, U.S. Patent No. 5,580,859. The expression constructs can also contain DNA sequences that facilitate integration of the HSP sequence into the genome of the host cell, *e.g.*, via homologous recombination. In this instance, it is not necessary to employ an expression vector comprising a replication origin suitable for appropriate host cells in order to propagate and express the HSP in the host cells.

A variety of expression vectors may be used including, but not limited to, plasmids, cosmids, phage, phagemids or modified viruses. Typically, such expression vectors comprise a functional origin of replication for propagation of the vector in an appropriate host cell, one or more restriction endonuclease sites for insertion of the HSP gene sequence, and one or more selection markers. The expression vector must be used with a compatible host cell which may be derived from a prokaryotic or an eukaryotic organism including but not limited to bacteria, yeasts, insects, mammals and humans.

The coding sequence of a heat shock protein can also be altered by adding one or more glycosylation sites that are not present in the native sequence. Glycosylation of polypeptides is typically either N-linked or O-linked. The term "N-linked" refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. The term "O-linked glycosylation" refers to the attachment of one of the sugars such as N-aceylgalactosamine, galactose, or xylose to a hydroxylamino acid, most commonly serine or threonine, although 5-hydroxyproline or 5-hydroxylysine may also be used.

Addition of glycosylation sites may be accomplished by various means. For example, the alteration may be made by changes at the DNA level, particularly by mutating the DNA encoding an interested protein or peptide at preselected bases such that codons are generated that will translate into the desired amino acids. The DNA mutation(s) may be made using methods described in U.S. Pat. No. 5,364,934.

For long term, high yield production of properly processed Hsp or Hsp-protein complexes, stable expression in mammalian cells is preferred. Cell lines that stably express HSP or Hsp-protein complexes may be engineered by using a vector that contains a selectable marker. By way of example but not limitation, following the introduction of the expression constructs, engineered cells may be allowed to grow for 1-2 days in an enriched media, and then are switched to a selective media. The selectable marker in the expression construct confers resistance to the selection and optimally allows cells to stably integrate the expression construct into their chromosomes and to grow in culture and to be expanded into cell lines. Such cells can be cultured for a long period of time while HSP is expressed continuously.

The recombinant cells may be cultured under standard conditions of temperature, incubation time, optical density and media composition. However, conditions for growth of recombinant cells may be different from those for expression of HSPs and antigenic proteins. Modified culture conditions and media may also be used to enhance production of the Hsp. For example, recombinant cells containing HSPs with their cognate promoters may be exposed to heat or other environmental stress, or chemical stress. Any techniques known in the art may be applied to establish the optimal conditions for producing HSP or HSP-protein complexes.

### 5.1.7 Peptide Synthesis

An alternative to producing Hsp by recombinant techniques is peptide synthesis. For example, an entire Hsp, or a peptide corresponding to a portion of an Hsp can be synthesized by use of a peptide synthesizer. Conventional peptide synthesis or other synthetic protocols well known in the art may be used.

Peptides having the amino acid sequence of a HSP or a portion thereof may be synthesized by solid-phase peptide synthesis using procedures similar to those described by Merrifield, 1963, J. Am. Chem. Soc., 85:2149. During synthesis, N-α-protected amino acids having protected side chains are added stepwise to a growing polypeptide chain linked by its C-terminal and to an insoluble polymeric support i.e., polystyrene beads. The peptides are synthesized by linking an amino group of an N-α-deprotected amino acid to an α-carboxyl group of an N-α-protected amino acid that has been activated by reacting it with a reagent such as dicyclohexylcarbodiimide. The attachment of a free amino group to the activated carboxyl leads to peptide bond formation. The most commonly used N-α-protecting groups include Boc which is acid labile and Fmoc which is base labile. Details of appropriate chemistries, resins, protecting groups, protected amino acids and reagents are well known in the art and so are not discussed in detail herein (See, Atherton, et al., 1989, Solid Phase Peptide Synthesis: A Practical Approach, IRL Press, and Bodanszky, 1993, Peptide Chemistry, A Practical Textbook, 2nd Ed., Springer-Verlag).

One or more glycosylation sites that are not present in the native sequence can also be added during the synthesis. For example, the amino acid sequence of an interested protein or peptide can be altered such that it contains one or more of the tripeptide sequences (for N-linked glycosylation sites) described in section 5.1.6. The alteration may also be made by the addition of, or substitution by, one or more serine or threonine residues to the native sequence (for O-linked glycosylation sites).

Purification of the resulting Hsp is accomplished using conventional procedures, such as preparative HPLC using gel permeation, partition and/or ion exchange chromatography. The choice of appropriate matrices and buffers are well known in the art and so are not described in detail herein.

### 5.2. Antigenic Molecules

The following subsections provide an overview of proteins (including peptides and polypeptides) that are useful as antigenic/immunogenic components of the molecular complexes of the invention, and how such proteins can be identified, *e.g*., for use in recombinant expression of the peptides for *in vitro* complexing of HSPs and Antigenic Molecules. However, in the practice of the present invention, the identity of the Antigenic Molecule(s) of the molecular complex need not be known, for example, when the HSP/peptide complex is purified directly from a cancerous cell or from a tissue infected with a pathogen.

Antigenic epitopes of an Antigenic Molecule can be identified using methods known in the art. As used herein, an "epitope" refers to a region of an antigenic peptide that binds or that is predicted to bind an antibody or major histocompatibility (MHC) molecule of a subject. Preferably, the epitope, upon binding to the MHC molecule, stimulates in vivo an immune response to the antigenic peptide. The peptides of the present invention contain epitopes that are predicted to be capable of binding selected MHC molecules and inducing an immune response. The antigenic peptide epitopes of the invention comprise conserved residues involved in binding proteins encoded by MHC alleles. The antigenic peptide epitopes predicted to bind MHC class I molecules are typically between 8 to 10 residues, while antigenic peptide epitopes predicted to bind MHC class II molecules are typically in the range of 10 to 20 residues.

Non-limiting examples of specific human MHC alleles predicted to bind the antigenic peptides of the invention include the following Human Leukocyte Antigen (HLA) molecules: HLA-A1, HLA-A201, HLA-A203, HLA-A3, HLA-A2402, HLA-A26, HLA-B702, HLA-B8, HLA-B1510, HLA-B2705, HLA-B2709, HLA-B4402, and HLA-B5101 (Rammensee, et al., Immunogenetics 41, 178-228, 1995). The capacity to bind MHC molecules can be measured in a variety of different ways, such as by inhibition of antigen presentation (Sette, et al., J. Immunol. 141:3893, 1991), in vitro assembly assays (Townsend, et al., Cell 62:285, 1990), and FACS based assays using mutated cells, such as RMA.S (Melief, et al., Eur. J. Immunol. 21:2963, 1991).

In some embodiments, the Antigenic Molecule is a glycoprotein, and forms oligomers in the presence of lectin. In other embodiments, the Antigenic Molecule is not a glycoprotein (but a different member of the complex is a glycoprotein). In some embodiments, the Antigenic Molecule are isolated from cell lysates. In some embodiments, the Antigenic Molecules are synthesized. In some embodiments, an Antigenic Molecule is engineered into a glycoprotein, *e.g.*, by adding one or more glycosylation sites that are not present in the native sequence of the Antigenic Molecule followed by the addition of carbohydrates. See, *e.g.*, Scott et al., Proc. Natl. Acad. Sci. USA 89:5398-5402 (1992).

### 5.2.1 Isolation of Antigenic/Immunogenic Components

It has been found that antigenic proteins (including peptides and polypeptides) and/or components can be eluted from Hsp complexes either in the presence of ATP or low pH. These experimental conditions may be used to isolate proteins and/or antigenic components from cells which may contain potentially useful antigenic determinants. Once isolated, the amino acid sequence of each antigenic protein may be determined using conventional amino acid sequencing methodologies. Such Antigenic Molecules can then be produced by chemical synthesis or recombinant methods, purified, and complexed to Hsps *in vitro* to form the Hsp complexes of the invention.

Similarly, it has been found that potentially immunogenic proteins may be eluted from MHC-protein complexes using techniques well known in the art (Falk, K. et al., 1990 Nature 348:248-251; Elliott, T. et al., 1990, Nature 348:195-197; Falk, K. et al., 1991, Nature 351:290-296).

Thus, potentially immunogenic or antigenic proteins may be isolated from either endogenous Hsp-protein complexes or endogenous MHC-protein complexes for use subsequently as Antigenic Molecules, by complexing *in vitro* to a glycoprotein, *e.g.*, a glycosylated heat shock protein, to form the molecular complexes of the invention. Exemplary protocols for isolating peptides and/or antigenic components from these complexes are known in the art are described hereinbelow.

### 5.2.2 Peptides From Hsp-Peptide Complexes

Two methods may be used to elute the peptide from an Hsp-peptide complex.

One approach involves incubating the Hsp-peptide complex in the presence of ATP. The other approach involves incubating the complexes in a low pH buffer.

Briefly, the complex of interest is centrifuged through a Centricon 10 assembly (Millipore) to remove any low molecular weight material loosely associated with the complex. The large molecular weight fraction may be removed and analyzed by SDS-PAGE while the low molecular weight may be analyzed by HPLC as described below. In the ATP incubation protocol, the stress protein-peptide complex in the large molecular weight fraction is incubated with 10mM ATP for 30 minutes at room temperature. In the low pH (*e.g.*, pH of 1, 2, 3, 4, 5, or 6) protocol, acetic acid or trifluoroacetic acid (TFA) is added to the stress protein-peptide complex to give a final concentration of 10% (vol/vol) and the mixture incubated at room temperature or in a boiling water bath or any temperature in between, for 10 minutes (See, Van Bleek, et al., 1990, Nature 348:213-216; and Li, et al., 1993, EMBO Journal 12:3143-3151).

The resulting samples are centrifuged through a Centricon 10 assembly as mentioned previously. The high and low molecular weight fractions are recovered. The remaining large molecular weight stress protein-peptide complexes can be reincubated with ATP or low pH (*e.g*., pH of 1, 2, 3, 4, 5, or 6) to remove any remaining peptides.

The resulting lower molecular weight fractions are pooled, concentrated by evaporation and dissolved in 0.1% TFA. The dissolved material is then fractionated by reverse phase high pressure liquid chromatography (HPLC) using for example a VYDAC C18 reverse phase column equilibrated with 0.1% TFA. The bound material is then eluted at a flow rate of about 0.8 ml/min by developing the column with a linear gradient of 0 to 80% acetonitrile in 0.1% TFA. The elution of the peptides can be monitored by OD210 and the fractions containing the peptides collected.

### 5.2.3 Peptides from MHC-peptide Complexes

The isolation of potentially immunogenic peptides from MHC molecules is well known in the art and so is not described in detail herein (See, Falk et al., 1990, Nature 348:248-251; Rotzsche at al., 1990, Nature 348:252-254; Elliott et al., 1990, Nature 348:191-197; Falk et al., 1991, Nature 351:290-296; Demotz et al., 1989, Nature 343:682-684; Rotzsche et al., 1990, Science 249:283-287) .

Briefly, MHC-peptide complexes may be isolated by a conventional immunoaffinity procedure. The peptides then may be eluted from the MHC-peptide complex by incubating the complexes in the presence of about 0.1% TFA in acetonitrile. The eluted peptides may be fractionated and purified by reverse phase HPLC, as before.

The amino acid sequences of the eluted peptides may be determined either by manual or automated amino acid sequencing techniques well known in the art. Once the amino acid sequence of a potentially protective peptide has been determined the peptide may be synthesized in any desired amount using conventional peptide synthesis or other protocols well known in the art.

Peptides having the same amino acid sequence as those isolated above may be synthesized by solid-phase peptide synthesis using procedures similar to those described by Merrifield, 1963, J. Am. Chem. Soc., 85:2149. During synthesis, N-α-protected amino acids having protected side chains are added stepwise to a growing polypeptide chain linked by its C-terminal and to an insoluble polymeric support i.e., polystyrene beads. The peptides are synthesized by linking an amino group of an N-α-deprotected amino acid to an α-carboxy group of an N-α-protected amino acid that has been activated by reacting it with a reagent such as dicyclohexylcarbodiimide. The attachment of a free amino group to the activated carboxyl leads to peptide bond formation. The most commonly used N-α-protecting groups include Boc which is acid labile and Fmoc which is base labile.

Briefly, the C-terminal N-α-protected amino acid is first attached to the polystyrene beads. The N-α-protecting group is then removed. The deprotected α-amino group is coupled to the activated α-carboxylate group of the next N-α-protected amino acid. The process is repeated until the desired peptide is synthesized. The resulting peptides are then cleaved from the insoluble polymer support and the amino acid side chains deprotected. Longer peptides can be derived by condensation of protected peptide fragments. Details of appropriate chemistries, resins, protecting groups, protected amino acids and reagents are well known in the art and so are not discussed in detail herein (See, Atherton, et al., 1989, Solid Phase Peptide Synthesis: A Practical Approach, IRL Press, and Bodanszky, 1993, Peptide Chemistry, A Practical Textbook, 2nd Ed., Springer-Verlag).

Purification of the resulting peptides is accomplished using conventional procedures, such as preparative HPLC using gel permeation, partition and/or ion exchange chromatography. The choice of appropriate matrices and buffers are well known in the art and so are not described in detail herein.

### 5.2.4 Exogenous Antigenic Molecules

Molecules that display the antigenicity of a known antigen of a pathogen or of a tumor-specific or tumor-associated antigen of a cancer type, e.g. antigens or antigenic portions thereof, can be selected for use as Antigenic Molecules, for complexing to glycoprotein and/or lectin, from among those known in the art or determined by immunoassay to be able to bind to antibody or MHC molecules (antigenicity) or generate immune response (immunogenicity). To determine immunogenicity or antigenicity by detecting binding to antibody, various immunoassays known in the art can be used, including but not limited to competitive and non-competitive assay systems using techniques such as radioimmunoassays, ELISA (enzyme linked immunosorbent assay), "sandwich" immunoassays, immunoradiometric assays, gel diffusion precipitin reactions, immunodiffusion assays, *in vivo* immunoassays (using colloidal gold, enzyme or radioisotope labels, for example), western blots, immunoprecipitation reactions, agglutination assays (*e.g.*, gel agglutination assays, hemagglutination assays), complement fixation assays, immunofluorescence assays, protein A assays, and immunoelectrophoresis assays, etc. In one embodiment, antibody binding is detected by detecting a label on the primary antibody. In another embodiment, the primary antibody is detected by detecting binding of a secondary antibody or reagent to the primary antibody. In a further embodiment, the secondary antibody is labelled. Many means are known in the art for detecting binding in an immunoassay and are envisioned for use. In one embodiment for detecting immunogenicity, T cell-mediated responses can be assayed by standard methods, *e.g., in vitro* cytoxicity assays or *in vivo* delayed-type hypersensitivity assays.

Potentially useful antigens or derivatives thereof for use as Antigenic Molecules can also be identified by various criteria, such as the antigen's involvement in neutralization of a pathogen's infectivity (wherein it is desired to treat or prevent infection by such a pathogen) (Norrby, 1985, Summary, in Vaccines 85, Lerner, et al. (eds.), Cold Spring Harbor Laboratory, Cold Spring Harbor, New York, pp. 388-389), type or group specificity, recognition by patients' antisera or immune cells, and/or the demonstration of protective effects of antisera or immune cells specific for the antigen. In addition, where it is desired to treat or prevent a disease caused by pathogen, the antigen's encoded epitope should preferably display a small or no degree of antigenic variation in time or amongst different isolates of the same pathogen.

Preferably, where it is desired to treat or prevent cancer, tumor-specific (*i.e.*, expressed in tumor cells) or tumor associated antigens (*i.e.*,relatively overexpressed in tumor cells) or fragments or derivatives thereof are used. For example, such tumor specific or tumor-associated antigens include but are not limited to KS 1/4 pan-carcinoma antigen (Perez and Walker, 1990, J. Immunol. 142:3662-3667; Bumal, 1988, Hybridoma 7(4):407-415); ovarian carcinoma antigen (CA125) (Yu, et al., 1991, Cancer Res. 51(2):468-475); prostatic acid phosphate (Tailer, et al., 1990, Nucl. Acids Res. 18(16):4928); prostate specific antigen (Henttu and Vihko, 1989, Biochem. Biophys. Res. Comm. 160(2):903-910; Israeli, et al., 1993, Cancer Res. 53:227-230); melanoma-associated antigen p97 (Estin, et al., 1989, J. Natl. Cancer Inst. 81(6):445-446); melanoma antigen gp75 (Vijayasardahl, et al., 1990, J. Exp. Med. 171(4):1375-1380); high molecular weight melanoma antigen (Natali, et al., 1987, Cancer 59:55-63) and prostate specific membrane antigen. Other exogenous antigens that may be complexed to a glycoprotein include portions or proteins that are mutated at a high frequency in cancer cells, such as oncogenes (*e.g.*, ras, in particular mutants of ras with activating mutations, which only occur in four amino acid residues (12, 13, 59 or 61) (Gedde-Dahl et al., 1994, Eur. J. Immunol. 24(2):410-414)) and tumor suppressor genes (*e.g.*, p53, for which a variety of mutant or polymorphic p53 peptide antigens capable of stimulating a cytotoxic T cell response have been identified (Gnjatic et al., 1995, Eur. J. Immunol. 25(6):1638-1642).

In a specific embodiment, an antigen or fragment or derivative thereof specific to a tumor is selected for complexing to HSPs to form a HSP-antigen complex for oligomerization and then administration to a patient having that tumor.

Preferably, where it is desired to treat or prevent viral diseases, molecules comprising epitopes of known viruses are used. For example, such antigenic epitopes may be prepared from viruses including, but not limited to, hepatitis type A, hepatitis type B, hepatitis type C, influenza, varicella, adenovirus, herpes simplex type I (HSV-I), herpes simplex type II (HSV-II), rinderpest, rhinovirus, echovirus, rotavirus, respiratory syncytial virus, papilloma virus, papova virus, cytomegalovirus, echinovirus, arbovirus, huntavirus, coxsackie virus, mumps virus, measles virus, rubella virus, polio virus, human immunodeficiency virus type I (HIV-I), and human immunodeficiency virus type II (HIV-II). Preferably, where it is desired to treat or prevent bacterial infections, molecules comprising epitopes of known bacteria are used. For example, such antigenic epitopes may be prepared from bacteria including, but not limited to, mycobacteria rickettsia, mycoplasma, neisseria and legionella.

Preferably, where it is desired to treat or prevent protozoal infections, molecules comprising epitopes of known protozoa are used. For example, such antigenic epitopes may be prepared from protozoa including, but not limited to, leishmania, kokzidioa, and trypanosoma.

Preferably, where it is desired to treat or prevent parasitic infections, molecules comprising epitopes of known parasites are used. For example, such antigenic epitopes may be from parasites including, but not limited to, chlamydia and rickettsia.

### 5.3. In Vitro Production of Hsp/Antigenic Molecule Complexes

In an embodiment in which specific complexes ofHsps and the Antigenic Molecules with which they are endogenously associated *in vivo* are not employed, complexes of Hsps to Antigenic Molecules are produced *in vitro.* As will be appreciated by those skilled in the art, the Antigenic Molecules either isolated by the aforementioned procedures or chemically synthesized or recombinantly produced may be reconstituted with a variety of purified natural or recombinant stress proteins *in vitro* to generate immunogenic non-covalent stress protein-Antigenic Molecule complexes. Alternatively, exogenous antigens or antigenic or immunogenic fragments or derivatives thereof can be complexed to stress proteins for use in the immunotherapeutic or prophylactic vaccines of the invention. A preferred, exemplary protocol for complexing a stress protein and an Antigenic Molecule *in vitro* is discussed below.

Prior to complexing, the Hsps are pretreated with ATP or low pH (*e.g*., pH of 1, 2, 3, 4, 5, or 6) to remove any peptides that may be associated with the Hsp of interest. When the ATP procedure is used, excess ATP is removed from the preparation by the addition of apyranase as described by Levy, et al., 1991, Cell 67:265-274. When the low pH procedure is used, the buffer is readjusted to neutral pH by the addition of pH modifying reagents.

The Antigenic Molecules (1µg) and the pretreated Hsp (9µg) are admixed to give an approximately 5 Antigenic Molecule: 1 stress protein molar ratio. Then, the mixture is incubated for 15 minutes to 3 hours at 4° to 45°C in a suitable binding buffer such as one containing 20mM sodium phosphate, pH 7.2, 350mM NaCl, 3mM MgCl₂ and 1mM phenyl methyl sulfonyl fluoride (PMSF). The preparations are centrifuged through a Centricon 10 assembly (Millipore) to remove any unbound peptide. The association of the peptides with the stress proteins can be assayed by SDS-PAGE. This is the preferred method for *in vitro* complexing of peptides isolated from MHC-peptide complexes of peptides disassociated from endogenous hsp-peptide complexes.

In an alternative embodiment of the invention, preferred for producing complexes of hsp70 to exogenous Antigenic Molecules such as proteins, 5-10 micrograms of purified Hsp is incubated with equal molar quantities of the Antigenic Molecule in 20mM sodium phosphate buffer pH 7.5, 0.5M NaCl, 3mM MgCl₂ and 1mM ADP in a volume of 100 microliter at 37°C for 1 hr. This incubation mixture is further diluted to 1ml in phosphate-buffered saline.

In an alternative embodiment of the invention, preferred for producing complexes of gp96 or hsp90 to peptides, 5-10 micrograms of purified gp96 or hsp90 is incubated with equimolar or excess quantities of the antigenic peptide in a suitable buffer such as one containing 20mM sodium phosphate buffer pH 7.5, 0.5M NaCl, 3nM MgCl2 at 37-65°C for 5-20 min. This incubation mixture is allowed to cool to room temperature and centrifuged one or more times if necessary, through a Centricon 10 assembly (Millipore) to remove any unbound peptide.

Following complexing, the immunogenic stress protein-Antigenic Molecule complexes can optionally be assayed *in vitro* using for example the mixed lymphocyte target cell assay (MLTC) described below. Once immunogenic complexes have been isolated they can be optionally characterized further in animal models using the preferred administration protocols and excipients discussed below.

As an alternative to non-covalent complexes ofHsps and Antigenic Molecules, Antigenic Molecules may be covalently attached to Hsps prior to administration according to the methods of the present invention. Hsp-Antigenic Molecule complexes are preferably cross-linked after their purification from cells or tissues as described in Sections 5.1.1. to 5.1.4. In one embodiment, Hsps are covalently coupled to Antigenic Molecules by chemical crosslinking. Chemical crosslinking methods are well known in the art. For example, in a preferred embodiment, glutaraldehyde crosslinking may be used. Glutaradehyde crosslinking has been used for formation of covalent complexes of peptides and hsps (see Barrios et al., 1992, Eur. J. Immunol. 22: 1365-1372). Preferably, 1-2 mg of Hsp-Antigenic Molecule complex is crosslinked in the presence of 0.002% glutaraldehyde for 2 hours. Glutaraldehyde is removed by dialysis against phosphate buffered saline (PBS) overnight (Lussow et al., 1991, Eur. J. Immunol. 21: 2297-2302).

In another embodiment, the Hsp and specific antigen(s) are crosslinked by ultraviolet (UV) crosslinking.

In another embodiment, recombinant fusion proteins, comprised of a heat shock protein sequence and an antigenic peptide sequence, are produced. To produce such a recombinant fusion protein, an expression vector is constructed using nucleic acid sequences encoding a heat shock protein fused to sequences encoding an antigen, using recombinant methods known in the art, such as those described in Section 5.1.6., above. Hsp-antigenic peptide fusions are then expressed and isolated. Such fusion proteins can be used to elicit an immune response (Suzue et al., 1997, Proc. Natl. Acad. Sci. U.S.A. 94: 13146-51). By specifically designing the antigenic peptide portion of the molecule, such fusion proteins can be used to elicit an immune response and in immunotherapy against cancer or infectious diseases.

### 5.4. Oligomerization of Biological Active Complexes

In accordance with the present invention, lectin or lectin-like molecules form a molecular complex with immunologically and/or biologically active glycoproteins. In some embodiments, the glycoprotein is a heat shock protein. In some embodiments, the glycoprotein is not a heat shock protein. In some embodiments, the glycoprotein is an Antigenic Molecule. In some embodiments, the glycoprotein is not an Antigenic Molecule. In certain embodiments, the molecular complex may further comprise one or more moieties that are not a glycoprotein. In a specific embodiment, the molecular complex further comprises an antigenic moiety. In a specific embodiment, the molecular complex comprises a lectin molecule associated with a glycoprotein and a heat shock protein. In another embodiment, the molecular complex comprises a lectin associated with a glycoprotein, a heat shock protein, and an Antigenic Molecule. In yet another specific embodiment, the molecular complex comprises a lectin molecule associated with glycosylated heat shock protein and an Antigenic Molecule, wherein said glycosylated heat shock protein can be a naturally occurring heat shock protein (*e.g.*, gp96, GRP170, Calreticulin, and Bip (GRP78)), or a non-naturally occurring heat shock protein that is converted into a glycoprotein by adding one or more glycosylation sites that are not present in the native amino acid sequences comprising the heat shock protein followed by addition of carbohydrate groups. A complex of glycoprotein (*e.g.*, heat shock protein) and Antigenic Molecule can be noncovalent or covalent. Preferably, a lectin binds noncovalently to one or more glycoproteins or the complex of glycoprotein (*e.g.*, heat shock protein) and one or more other moieties (*e.g.*, Antigenic Molecules). In a preferred embodiment, the lectin molecule is a mannose-binding lectin molecule including, but not limited to, those listed in Table 1. More preferably, the mannose-binding lectin molecule is Concanavalin A (Con A).

In a preferred embodiment, the number of the lectins present in a molecular complex is more than the number of glycoproteins that are present in the molecular complex. In some embodiments, the molar ratio between glycoprotein and lectin is 3:1, 2:1, or 1:1. In a preferred embodiment, the lectin is Con A in the form of a tetramer, and for each tetramer Con A molecule, there are three, two, or one glycoprotein(s) attached.

A molecular complex of the invention may be prepared by different methods. In certain embodiments, the molecular complex is formed *in vivo* and the molecular complex is isolated from cells. In certain embodiments, the molecular complex is produced *in vitro* from purified preparations of one or more components of the molecular complex. The techniques that can be used in preparation of the molecular complex of the invention depend on the nature of the complex. Non-limiting examples of preparing components of a molecular complex of the invention are given in sections 5.1.- 5.3., *supra.* Other techniques that are well-known in the art of protein purification may also be exploited, which include but are not limited to, separation by adsorption, *e.g.*, chromatography, ion exchange, inorganic adsorbents, hydrophobic adsorbents, immobilized metal affinity chromatography, immunoadsorbents, dye ligand chromatography, affinity elution from ion exchangers and other adsorbents; gel filtration; electrophoretic methods; liquid phase partitioning, and ultrafiltration. *See* Scopes, 1994, PROTEIN PURIFICATION, PRINCIPLES AND PRACTICE, 3rd ed., Springer.

Lectins can be added at various stages of preparation of a molecular complex, *e.g.*, prior to, or subsequent to, various chromatography steps used to purify the immunologically and/or biologically active moieties (*e.g.*, Hsp-protein complexes). Lectins can be added in various forms, such as powder or a liquid solution. Different lectins can be used in combination to prepare the oligomers of the invention. Lectins can also be cross-linked by using methods well known in the art before adding to a molecular preparation of the invention to promote oligomerization. In some embodiments, adding lectins is one of the steps in purifying the molecular complex. In some embodiments, other components of the molecular complex is prepared first, and lectins are added to the final product to promote the oligomerization of the molecular complex. In a preferred embodiment, the molecular complex of the invention comprises a heat shock protein, an Antigenic Molecule, and lectin, wherein the amount of lectin added is sufficient to promote the oligomerization of the preparation, and to produce a final product(s) wherein the amount of lectin present in the final product(s) relative to the amount of heat shock protein is equal to or greater than 5ng, 10ng, 20ng, 30ng, 40ng, 50ng, 75ng, 100ng, or 200ng per microgram of heat shock protein. Preferably, the amount of lectin present in the final product(s) relative to the amount of heat shock protein is 40ng to 1000ng, 50ng to 1000ng, 50ng to 500ng, 100ng to 250ng, or 150ng to 200ng lectin per microgram of heat shock protein. In some embodiments, the amount of lectin added is sufficient to promote oligomerization of the preparation and to produce a final product or products wherein the amount of lectin present in final product(s) relative to the amount of heat shock protein is equal to or less than 5ng per microgram of heat shock protein. Preferably, the amount of lectin present in the final product(s) relative to the amount of heat shock protein is between 0.1ng to 5ng, 0.2ng to 4ng, 0.3ng to 3ng, 0.5ng to 2ng, or 0.1ng to 1ng lectin per microgram of glycoprotein.

Any assay known in the art can be used to confirm the oligomerization of the molecular complex. For example, SEC profiling can be used, wherein an oligomerized molecular complex is eluted in a different fraction compared to an un-oligomerized molecular complex in size exclusion column purification. (for example, see section 7)

Any assay known in the art can be used to confirm the presence of the lectin molecule in the oligomer, including but is not limited to, any immuno-based methods, such as ELISA, radioimmunoassays, "sandwich" immunoassays, immunoradiometric assays, immunodiffusion assays, immunofluorescence assays, immunoelectrophoresis assays, etc. all these assays are well known in the art and are not descibed in detail here. (for an example of Con A specific ELISA, see section 6)

The correlation of oliogmerization of the molecular complex to its *in vivo* and *in vitro* activities can be demonstrated by any assay known in the art, including but is not limited to, those assays that detect the biological activity and/or immunogenicity of the molecular complexes of the invention (such as but not limited to those described in section *5.5, infra.),* assays that test the *in vitro* acitivities of the molecular complexes of the invention (such as but not limited to, representation assays, *e.g.,* CD71 *in vitro* representation assay, Meth A *in vitro* representation assay, and CT26 *in vitro* antigen representation assay, see Example sections, *infra*), and assays that test *in vivo* actitivities of the molecular complexes of the invention using animal models (*e.g., in vivo* Meth A tumor inhibition assay, see Example sections, *infra*). In a preferred embodiment, the representation assay or a tumor inhibition assay is used.

### 5.5. Determination Of Immunogenicity of the Molecular Complexes

Optionally, the molecular complexes of the invention can be assayed for immunogenicity using any method known in the art. By way of example but not limitation, one of the following procedures can be used.

### 5.5.1 The MLTC Assay

Briefly, mice are injected with an amount of the molecular complex of the invention, using any convenient route of administration. As a negative control, other mice are injected with, e.g., molecular complexes that are do not comprising oligomerized glycoproteins. Cells known to contain specific antigens, e.g. tumor cells or cells infected with an agent of an infectious disease, may act as a positive control for the assay. The mice are injected twice, 7-10 days apart. Ten days after the last immunization, the spleens are removed and the lymphocytes released. The released lymphocytes may be re-stimulated subsequently *in vitro* by the addition of dead cells that expressed the antigen of interest.

For example, 8x10⁶ immune spleen cells may be stimulated with 4x10⁴ mitomycin C treated or γ-irradiated (5-10,000 rads) cells containing the antigen of interest (or cells transfected with an appropriate gene, as the case may be) in 3ml RPMI medium containing 10% fetal calf serum. In certain cases 33% secondary mixed lymphocyte culture supernatant may be included in the culture medium as a source of T cell growth factors (*See,* Glasebrook, et al., 1980, J. Exp. Med. 151:876). To test the primary cytotoxic T cell response after immunization, spleen cells may be cultured without stimulation. In some experiments spleen cells of the immunized mice may also be re-stimulated with antigenically distinct cells, to determine the specificity of the cytotoxic T cell response.

Six days later the cultures are tested for cytotoxicity in a 4 hour ⁵¹Cr-release assay (*See,* Palladino, et al., 1987, Cancer Res. 47:5074-5079 and Blachere, at al., 1993, J. Immunotherapy 14:352-356). In this assay, the mixed lymphocyte culture is added to a target cell suspension to give different effector:target (E:T) ratios (usually 1:1 to 40:1). The target cells are prelabelled by incubating 1x10⁶ target cells in culture medium containing 20 mCi ⁵¹Cr/ml for one hour at 37°C. The cells are washed three times following labeling. Each assay point (E:T ratio) is performed in triplicate and the appropriate controls incorporated to measure spontaneous ⁵¹Cr release (no lymphocytes added to assay) and 100% release (cells lysed with detergent). After incubating the cell mixtures for 4 hours, the cells are pelletted by centrifugation at 200g for 5 minutes. The amount of ⁵¹Cr released into the supernatant is measured by a gamma counter. The percent cytotoxicity is measured as cpm in the test sample minus spontaneously released cpm divided by the total detergent released cpm minus spontaneously released cpm.

In order to block the MHC class I cascade a concentrated hybridoma supernatant derived from K-44 hybridoma cells (an anti-MHC class I hybridoma) is added to the test samples to a final concentration of 12.5%.

### 5.5.2 CD4⁺ T Cell Proliferation Assay

Primary T cells are obtained from spleen, fresh blood, or CSF and purified by centrifugation using FICOLL-PAQUE PLUS (Pharmacia, Upsalla, Sweden) essentially as described by Kruse and Sebald, 1992, EMBO J. 11: 3237-3244. The peripheral blood mononuclear cells are incubated for 7-10 days with a lysate of cells expressing an Antigenic Molecule. Antigen presenting cells may, optionally be added to the culture 24 to 48 hours prior to the assay, in order to process and present the antigen in the lysate. The cells are then harvested by centrifugation, and washed in RPMI 1640 media (GibcoBRL, Gaithersburg, Md.). 5x10⁴ activated T cells/well (PHA-blasts) are in RPMI 1640 media containing 10% fetal bovine serum, 10 mM HEPES, pH 7.5, 2 mM L-glutamine, 100 units/ml penicillin G, and 100 µg/ml streptomycin sulphate in 96 well plates for 72 hrs at 37°C., pulsed with 1 µCi ³H-thymidine (DuPont NEN, Boston, Mass.)/well for 6 hrs, harvested, and radioactivity measured in a TOPCOUNT scintillation counter (Packard Instrument Co., Meriden, Conn.).

### 5.5.3 Antibody Response Assay

In a certain embodiment of the invention, the immunogenicity of a molecular complex of the invention comprising oligomerized glycoproteins is determined by measuring antibodies produced in response to the administration with the complex. In one mode of the embodiment, microtitre plates (96-well Immuno Plate II, Nunc) are coated with 50 µl/well of a 0.75 µg/ml solution of a purified, non-complexed form of the antigenic peptide used in the molecular complex (*e.g.* Aβ42) in PBS at 4°C for 16 hours and at 20°C for 1 hour. The wells are emptied and blocked with 200 µl PBS-T-BSA (PBS containing 0.05% (v/v) TWEEN 20 and 1% (w/v) bovine serum albumin) per well at 20°C for 1 hour, then washed 3 times with PBS-T. Fifty µl/well of plasma or CSF from a animal (such as a model mouse or a human patient) that has received the molecular complex of the invention is applied at 20°C for 1 hour, and the plates are washed 3 times with PBS-T. The anti-peptide antibody activity is then measured calorimetrically after incubating at 20°C for 1 hour with 50µl/well of sheep anti-mouse or anti-human immunoglobulin, as appropriate, conjugated with horseradish peroxidase (Amersham) diluted 1:1,500 in PBS-T-BSA and (after 3 further PBS-T washes as above) with 50 µl of an o-phenylene diamine (OPD)-H₂O₂ substrate solution. The reaction is stopped with 150 µl of 2M H₂SO₄ after 5 minutes and absorbance is determined in a Kontron SLT-210 photometer (SLT Lab-instr., Zurich, Switzerland) at 492 nm (ref. 620 nm).

### 5.5.4 Cytokine Detection Assay

The CD4+ T cell proliferative response to HSP-complexes of the invention may be measured by detection and quantitation of the levels of specific cytokines. In one embodiment, for example, intracellular cytokines may be measured using an IFN-γ detection assay to test for immunogenicity of a complex of the invention. In an example of this method, peripheral blood mononuclear cells from a subject treated with a lectin-HSP-peptide complex are stimulated with peptide antigens of a given tumor or with peptide antigens of an agent of infectious disease. Cells are then stained with T cell-specific labeled antibodies detectable by flow cytometry, for example FITC-conjugated anti-CD8 and PerCP-labeled anti-CD4 antibodies. After washing, cells are fixed, permeabilized, and reacted with dye-labeled antibodies reactive with human IFN-γ (PE- anti-IFN-γ). Samples are analyzed by flow cytometry using standard techniques.

Alternatively, a filter immunoassay, the enzyme-linked immunospot assay (ELISPOT) assay, may be used to detect specific cytokines surrounding a T cell. In one embodiment, for example, a nitrocellulose-backed microtiter plate is coated with a purified cytokine-specific primary antibody, *i.e.*, anti-IFN-γ, and the plate is blocked to avoid background due to nonspecific binding of other proteins. A sample of mononuclear blood cells, containing cytokine-secreting cells, obtained from a subject treated with a lectin-HSP-peptide complex, which sample is diluted onto the wells of the microtitre plate. A labeled, e.g., biotin-labeled, secondary anti-cytokine antibody is added. The antibody cytokine complex can then be detected, *i.e.* by enzyme-conjugated streptavidin - cytokine-secreting cells will appear as "spots" by visual, microscopic, or electronic detection methods.

### 5.5.5 Tetramer Assay

In another embodiment, the "tetramer staining" assay (Altman et al., 1996, Science 274: 94-96) may be used to identify antigen-specific T-cells. For example, in one embodiment, an MHC molecule containing a specific peptide antigen, such as a tumor-specific antigen, is multimerized to make soluble peptide tetramers and labeled, for example, by complexing to streptavidin. The MHC-peptide antigen complex is then mixed with a population of T cells obtained from a subject treated with a lectin-HSP- complex. Biotin is then used to stain T cells which express the antigen of interest, i.e., the tumor-specific antigen.

### 5.6. Combination With Adoptive Immunotherapy

Adoptive immunotherapy refers to a therapeutic approach for treating cancer or infectious diseases in which immune cells are administered to a host with the aim that the cells mediate either directly or indirectly specific immunity to tumor cells and/or antigenic components or regression of the tumor or treatment of infectious diseases, as the case may be. (See U.S. Patent No. 5,985,270, issued November 16, 1999 .) As an optional step, in accordance with the methods described herein, APC are sensitized with the molecular complex of the invention and used in adoptive immunotherapy.

In one embodiment, antigen presenting cells (APC) for use in adoptive immunotherapy are sensitized with lectin associated HSPs complexed with antigenic proteins prepared in accordance with the methods described herein. The complexes can be produced by complexing lectin-Hsp to antigenic proteins that are derived from at least 50% of the different proteins or at least 100 different proteins present in antigenic cells or viral particles that express an antigenic determinant of an agent that causes the infectious disease. The complexes can also be produced by (a) subjecting a protein preparation derived from cells of said type of cancer to either digestion with a protease or contact with ATP, guanidium hydrochloride, and/or acid, to generate a population of antigenic peptides, and (b) complexing the population of antigenic peptides to lectin-Hsp.

In another embodiment, therapy by administration of *in vitro* complexed antigenic peptides, HSPs and lectins prepared by the methods of the invention may be combined with adoptive immunotherapy using APC sensitized by HSP-antigenic peptide complexes prepared by any method known in the art (see *e.g.*, U.S. Patent No. 5,985,270) in which the antigenic peptides display the desired antigenicity (*e.g.*, of the type of cancer or pathogen). The sensitized APC can be administered alone, in combination with the *in vitro* complexed proteins, HSPs and lectins, or before or after administration of the complexed proteins, HSPs and lectins. In particular, the use of sensitized APC to prevent and treat cancer can further comprise administering to the subject an amount, effective for said treatment or prevention, of complexes comprising lectin and heat shock protein complexed to antigenic proteins, wherein said complexes were produced as described above. Similarly, the use of sensitized APC in treating or preventing a type of infectious disease, can further comprise administering to the subject an amount, effective for said treatment or prevention, of complexes comprising lectin associated heat shock proteins and antigenic proteins.

Furthermore, the mode of administration of the *in vitro* produced molecular complexes of the invention can be varied, including but not limited to, *e.g.*, subcutaneously, intravenously or intramuscularly, although intradermally is preferred. In another specific embodiment, adoptive immunotherapy by administration of the antigen presenting cells sensitized with complexes made according to the present invention can be combined with therapy by administration of lectin associated with HSP-Antigenic Molecule complexes prepared by any method known in the art (see *e.g.*, U.S. Patent No. 5,750,119, 5,837,251, 5,961,979, 5,935,576, PCT publications WO 94/14976 or WO 99/50303) in which the Antigenic Molecules display the desired antigenicity (*e.g.*, of the type of cancer or pathogen).

### 5.6.1 Obtaining Macrophages and Antigen Presenting Cells

The antigen-presenting cells, including but not limited to macrophages, dendritic cells and B-cells, are preferably obtained by production *in vitro* from stem and progenitor cells from human peripheral blood or bone marrow as described by Inaba, K., et al., 1992, J. Exp. Med., 176:1693-1702.

APC can be obtained by any of various methods known in the art. In a preferred aspect human macrophages are used, obtained from human blood cells. By way of example but not limitation, macrophages can be obtained as follows:

Mononuclear cells are isolated from peripheral blood of a patient (preferably the patient to be treated), by Ficoll-Hypaque gradient centrifugation and are seeded on tissue culture dishes which are pre-coated with the patient's own serum or with other AB+ human serum. The cells are incubated at 37°C for 1 hour, then non-adherent cells are removed by pipetting. To the adherent cells left in the dish, is added cold (4°C) 1 mM EDTA in phosphate-buffered saline and the dishes are left at room temperature for 15 minutes. The cells are harvested, washed with RPMI buffer and suspended in RPMI buffer. Increased numbers of macrophages may be obtained by incubating at 37°C with macrophage-colony stimulating factor (M-CSF); increased numbers of dendritic cells may be obtained by incubating with granulocyte-macrophage-colony stimulating factor (GM-CSF) as described in detail by Inaba, K., et al., 1992, J. Exp. Med., 176:1693-1702.

### 5.6.2 Sensitizing of Macrophages

### and Antigen Presenting Cells with Molecular Complexes of the Invention

APC are sensitized with molecular complexes of the invention, preferably by incubating the cells *in vitro* with the complexes. The APC are sensitized with complexes comprising glycoproteins/glycopeptides associated with lectin and Antigenic Molecules by incubating *in vitro* with the complexes at 37°C for 15 minutes to 24 hours. By way of example but not limitation, 5x10⁴ macrophages can be incubated with desired concentration of Hsp, starting at 150 µg/ml and titrating down, at 37°C for 15 minutes-24 hours in 1 ml plain RPMI medium. The cells are washed three times and resuspended in a physiological medium preferably sterile, at a convenient concentration (e.g., 1x10⁷/ml) for injection in a patient. Preferably, the patient into which the sensitized APCs are injected is the patient from which the APC were originally isolated (autologous embodiment).

Optionally, the ability of sensitized APC to stimulate, for example, the antigen-specific, class I-restricted cytotoxic T-lymphocytes (CTL) can be monitored by their ability to stimulate CTLs to release tumor necrosis factor, and by their ability to act as targets of such CTLs.

### 5.6.3 Reinfusion of Sensitized APC

The molecular complex-sensitized APC are reinfused into the patient systemically, preferably intravenously, by conventional clinical procedures. These activated cells are reinfused, preferentially by systemic administration into the autologous patient. Patients generally receive from about 10⁶ to about 10¹² sensitized macrophages, depending on the condition of the patient. In some regimens, patients may optionally receive in addition a suitable dosage of a biological response modifier including but not limited to the cytokines IFN-α, IFN-γ, IL-2, IL-4, IL-6, TNF or other cytokine growth factor.

### 5.7. Passive Immunotherapy

The compositions of the invention can also be used for passive immunotherapy against cancers and infectious diseases. Passive immunity is the short-term protection of a host, achieved by the administration of pre-formed antibody directed against a heterologous organism. For example, compositions of the invention comprising Hsp-peptide complexes obtained from cells infected with an infectious organism and oligomerized with lectin molecules may be used to elicit an immune response in a subject, the sera removed from the subject can also be used for treatment or prevention of a disease that is caused by the infectious organism in other subject.

### 5.8. Prevention and Treatment of Diseases

The present invention further provides a prophylactically or therapeutically effective amount of a composition comprising one or more molecular complexes for use in preventing or treating a disease (*e.g.*, cancer, infectious diseases, anemia, growth hormone deficiencies, enzyme deficiency diseases, conditions of immune suppression, etc.) , wherein each complex comprises a lectin associated with an immunologically and/or biologically active glycoprotein. In one embodiment, the glycoprotein is an Antigenic Molecule. In a specific embodiment, the complex comprises a lectin, a glycoprotein that is an Antigenic Molecule, and another molecule, such as a heat shock protein, that may or may not be glycosylated. In another embodiment, the glycoprotein is not an Antigenic Molecule. In a specific embodiment, the glycoprotein is a glycosylated heat shock protein. In yet another embodiment, the molecular complex of the invention comprises a lectin, a glycoprotein that is not an Antigenic Molecule, and an Antigenic Molecule (which may or may not be a glycoprotein). In a specific embodiment, the Antigenic Molecule is a protein (including peptide and polypeptide) that displays the antigenicity of an antigen of a type of cancer or of an agent of an infectious disease. The compositions may further comprises a pharmaceutically acceptable carrier. In some embodiments, the subject is an animal. In some embodiments, the subject is a mammal. In some embodiments, the subject is a farm animal, such as a horse, a chicken, a sheep, or a pig. In some embodiments, the subject is a pet, such as a bird, a dog, or a cat. In a preferred embodiment, the subject is a human.

In one embodiment, "treatment" or "treating" refers to an amelioration of a disease, or at least one discernible symptom thereof. In another embodiment, "treatment" or "treating" refers to an amelioration of at least one measurable physical parameter associated with a disease, not necessarily discernible by the subject. In yet another embodiment, "treatment" or "treating" refers to inhibiting the progression of a disease, either physically, *e.g.*, stabilization of a discernible symptom, physiologically, *e.g.*, stabilization of a physical parameter, or both. In yet another embodiment, "treatment" or "treating" refers to delaying the onset of a disease or disorder.

In certain embodiments, the compositions of the present invention are administered to a subject as a preventative measure against a disease. As used herein, "prevention" or "preventing" refers to a reduction of the risk of acquiring a given disease such as cancer or infectious disease. In one mode of the embodiment, the compositions of the present invention are administered as a preventative measure to a subject having a genetic predisposition to a cancer. In another mode of the embodiment, the compositions of the present invention are administered as a preventative measure to a subject facing exposure to carcinogens including but not limited to chemicals and/or radiation, or to a subject facing exposure to an agent of an infectious disease.

For example, in certain embodiments, administration of the compositions of the invention lead to an inhibition or reduction of the growth of cancerous cells or infectious agents by at least 99%, at least 95%, at least 90%, at least 85%, at least 80%, at least 75%, at least 70%, at least 60%, at least 50%, at least 45%, at least 40%, at least 45%, at least 35%, at least 30%, at least 25%, at least 20%, or at least 10% relative to the growth in absence of said composition.

The compositions prepared by methods disclosed herein comprise complexes of lectin associated with glycoprotein(s), preferably heat shock protein(s). The compositions may further comprise a population of antigenic peptides (which may or may not be glycoproteins). The compositions of the invention can be used to induce an inflammatory reaction at the tumor site and can ultimately cause a regression of the tumor burden in the cancer patients treated. The compositions of the invention can enhance the immunocompetence of the subject and elicit specific immunity against infectious agents or specific immunity against pre-neoplastic and neoplastic cells. The compositions of the invention can also be used to prevent the onset and progression of infectious diseases, and to inhibit the growth and progression of tumor cells.

Combination therapy refers to the use of the molecular complexes of the invention with another modality to prevent or treat a disease, *e.g*., cancer, infectious diseases, anemia, growth hormone deficiencies, enzyme deficiency diseases, conditions of immune suppression. The administration of the complexes of the invention can augment the effect of prophylactic or therapeutic agents, such as anti-cancer agents or anti-infectives, and vice versa. Preferably, this additional form of modality is a non-lectin-HSP based modality, *i.e*., this modality does not comprise either HSP or lectin as a component. This approach is commonly termed combination therapy, adjunctive therapy or conjunctive therapy (the terms are used interchangeably herein). With combination therapy, additive potency or additive therapeutic effect can be observed. Synergistic outcomes where the therapeutic efficacy is greater than additive can also be expected. The use of combination therapy can also provide better therapeutic profiles than the administration of the treatment modality, or the lectin-HSP complexes alone. The additive or synergistic effect may allow the dosage and/or dosing frequency of either or both modalities be adjusted to reduce or avoid unwanted or adverse effects.

According to the invention, molecular complexes of the invention can be used alone or in combination with many different types of treatment modalities. Some of such modalities are particularly useful for a specific type of cancer or infectious disease and are discussed in Section 5.8.1 and 5.8.2. Many other modalities have an effect on the functioning of the immune system and are applicable generally to both neoplastic and infectious diseases.

In one embodiment, molecular complexes of the invention are used in combination with one or more biological response modifiers to treat cancer or infectious disease. One group of biological response modifiers is the cytokines. In one such embodiment, a cytokine is administered to a subject receiving molecular complexes of the invention. In another such embodiment, the molecular complexes are administered to a subject receiving a chemotherapeutic agent in combination with a cytokine. In various embodiments, one or more cytokine(s) can be used and are selected from the group consisting of IL-1α, IL- 1β, IL-2, IL-3, IL-4, IL-5, IL-6, IL-7, IL-8, IL-9, IL-10, IL-11, IL-12, IFNα, IFNβ, IFNγ, TNFα, TNFβ, G-CSF, GM-CSF, TGF-β, IL-15, IL-18, GM-CSF, INF-γ, INF-α, SLC, endothelial monocyte activating protein-2 (EMAP2), MIP-3α, MIP-3β, or an MHC gene, such as HLA-B7. Addtionally, other exemplary cytokines include other members of the TNF family, including but not limited to TNF-α-related apoptosis-inducing ligand (TRAIL), TNF-α-related activation-induced cytokine (TRANCE), TNF-α-related weak inducer of apoptosis (TWEAK), CD40 ligand (CD40L), lymphotoxin alpha (LT-α), lymphotoxin beta (LT-β), OX40 ligand (OX40L), Fas ligand (FasL), CD27 ligand (CD27L), CD30 ligand (CD30L), 41BB ligand (41BBL), APRIL, LIGHT, TL1, TNFSF16, TNFSF17, and AITR-L, or a functional portion thereof. See, *e.g.,* Kwon et al., 1999, Curr. Opin. Immunol. 11:340-345 for a general review of the TNF family. Preferably, the molecular complexes are administered prior to the treatment modalities. In a specific embodiment, complexes of the invention are administered to a subject receiving cyclophosphamide in combination with IL-12 for treatment of cancer.

In another embodiment, molecular complexes of the invention are used in combination with one or more biological response modifiers which are agonists or antagonists of various ligands, receptors and signal transduction molecules of the immune system. For examples, the biological response modifiers include but are not limited to agoinsts of Toll-like receptors (TLR-2, TLR-7, TLR-8 and TLR-9; LPS; agonists of 41BB, OX40, ICOS, and CD40; and antagonists of Fas ligand, PD1, and CTLA-4. These agonists and antagonists can be antibodies, antibody fragments, peptides, peptidomimetic compounds, and polysaccharides.

In yet another embodiment, molecular complexes of the invention are used in combination with one or more biological response modifiers which are immunostimulatory nucleic acids. Such nucleic acids, many of which are oligonucleotides comprising an unmethylated CpG motif, are mitogenic to vertebrate lymphocytes, and are known to enhance the immune response. See Woolridge et al., 1997, Blood 89:2994-2998. Such oligonucleotides are described in International Patent Publication Nos. WO 01/22972, WO 01/51083, WO 98/40100 and WO 99/61056 , as well as United States Patent Nos. 6,207,646, 6,194,388, 6,218,371, 6,239,116, 6,429,199, and 6,406,705. Other kinds of immunostimulatory oligonucleotides such as phosphorothioate oligodeoxynucleotides containing YpG- and CpR-motifs have been described by Kandimalla et al. in Bioorganic & Medicinal Chemistry 9:807-813 (2001). Also encompassed are immunostimulatory oligonucleotides that lack CpG dinucleotides which when administered by mucosal routes (including low dose administration) or at high doses through parenteral routes, augment antibody responses, often as much as did the CpG nucleic acids, however the response was Th2-biased (IgG1>>IgG2a). See United States Patent Publication No. 20010044416 A1 . Methods of determining the activity of immunostimulatory oligonucleotides can be performed as described in the aforementioned patents and publications. Moreover, immunostimulatory oligonucleotides can be modified within the phosphate backbone, sugar, nucleobase and internucleotide linkages in order to modulate the activity. Such modifications are known to those of skill in the art.

In yet another embodiment, molecular complexes of the invention are used in combination with one or more adjuvants. In a specific embodiment, molecular complexes of the invention are used in combination with a saponin (e.g., QS-21) and/or an immunostimulatory oligonucleotide (*e.g.*, an oligonucleotide comprising at least a CpG dinucleotide). The adjuvant(s) can be administered separately or present in a composition in admixture with complexes of the invention. A systemic adjuvant is an adjuvant that can be delivered parenterally. Systemic adjuvants include adjuvants that creates a depot effect, adjuvants that stimulate the immune system and adjuvants that do both. An adjuvant that creates a depot effect as used herein is an adjuvant that causes the antigen to be slowly released in the body, thus prolonging the exposure of immune cells to the antigen. This class of adjuvants includes but is not limited to alum (*e.g.*, aluminum hydroxide, aluminum phosphate); or emulsion-based formulations including mineral oil, non-mineral oil, water-in-oil or oil-in-water-in oil emulsion, oil-in-water emulsions such as Seppic ISA series of Montanide adjuvants (*e.g.*, Montanide ISA 720, AirLiquide, Paris, France); MF-59 (a squalene-in-water emulsion stabilized with Span 85 and Tween 80; Chiron Corporation, Emeryville, Calif.; and PROVAX (an oil-in-water emulsion containing a stabilizing detergent and a micelle-forming agent; IDEC, Pharmaceuticals Corporation, San Diego, Calif.).

Other adjuvants stimulate the immune system, for instance, cause an immune cell to produce and secrete cytokines or IgG. This class of adjuvants includes but is not limited to immunostimulatory nucleic acids, such as CpG oligonucleotides; saponins purified from the bark of the Q. saponaria tree, such as QS21; poly[di(carboxylatophen-oxy)phosphazene (PCPP polymer; Virus Research Institute, USA); derivatives of lipopolysaccharides (LPS) such as monophosphoryl lipid A (MPL; Ribi ImmunoChem Research, Inc., Hamilton, Mont.), muramyl Bipeptide (MDP; Ribi) andthreonyl-muramyl Bipeptide (t-MDP; Ribi); OM-174 (a glucosamine disaccharide related to lipid A; OM Pharma SA, Meyrin, Switzerland); Aminoalkyl Glucosaminide phosphates (AGPs, Corixa Corporation), and Leishmania elongation factor (a purified Leishmania protein; Corixa Corporation, Seattle, Wash.).

Other systemic adjuvants are adjuvants that create a depot effect and stimulate the immune system. These compounds are those compounds which have both of the above-identified functions of systemic adjuvants. This class of adjuvants includes but is not limited to ISCOMs (Immunostimulating complexes which contain mixed saponins, lipids and form virus-sized particles with pores that can hold antigen; CSL, Melbourne, Australia); SB-AS2 (SmithKline Beecham adjuvant system #2 which is an oil-in-water emulsion containing MPL and QS21: SmithKline Beecham Biologicals [SBB], Rixensart, Belgium); SB-AS4 (SmithKline Beecham adjuvant system #4 which contains alum and MPL; SBB, Belgium); non-ionic block copolymers that form micelles such as CRL 1005 (these contain a linear chain of hydrophobic polyoxpropylene flanked by chains of polyoxyethylene; Vaxcel, Inc., Norcross, Ga.); and Syntex Adjuvant Formulation (SAF, an oil-in-water emulsion containing Tween 80 and a nonionic block copolymer; Syntex Chemicals, Inc., Boulder, Colo.).

The mucosal adjuvants useful according to the invention are adjuvants that are capable of inducing a mucosal immune response in a subject when administered to a mucosal surface in conjunction with complexes of the invention. Mucosal adjuvants include but are not limited to CpG nucleic acids (*e.g*. PCT published patent application WO 99/61056), Bacterial toxins: *e.g*., Cholera toxin (CT), CT derivatives including but not limited to CT B subunit (CTB) (Wu *et al.,* 1998, Tochikubo *et al.,* 1998); CTD53 (Val to Asp) (Fontana *et al.,* 1995); CTK97 (Val to Lys) (Fontana *et al.,* 1995); CTK104 (Tyr to Lys) (Fontana *et al.,* 1995); CTD53/K63 (Val to Asp, Ser to Lys) (Fontana *et al.,* 1995); CTH54 (Arg to His) (Fontana *et al.,* 1995); CTN107 (His to Asn) (Fontana *et al.,* 1995); CTE114 (Ser to Glu) (Fontana *et al.,* 1995); CTE112K (Glu to Lys) (Yamamoto *et al.,* 1997a); CTS61F (Ser to Phe) (Yamamoto *et al.,* 1997a, 1997b); CTS106 (Pro to Lys) (Douce *et al.,*1997, Fontana *et al.,* 1995); and CTK63 (Ser to Lys) (Douce *et al.,* 1997, Fontana *et al.,* 1995), Zonula occludens toxin, zot, Escherichia coli heat-labile enterotoxin, Labile Toxin (LT), LT derivatives including but not limited to LT B subunit (LTB) (Verweij *et al.,* 1998); LT7K (Arg to Lys) (*Komase et al.,* 1998, Douce *et al.,* 1995); LT61F (Ser to Phe) (*Komase et al.,* 1998); LT112K (Glu to Lys) (Komase *et al.,* 1998); LT118E (Gly to Glu) (Komase *et al.,* 1998); LT146E (Arg to Glu) (*Komase et al.,* 1998); LT192G (Arg to Gly) (Komase *et al.,* 1998); LTK63 (Ser to Lys) (Marchetti *et al.,* 1998, Douce *et al.,* 1997, 1998, Di Tommaso *et al.,* 1996); and LTR72 (Ala to Arg) (Giuliani *et al.,* 1998), Pertussis toxin, PT. (Lycke *et al.,* 1992, Spangler BD, 1992, Freytag and Clemments, 1999, Roberts *et al.,* 1995, Wilson *et al.,* 1995) including PT-9K/129G (Roberts *et al.,* 1995, Cropley *et al.,* 1995); Toxin derivatives (see below) (Holmgren *et al.,* 1993, Verweij *et al.,* 1998, Rappuoli *et al.,* 1995, Freytag and Clements, 1999); Lipid A derivatives (*e.g.*, monophosphoryl lipid A, MPL) (Sasaki *et al.,* 1998, Vancott *et al.,* 1998; Muramyl Bipeptide (MDP) derivatives (Fukushima *et al.,* 1996, Ogawa *et al.,* 1989, Michalek *et al.,* 1983, Morisaki *et al.,* 1983); bacterial outer membrane proteins (e.g., outer surface protein A (OspA) lipoprotein of Borrelia burgdorferi, outer membrane protine of Neisseria meningitidis) (Marinaro *et al.,* 1999, Van de Verg *et al.,* 1996); oil-in-water emulsions (*e.g*., MF59) (Barchfield *et al.,* 1999, Verschoor *et al.,* 1999, O'Hagan, 1998); aluminum salts (Isaka *et al.,* 1998, 1999); and Saponins (*e.g.,* QS21) Aquila Biopharmaceuticals, Inc., Worster, Me.) (Sasaki *et al.,* 1998, MacNeal *et al.,* 1998), ISCOMs, MF-59 (a squalene-in-water emulsion stabilized with Span 85 and Tween 80; Chiron Corporation, Emeryville, Calif.); the Seppic ISA series of Montanide adjuvants (*e.g*., Montanide ISA 720; AirLiquide, Paris, France); PROVAX (an oil-in-water emulsion containing a stabilizing detergent and a micell-forming agent; IDEC Pharmaceuticals Corporation, San Diego, Calif.); Syntext Adjuvant Formulation (SAF; Syntex Chemicals, Inc., Boulder, Colo.); poly[di(carboxylatophenoxy)phosphazene (PCPP polymer; Virus Research Institute, USA) and Leishmania elongation factor (Corixa Corporation, Seattle, Wash.).

### 5.8.1 TARGET CANCERS

Administration of the compositions of the invention, alone or with the sensitized APCs, stimulates the immunocompetence of the host subject and elicits specific immunity against the preneoplastic and/or neoplastic cells. As used herein, "preneoplastic" cell refers to a cell which is in transition from a normal to a neoplastic form. Morphological evidence, increasingly supported by molecular biologic studies, indicates that preneoplasia progresses through multiple steps. Non-neoplastic cell growth commonly consists of hyperplasia, metaplasia, or most particularly, dysplasia (for review of such abnormal growth conditions, *see* Robbins and Angell, 1976, Basic Pathology, 2d Ed., W.B. Saunders Co., Philadelphia, pp. 68-79). Hyperplasia is a form of controlled cell proliferation involving an increase in cell number in a tissue or organ, without significant alteration in structure or function. For example, endometrial hyperplasia often precedes endometrial cancer. Metaplasia is a form of controlled cell growth in which one type of adult or fully differentiated cell substitutes for another type of adult cell. Metaplasia can occur in epithelial or connective tissue cells. Atypical metaplasia involves a somewhat disorderly metaplastic epithelium. Dysplasia is frequently a forerunner of cancer, and is found mainly in the epithelia; it is the most disorderly form of non-neoplastic cell growth, involving a loss in individual cell uniformity and in the architectural orientation of cells. Dysplastic cells often have abnormally large, deeply stained nuclei, and exhibit pleomorphism. Dysplasia characteristically occurs where there exists chronic irritation or inflammation, and is often found in the cervix, respiratory passages, oral cavity, and gall bladder. Although preneoplastic lesions may progress to neoplasia, they may also remain stable for long periods and may even regress, particularly if the inciting agent is removed or if the lesion succumbs to an immunological attack by its host. Cancers which can be treated with the compositions of the present invention also include, but are not limited to, human sarcomas and carcinomas. Human sarcomas and carcinomas are also responsive to adoptive immunotherapy by the oligomerized glycoprotein complex sensitized APCs.

In one embodiment, combination therapy encompasses, in addition to the administration of the molecular complexes of the invention, the adjunctive uses of one or more modalities that aid in the prevention or treatment of cancer, which modalities include, but is not limited to chemotherapeutic agents, immunotherapeutics, anti-angiogenic agents, cytokines, hormones, antibodies, polynucleotides, radiation and photodynamic therapeutic agents. In specific embodiments, combination therapy can be used to prevent the recurrence of cancer, inhibit metastasis, or inhibit the growth and/or spread of cancer or metastasis.

Types of cancers that can be treated or prevented by the methods of the present invention include, but are not limited to human sarcomas and carcinomas, *e.g*., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma; leukemias, *e.g*., acute lymphocytic leukemia and acute myelocytic leukemia (myeloblastic, promyelocytic, myelomonocytic, monocytic and erythroleukemia); chronic leukemia (chronic myelocytic (granulocytic) leukemia and chronic lymphocytic leukemia); and polycythemia vera, lymphoma (Hodgkin's disease and non-Hodgkin's disease), multiple myeloma, Waldenström's macroglobulinemia, and heavy chain disease.

In another embodiment, the patient having a cancer is immunosuppressed by reason of having undergone anti-cancer therapy (*e.g*., chemotherapy radiation) prior to administration of the molecular complexes of the invention or administration of the lectin-Hsp sensitized APC.

There are many reasons why immunotherapy as provided by the present invention is desired for use in cancer patients. First, surgery with anesthesia may lead to immunosuppression. With appropriate immunotherapy in the preoperative period, this immunosuppression may be prevented or reversed. This could lead to fewer infectious complications and to accelerated wound healing. Second, tumor bulk is minimal following surgery and immunotherapy is most likely to be effective in this situation. A third reason is the possibility that tumor cells are shed into the circulation at surgery and effective immunotherapy applied at this time can eliminate these cells.

The preventive and therapeutic methods disclosed herein are directed at enhancing the immunocompetence of the cancer patient either before surgery, at or after surgery, and to induce tumor-specific immunity to cancer cells, with the objective being inhibition of cancer, and with the ultimate clinical objective being total cancer regression and eradication. The methods of the invention can also be used in individuals at enhanced risk of a particular type of cancer, *e.g*., due to familial history or environmental risk factors.

In some embodiments, one or more anti-cancer agent, in addition to the molecular complexes of the invention, is administered to a subject in need thereof for treating or preventing a cancer. An anti-cancer agent refers to any molecule or compound that assists in the treatment of tumors or cancer. Examples of anti-cancer agents that may be used in the methods of the present invention include, but are not limited to: acivicin; aclarubicin; acodazole hydrochloride; acronine; adozelesin; aldesleukin; altretamine; ambomycin; ametantrone acetate; aminoglutethimide; amsacrine; anastrozole; anthramycin; asparaginase; asperlin; azacitidine; azetepa; azotomycin; batimastat; benzodepa; bicalutamide; bisantrene hydrochloride; bisnafide dimesylate; bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; chlorambucil; cirolemycin; cisplatin; cladribine; crisnatol mesylate; cyclophosphamide; cytarabine; dacarbazine; dactinomycin; daunorubicin hydrochloride; decitabine; dexormaplatin; dezaguanine; dezaguanine mesylate; diaziquone; docetaxel; doxorubicin; doxorubicin hydrochloride; droloxifene; droloxifene citrate; dromostanolone propionate; duazomycin; edatrexate; eflornithine hydrochloride; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; flurocitabine; fosquidone; fostriecin sodium; gemcitabine; gemcitabine hydrochloride; hydroxyurea; idarubicin hydrochloride; ifosfamide; ilmofosine; interleukin II (including recombinant interleukin II, or rIL2), interferon alfa-2a; interferon alfa-2b; interferon alfa-n1; interferon alfa-n3; interferon beta-I a; interferon gamma-I b; iproplatin; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine hydrochloride; megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxisuran; paclitaxel; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; rogletimide; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; zorubicin hydrochloride.

Other anti-cancer drugs that can be used include, but are not limited to: 20-epi-1,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; canarypox IL-2; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorlns; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin B; deslorelin; dexamethasone; dexifosfamide; dexrazoxane; dexverapamil; diaziquone; didemnin B; didox; diethylnorspermine; dihydro-5-azacytidine; dihydrotaxol, 9-; dioxamycin; Biphenyl spiromustine; docetaxel; docosanol; dolasetron; doxifluridine; droloxifene; dronabinol; duocarmycin SA; ebselen; ecomustine; edelfosine; edrecolomab; eflornithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; hypericin; ibandronic acid; idarubicin; idoxifene; idramantone; ilmofosine; ilomastat; imidazoacridones; imiquimod; immunostimulant peptides; insulin-like growth factor-1 receptor inhibitor; interferon agonists; interferons; interleukins; iobenguane; iododoxorubicin; ipomeanol, 4-; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; lovastatin; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mismatched double stranded RNA; mitoguazone; mitolactol; mitomycin analogues; mitonafide; mitotoxin fibroblast growth factor-saporin; mitoxantrone; mofarotene; molgramostim; monoclonal antibody, human chorionic gonadotrophin; monophosphoryl lipid A+myobacterium cell wall sk; mopidamol; multiple drug resistance gene inhibitor; multiple tumor suppressor 1-based therapy; mustard anti-cancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; 06-benzylguanine; octreotide; okicenone; oligonucleotides; onapristone; ondansetron; ondansetron; oracin; oral cytokine inducer; ormaplatin; osaterone; oxaliplatin; oxaunomycin; paclitaxel; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rogletimide; rohitukine; romurtide; roquinimex; rubiginone B1; ruboxyl; safingol; saintopin; SarCNU; sarcophytol A; sargramostim; Sdi 1 mimetics; semustine; senescence derived inhibitor 1; sense oligonucleotides; signal transduction inhibitors; signal transduction modulators; single chain antigen binding protein; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem-cell division inhibitors; stipiamide; stromelysin inhibitors; sulfinosine; superactive vasoactive intestinal peptide antagonist; suradista; suramin; swainsonine; synthetic glycosaminoglycans; tallimustine; tamoxifen methiodide; tauromustine; tazarotene; tecogalan sodium; tegafur; tellurapyrylium; telomerase inhibitors; temoporfin; temozolomide; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; totipotent stem cell factor; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; vector system, erythrocyte gene therapy; velaresol; veramine; verdins; verteporfin; vinorelbine; vinxaltine; vitaxin; vorozole; zanoterone; zeniplatin; zilascorb; and zinostatin stimalamer.

An anti-cancer agent can be a chemotherapeutic agents which include but are not limited to, the following groups of compounds : cytotoxic antibiotics, antimetabolities, anti-mitotic agents, alkylating agents, platinum compounds, arsenic compounds, DNA topoisomerase inhibitors, taxanes, nucleoside analogues, plant alkaloids, and toxins; and synthetic derivatives thereof. Table 2 lists exemplary compounds of the groups:

**TABLE 2**

| Alkylating agents | |
|---|---|
| Nitrogen mustards: | Cyclophosphamide |
| | Ifosfamide |
| | Trofosfamide |
| | Chlorambucil |
| Nitrosoureas: | Carmustine (BCNU) |
| | Lomustine (CCNU) |
| Alkylsulphonates: | Busulfan |
| | Treosulfan |
| Triazenes: | Dacarbazine |
| Platinum containing compounds: | Cisplatin |
| | Carboplatin |
| | Aroplatin |
| | Oxaliplatin |

| Plant Alkaloids | |
|---|---|
| Vinca alkaloids: | Vincristine |
| | Vinblastine |
| | Vindesine |
| | Vinorelbine |
| Taxoids: | Paclitaxel |
| | Docetaxol |
| | |

| DNA Topoisomerase Inhibitors | |
|---|---|
| Epipodophyllins: | Etoposide |
| | Teniposide |
| | Topotecan |
| | 9-aminocamptothecin |
| | Camptothecin |
| | Crisnatol |
| mitomycins: | Mitomycin C |
| | |

| Anti-folates: | |
|---|---|
| DHFR inhibitors: | Methotrexate |
| | Trimetrexate |
| IMP dehydrogenase Inhibitors: | Mycophenolic acid |
| | Tiazofurin |
| | Ribavirin |
| | EICAR |
| Ribonuclotide reductase Inhibitors: | Hydroxyurea |
| | Deferoxamine |

| Pyrimidine analogs: | |
|---|---|
| Uracil analogs: | 5-Fluorouracil |
| | Floxuridine |
| | Doxifluridine |
| | Ratitrexed |
| Cytosine analogs: | Cytarabine (ara C) |
| | Cytosine arabinoside |
| | Fludarabine |

| Purine analogs: | Mercaptopurine |
|---|---|
| | Thioguanine |

| DNA Antimetabolites: | 3-HP |
|---|---|
| | 2'-deoxy-5-fluorouridine |
| | 5-HP |
| | alpha-TGDR |
| | aphidicolin glycinate |
| | ara-C |
| | 5-aza-2'-deoxycytidine |
| | beta-TGDR cyclocytidine guanazole |
| | inosine glycodialdehyde macebecin II |
| | pyrazoloimidazole |

| Antimitotic agents: | allocolchicine |
|---|---|
| | Halichondrin B |
| | colchicine |
| | colchicine derivative |
| | dolstatin 10 |
| | maytansine |
| | rhizoxin |
| | thiocolchicine |
| | trityl cysteine |

| Others: | |
|---|---|
| Isoprenylation inhibitors: | |
| Dopaminergic neurotoxins: | 1-methyl-4-phenylpyridinium ion |
| Cell cycle inhibitors: | Staurosporine |
| Actinomycins: | Actinomycin D |
| | Dactinomycin |
| Bleomycins: | Bleomycin A2 |
| | Bleomycin B2 |
| | Peplomycin |
| Anthracyclines: | Daunorubicin |
| | Doxorubicin (adriamycin) |
| | Idarubicin |
| | Epirubicin |
| | Pirarubicin |
| | Zorubicin |
| | Mitoxantrone |
| MDR inhibitors: | Verapamil |
| Ca²⁺ ATPase inhibitors: | Thapsigargin |

Compositions comprising one or more chemotherapeutic agents (*e.g*., FLAG, CHOP) are also contemplated by the present invention. FLAG comprises fludarabine, cytosine arabinoside (Ara-C) and G-CSF. CHOP comprises cyclophosphamide, vincristine, doxorubicin, and prednisone. Each of the foregoing lists is illustrative, and is not intended to be limiting.

In one embodiment, breast cancer can be treated with a pharmaceutical composition comprising complexes of the invention in combination with 5-fluorouracil, cisplatin, docetaxel, doxorubicin, Herceptin®, gemcitabine, IL-2, paclitaxel, and/or VP-16 (etoposide).

In another embodiment, prostate cancer can be treated with a pharmaceutical composition comprising complexes of the invention in combination with paclitaxel, docetaxel, mitoxantrone, and/or an androgen receptor antagonist (*e.g*., flutamide).

In another embodiment, leukemia can be treated with a pharmaceutical composition comprising complexes of the invention in combination with fludarabine, cytosine arabinoside, gemtuzumab (MYLOTARG), daunorubicin, methotrexate, vincristine, 6-mercaptopurine, idarubicin, mitoxantrone, etoposide, asparaginase, prednisone and/or cyclophosphamide. As another example, myeloma can be treated with a pharmaceutical composition comprising complexes of the invention in combination with dexamethasone.

In another embodiment, melanoma can be treated with a pharmaceutical composition comprising complexes of the invention in combination with dacarbazine.

In another embodiment, colorectal cancer can be treated with a pharmaceutical composition comprising complexes of the invention in combination with irinotecan.

In another embodiment, lung cancer can be treated with a pharmaceutical composition comprising complexes of the invention in combination with paclitaxel, docetaxel, etoposide and/or cisplatin.

In another embodiment, non-Hodgkin's lymphoma can be treated with a pharmaceutical composition comprising complexes of the invention in combination with cyclophosphamide, CHOP, etoposide, bleomycin, mitoxantrone and/or cisplatin.

In another embodiment, gastric cancer can be treated with a pharmaceutical composition comprising complexes of the invention in combination with cisplatin.

In another embodiment, pancreatic cancer can be treated with a pharmaceutical composition comprising complexes of the invention in combination with gemcitabine.

According to the invention, the complexes of the invention can be administered prior to, subsequently, or concurrently with anti-cancer agent(s), for the prevention or treatment of cancer. Depending on the type of cancer, the subject's history and condition, and the anti-cancer agent(s) of choice, the use of the complexes of the invention can be coordinated with the dosage and timing of chemotherapy.

The use of the complexes of the invention can be added to a regimen of chemotherapy. In one embodiment, the chemotherapeutic agent is gemcitabine at a dose ranging from 100 to 1000 mg/m2/cycle. In one embodiment, the chemotherapeutic agent is dacarbazine at a dose ranging from 200 to 4000 mg/m2/cycle. In a preferred embodiment, the dose of dacarbazine ranges from 700 to 1000 mg/m2/cycle. In another embodiment, the chemotherapeutic agent is fludarabine at a dose ranging from 25 to 50 mg/m2/cycle. In another embodiment, the chemotherapeutic agent is cytosine arabinoside (Ara-C) at a dose ranging from 200 to 2000 mg/m2/cycle. In another embodiment, the chemotherapeutic agent is docetaxel at a dose ranging from 1.5 to 7.5 mg/kg/cycle. In another embodiment, the chemotherapeutic agent is paclitaxel at a dose ranging from 5 to 15 mg/kg/cycle. In yet another embodiment, the chemotherapeutic agent is cisplatin at a dose ranging from 5 to 20 mg/kg/cycle. In yet another embodiment, the chemotherapeutic agent is 5-fluorouracil at a dose ranging from 5 to 20 mg/kg/cycle. In yet another embodiment, the chemotherapeutic agent is doxorubicin at a dose ranging from 2 to 8 mg/kg/cycle. In yet another embodiment, the chemotherapeutic agent is epipodophyllotoxin at a dose ranging from 40 to 160 mg/kg/cycle. In yet another embodiment, the chemotherapeutic agent is cyclophosphamide at a dose ranging from 50 to 200 mg/kg/cycle. In yet another embodiment, the chemotherapeutic agent is irinotecan at a dose ranging from 50 to 75, 75 to 100, 100 to 125, or 125 to 150 mg/m2/cycle. In yet another embodiment, the chemotherapeutic agent is vinblastine at a dose ranging from 3.7 to 5.4, 5.5 to 7.4, 7.5 to 11, or 11 to 18.5 mg/m2/cycle. In yet another embodiment, the chemotherapeutic agent is vincristine at a dose ranging from 0.7 to 1.4, or 1.5 to 2 mg/m2/cycle. In yet another embodiment, the chemotherapeutic agent is methotrexate at a dose ranging from 3.3 to 5, 5 to 10, 10 to 100, or 100 to 1000 mg/m2/cycle.

In a preferred embodiment, the invention further encompasses the use of low doses of chemotherapeutic agents when administered as part of the combination therapy regimen. For example, initial treatment with the complexes of the invention increases the sensitivity of a tumor to subsequent challenge with a dose of chemotherapeutic agent, which dose is near or below the lower range of dosages when the chemotherapeutic agent is administered without complexes of the invention.

In one embodiment, complexes of the invention and a low dose (*e.g*., 6 to 60 mg/m2/day or less) of docetaxel are administered to a cancer patient. In another embodiment, complexes of the invention and a low dose (*e.g*., 10 to 135 mg/m2/day or less) of paclitaxel are administered to a cancer patient. In yet another embodiment, complexes of the invention and a low dose (*e.g*., 2.5 to 25 mg/m2/day or less) of fludarabine are administered to a cancer patient. In yet another embodiment, complexes of the invention and a low dose (*e.g*., 0.5 to 1.5 g/m2/day or less) of cytosine arabinoside (Ara-C) are administered to a cancer patient.

In another embodiment, the chemotherapeutic agent is gemcitabine at a dose ranging from 10 to 100mg/m2/cycle. In another embodiment, the chemotherapeutic agent is cisplatin, *e.g*., PLATINOL or PLATINOL-AQ (Bristol Myers), at a dose ranging from 5 to 10, 10 to 20, 20 to 40, or 40 to 75 mg/m2/cycle. In yet another embodiment, a dose of cisplatin ranging from 7.5 to 75 mg/m2/cycle is administered to a patient with ovarian cancer. In yet another embodiment, a dose of cisplatin ranging from 5 to 50 mg/m2/cycle is administered to a patient with bladder cancer. In yet another embodiment, the chemotherapeutic agent is carboplatin, *e.g*., PARAPLATIN (Bristol Myers), at a dose ranging from 2 to 4, 4 to 8, 8 to 16, 16 to 35, or 35 to 75 mg/m2/cycle. In yet another embodiment, a dose of carboplatin ranging from 7.5 to 75 mg/m2/cycle is administered to a patient with ovarian cancer. In another embodiment, a dose of carboplatin ranging from 5 to 50 mg/m2/cycle is administered to a patient with bladder cancer. In yet another embodiment, a dose of carboplatin ranging from 2 to 20 mg/m2/cycle is administered to a patient with testicular cancer. In yet another embodiment, the chemotherapeutic agent is docetaxel, *e.g*., TAXOTERE (Rhone Poulenc Rorer) at a dose ranging from 6 to 10, 10 to 30, or 30 to 60 mg/m2/cycle. In yet another embodiment, the chemotherapeutic agent is paclitaxel, *e.g*., TAXOL (Bristol Myers Squibb), at a dose ranging from 10 to 20, 20 to 40, 40 to 70, or 70 to 135 mg/kg/cycle. In yet another embodiment, the chemotherapeutic agent is 5-fluorouracil at a dose ranging from 0.5 to 5 mg/kg/cycle. In yet another embodiment, the chemotherapeutic agent is doxorubicin, *e.g*., ADRIAMYCIN (Pharmacia & Upjohn), DOXIL (Alza), RUBEX (Bristol Myers Squibb), at a dose ranging from 2 to 4, 4 to 8, 8 to 15, 15 to 30, or 30 to 60 mg/kg/cycle.

In another embodiment, complexes of the invention is administered in combination with one or more immunotherapeutic agents, such as antibodies and vaccines. In a preferred embodiment, the antibodies have *in vivo* therapeutic and/or prophylactic uses against cancer. In some embodiments, the antibodies can be used for treatment and/or prevention of infectious disease. Examples of therapeutic and prophylactic antibodies include, but are not limited to, MDX-010 (Medarex, NJ) which is a humanized anti-CTLA-4 antibody currently in clinic for the treatment of prostate cancer; SYNAGIS® (MedImmune, MD) which is a humanized anti-respiratory syncytial virus (RSV) monoclonal antibody for the treatment of patients with RSV infection; HERCEPTIN® (Trastuzumab) (Genentech, CA) which is a humanized anti-HER2 monoclonal antibody for the treatment of patients with metastatic breast cancer. Other examples are a humanized anti-CD18 F(ab')2 (Genentech); CDP860 which is a humanized anti-CD18 F(ab')2 (Celltech, UK); PRO542 which is an anti-HIV gp120 antibody fused with CD4 (Progenics/Genzyme Transgenics); Ostavir which is a human anti Hepatitis B virus antibody (Protein Design Lab/Novartis); PROTOVIR™ which is a humanized anti-CMV IgG1 antibody (Protein Design Lab/Novartis); MAK-195 (SEGARD) which is a murine anti-TNF-αF(ab')2 (Knoll Pharma/BASF); IC 14 which is an anti-CD 14 antibody (ICOS Pharm); a humanized anti-VEGF IgG1 antibody (Genentech); OVAREX™ which is a murine anti-CA 125 antibody (Altarex); PANOREX™ which is a murine anti-17-IA cell surface antigen IgG2a antibody (Glaxo Wellcome/Centocor); BEC2 which is a murine anti-idiotype (GD3 epitope) IgG antibody (ImClone System); IMC-C225 which is a chimeric anti-EGFR IgG antibody (ImClone System); VITAXIN™ which is a humanized anti-αVβ3 integrin antibody (Applied Molecular Evolution/MedImmune); Campath 1H/LDP-03 which is a humanized anti CD52 IgG1 antibody (Leukosite); Smart M195 which is a humanized anti-CD33 IgG antibody (Protein Design Lab/Kanebo); RITUXAN™ which is a chimeric anti-CD20 IgG1 antibody (IDEC Pharm/Genentech, Roche/Zettyaku); LYMPHOCIDE™ which is a humanized anti-CD22 IgG antibody (Immunomedics); Smart ID 10 which is a humanized anti-HLA antibody (Protein Design Lab); ONCOLYM™ (Lym-1) is a radiolabelled murine anti-HLA DIAGNOSTIC REAGENT antibody (Techniclone); ABX-IL8 is a human anti-IL8 antibody (Abgenix); anti-CD11a is a humanized IgG1 antibody (Genentech/Xoma); ICM3 is a humanized anti-ICAM3 antibody (ICOS Pharm); IDEC-114 is a primatized anti-CD80 antibody (IDEC Pharm/Mitsubishi); ZEVALIN™ is a radiolabelled murine anti-CD20 antibody (IDEC/Schering AG); IDEC-131 is a humanized anti-CD40L antibody (IDEC/Eisai); IDEC-151 is a primatized anti-CD4 antibody (IDEC); IDEC-152 is a primatized anti-CD23 antibody (IDEC/Seikagaku); SMART anti-CD3 is a humanized anti-CD3 IgG (Protein Design Lab); 5G1.1 is a humanized anti-complement factor 5 (C5) antibody (Alexion Pharm); D2E7 is a humanized anti-TNF-α antibody (CAT/BASF); CDP870 is a humanized anti-TNF-α Fab fragment (Celltech); IDEC-151 is a primatized anti-CD4 IgG1 antibody (IDEC Pharm/SmithKline Beecham); MDX-CD4 is a human anti-CD4 IgG antibody (Medarex/Eisai/Genmab); CDP571 is a humanized anti-TNF-αIgG4 antibody (Celltech); LDP-02 is a humanized anti-α4β7 antibody (LeukoSite/Genentech); OrthoClone OKT4A is a humanized anti-CD4 IgG antibody (Ortho Biotech); ANTOVA™ is a humanized anti-CD40L IgG antibody (Biogen); ANTEGREN™ is a humanized anti-VLA-4 IgG antibody (Elan); MDX-33 is a human anti-CD64 (FcγR) antibody (Medarex/Centeon); SCH55700 is a humanized anti-IL-5 IgG4 antibody (Celltech/Schering); SB-240563 and SB-240683 are humanized anti-IL-5 and IL-4 antibodies, respectively, (SmithKline Beecham); rhuMab-E25 is a humanized anti-IgE IgG1 antibody (Genentech/Norvartis/Tanox Biosystems); ABX-CBL is a murine anti CD-147 IgM antibody (Abgenix); BTI-322 is a rat anti-CD2 IgG antibody (MedimmuneBio Transplant); Orthoclone/OKT3 is a murine anti-CD3 IgG2a antibody (ortho Biotech); SIMULECT™ is a chimeric anti-CD25 IgG1 antibody (Novartis Pharm); LDP-01 is a humanized anti-β2-integrin IgG antibody (LeukoSite); Anti-LFA-1 is a murine anti CD18 F(ab')2 (Pasteur-Merieux/humunotech); CAT-152 is a human anti-TGF-β2 antibody (Cambridge Ab Tech); and Corsevin M is a chimeric anti-Factor VII antibody (Centocor). The above-listed immunoreactive reagents, as well as any other immunoreactive reagents, may be administered according to any regimen known to those of skill in the art, including the regimens recommended by the suppliers of the immunoreactive reagents. In a preferred embodiment, molecular complexes of the invention is administered in combination with anti-CTLA4 antibody, or an anti-41BB antibody.

In another embodiment, complexes of the invention is administered in combination with one or more anti-angiogenic agents, which includes, but is not limited to, angiostatin, thalidomide, kringle 5, endostatin, Serpin (Serine Protease Inhibitor) anti-thrombin, 29 kDa N-terminal and a 40 kDa C-terminal proteolytic fragments of fibronectin, 16 kDa proteolytic fragment of prolactin, 7.8 kDa proteolytic fragment of platelet factor-4, a 13-amino acid peptide corresponding to a fragment of platelet factor-4 (Maione et al., 1990, Cancer Res. 51:2077-2083), a 14-amino acid peptide corresponding to a fragment of collagen I (Tolma et al., 1993, J. Cell Biol. 122:497-511), a 19 amino acid peptide corresponding to a fragment of Thrombospondin I (Tolsma et al., 1993, J. Cell Biol. 122:497-511), a 20-amino acid peptide corresponding to a fragment of SPARC (Sage et al., 1995, J. Cell. Biochem. 57:1329-1334), or any fragments, family members, or variants thereof, including pharmaceutically acceptable salts thereof.

Other peptides that inhibit angiogenesis and correspond to fragments of laminin, fibronectin, procollagen, and EGF have also been described (see, *e.g*., Cao, 1998, Prog Mol Subcell Biol. 20:161-176). Monoclonal antibodies and cyclic pentapeptides, which block certain integrins that bind RGD proteins (i.e., possess the peptide motif Arg-Gly-Asp), have been demonstrated to have anti-vascularization activities (Brooks et al., 1994, Science 264:569-571; Hammes et al., 1996, Nature Medicine 2:529-533). Moreover, inhibition of the urokinase plasminogen activator receptor by receptor antagonists inhibits angiogenesis, tumor growth and metastasis (Min et al., 1996, Cancer Res. 56: 2428-33; Crowley et al., 1993, Proc Natl Acad Sci. 90:5021-25). Use of such anti-angiogenic agents in combination with the complexes is also contemplated by the present invention.

In yet another embodiment, complexes of the invention is used in association with a hormonal treatment. Hormonal therapeutic treatments comprise hormonal agonists, hormonal antagonists (*e.g*., flutamide, bicalutamide, tamoxifen, raloxifene, leuprolide acetate (LUPRON), LH-RH antagonists), inhibitors of hormone biosynthesis and processing, and steroids (*e.g*., dexamethasone, retinoids, deltoids, betamethasone, cortisol, cortisone, prednisone, dehydrotestosterone, glucocorticoids, mineralocorticoids, estrogen, testosterone, progestins), vitamin A derivatives (*e.g*., all-trans retinoic acid (ATRA)); vitamin D3 analogs; antigestagens (*e.g*., mifepristone, onapristone), and antiandrogens (*e.g*., cyproterone acetate).

In yet another embodiment, complexes of the invention are used in association with a gene therapy program in the treatment of cancer. In one embodiment, gene therapy with recombinant cells secreting interleukin-2 is administered in combination with complexes of the invention to prevent or treat cancer, particularly breast cancer (See, *e.g*., Deshmukh et al., 2001, J Neurosurg. 94:287-92). In other embodiments, gene therapy is conducted with the use of polynucleotide compounds, such as but not limited to antisense polynucleotides, ribozymes, RNA interference molecules, triple helix polynucleotides and the like, where the nucleotide sequence of such compounds are related to the nucleotide sequences of DNA and/or RNA of genes that are linked to the initiation, progression, and/or pathology of a tumor or cancer. For example, many are oncogenes, growth factor genes, growth factor receptor genes, cell cycle genes, DNA repair genes, and are well known in the art.

In another embodiment, complexes of the invention is administered in conjunction with a regimen of radiation therapy. For radiation treatment, the radiation can be gamma rays or X-rays. The methods encompass treatment of cancer comprising radiation therapy, such as external-beam radiation therapy, interstitial implantation of radioisotopes (I-125, palladium, iridium), radioisotopes such as strontium-89, thoracic radiation therapy, intraperitoneal P-32 radiation therapy, and/or total abdominal and pelvic radiation therapy. For a general overview of radiation therapy, see Hellman, Chapter 16: Principles of Cancer Management: Radiation Therapy, 6th edition, 2001, DeVita et al., eds., J.B. Lippencott Company, Philadelphia. In preferred embodiments, the radiation treatment is administered as external beam radiation or teletherapy wherein the radiation is directed from a remote source. In various preferred embodiments, the radiation treatment is administered as internal therapy or brachytherapy wherein a radiaoactive source is placed inside the body close to cancer cells or a tumor mass. Also encompassed is the combined use of complexes of the invention with photodynamic therapy comprising the administration of photosensitizers, such as hematoporphyrin and its derivatives, Vertoporfin (BPD-MA), phthalocyanine, photosensitizer Pc4, demethoxy-hypocrellin A; and 2BA-2-DMHA.

In various embodiments, complexes of the invention is administered, in combination with at least one chemotherapeutic agent, for a short treatment cycle to a cancer patient to treat cancer. The duration of treatment with the chemotherapeutic agent may vary according to the particular cancer therapeutic agent used. The invention also contemplates discontinuous administration or daily doses divided into several partial administrations. An appropriate treatment time for a particular cancer therapeutic agent will be appreciated by the skilled artisan, and the invention contemplates the continued assessment of optimal treatment schedules for each cancer therapeutic agent. The present invention contemplates at least one cycle, preferably more than one cycle during which a single therapeutic or sequence of therapeutics is administered. An appropriate period of time for one cycle will be appreciated by the skilled artisan, as will the total number of cycles, and the interval between cycles.

In another embodiment, complexes of the invention are used in combination with compounds that ameliorate the symptoms of the cancer (such as but not limited to pain) and the side effects produced by the complexes of the invention (such as but not limited to flu-like symptoms, fever, etc). Accordingly, many compounds known to reduce pain, flu-like symptoms, and fever can be used in combination or in admixture with complexes of the invention. Such compounds include analgesics (*e.g*, acetaminophen), decongestants (*e.g*., pseudoephedrine), antihistamines (*e.g*., chlorpheniramine maleate), and cough suppressants (*e.g*., dextromethorphan).

### 5.8.2 TARGET INFECTIOUS DISEASES

Infectious diseases that can be treated or prevented by the methods of the present invention are caused by infectious agents including, but not limited to, viruses, bacteria, fungi protozoa, helminths, and parasites. The invention is not limited to treating or preventing infectious diseases caused by intracellular pathogens. Many medically relevant microorganisms have been described extensively in the literature, *e.g*., see C.G.A Thomas, Medical Microbiology, Bailliere Tindall, Great Britain 1983.

Combination therapy encompasses in addition to the administration of complexes of the invention, the uses of one or more modalities that aid in the prevention or treatment of infectious diseases, which modalities include, but is not limited to antibiotics, antivirals, antiprotozoal compounds, antifungal compounds, and antihelminthics. Other treatment modalities that can be used to treat or prevent infectious diseases include immunotherapeutics, polynucleotides, antibodies, cytokines, and hormones as described above.

Infectious virus of both human and non-human vertebrates, include retroviruses, RNA viruses and DNA viruses. Examples of virus that have been found in humans include but are not limited to: Retroviridae (*e.g*. human immunodeficiency viruses, such as HIV-1 (also referred to as HTLV-III, LAV or HTLV-III/LAV, or HIV-III; and other isolates, such as HIV-LP; Picomaviridae (*e.g*. polio viruses, hepatitis A virus; enteroviruses, human Coxsackie viruses, rhinoviruses, echoviruses); Calciviridae (*e.g*. strains that cause gastroenteritis); Togaviridae (*e.g*. equine encephalitis viruses, rubella viruses); Flaviridae (*e.g*. dengue viruses, encephalitis viruses, yellow fever viruses); Coronaviridae (*e.g*. coronaviruses); Rhabdoviridae (*e.g*. vesicular stomatitis viruses, rabies viruses); Filoviridae (*e.g*. ebola viruses); Paramyxoviridae (*e.g*. parainfluenza viruses, mumps virus, measles virus, respiratory syncytial virus); Orthomyxoviridae (*e.*g. influenza viruses); Bungaviridae (*e.g*. Hantaan viruses, bunga viruses, phleboviruses and Nairo viruses); Arena viridae (hemorrhagic fever viruses); Reoviridae (*e.*g. reoviruses, orbiviurses and rotaviruses); Birnaviridae; Hepadnaviridae (Hepatitis B virus); Parvovirida (parvoviruses);
Papovaviridae (papilloma viruses, polyoma viruses); Adenoviridae (most adenoviruses); Herpesviridae (herpes simplex virus (HSV) 1 and 2, varicella zoster virus, cytomegalovirus (CMV), herpes virus; Poxviridae (variola viruses, vaccinia viruses, pox viruses); and Iridoviridae (*e.g*. African swine fever virus); and unclassified viruses (*e.g*. the etiological agents of Spongiform encephalopathies, the agent of delta hepatitis (thought to be a defective satellite of hepatitis B virus), the agents of non-A, non-B hepatitis (class 1=internally transmitted; class 2=parenterally transmitted (i.e. Hepatitis C); Norwalk and related viruses, and astroviruses).

Retroviruses that are contemplated include both simple retroviruses and complex retroviruses. The simple retroviruses include the subgroups of B-type retroviruses, C-type retroviruses and D-type retroviruses. An example of a B-type retrovirus is mouse mammary tumor virus (MMTV). The C-type retroviruses include subgroups C-type group A (including Rous sarcoma virus (RSV), avian leukemia virus (ALV), and avian myeloblastosis virus (AMV)) and C-type group B (including murine leukemia virus (MLV), feline leukemia virus (FeLV), murine sarcoma virus (MSV), gibbon ape leukemia virus (GALV), spleen necrosis virus (SNV), reticuloendotheliosis virus (RV) and simian sarcoma virus (SSV)). The D-type retroviruses include Mason-Pfizer monkey virus (MPMV) and simian retrovirus type 1 (SRV-1). The complex retroviruses include the subgroups of lentiviruses, T-cell leukemia viruses and the foamy viruses. Lentiviruses include HIV-1, but also include HIV-2, SIV, Visna virus, feline immunodeficiency virus (FIV), and equine infectious anemia virus (EIAV). The T-cell leukemia viruses include HTLV-1, HTLV-II, simian T-cell leukemia virus (STLV), and bovine leukemia virus (BLV). The foamy viruses include human foamy virus (HFV), simian foamy virus (SFV) and bovine foamy virus (BFV).

Examples ofRNA viruses that are antigens in vertebrate animals include, but are not limited to, the following: members of the family Reoviridae, including the genus Orthoreovirus (multiple serotypes of both mammalian and avian retroviruses), the genus Orbivirus (Bluetongue virus, Eugenangee virus, Kemerovo virus, African horse sickness virus, and Colorado Tick Fever virus), the genus Rotavirus (human rotavirus, Nebraska calf diarrhea virus, murine rotavirus, simian rotavirus, bovine or ovine rotavirus, avian rotavirus); the family Picomaviridae, including the genus Enterovirus (poliovirus, Coxsackie virus A and B, enteric cytopathic human orphan (ECHO) viruses, hepatitis A virus, Simian enteroviruses, Murine encephalomyelitis (ME) viruses, Poliovirus muris, Bovine enteroviruses, Porcine enteroviruses, the genus Cardiovirus (Encephalomyocarditis virus (EMC), Mengovirus), the genus Rhinovirus (Human rhinoviruses including at least 113 subtypes; other rhinoviruses), the genus Apthovirus (Foot and Mouth disease (FMDV); the family Calciviridae, including Vesicular exanthema of swine virus, San Miguel sea lion virus, Feline picornavirus and Norwalk virus; the family Togaviridae, including the genus Alphavirus (Eastern equine encephalitis virus, Semliki forest virus, Sindbis virus, Chikungunya virus, O'Nyong-Nyong virus, Ross river virus, Venezuelan equine encephalitis virus, Western equine encephalitis virus), the genus Flavirius (Mosquito borne yellow fever virus, Dengue virus, Japanese encephalitis virus, St. Louis encephalitis virus, Murray Valley encephalitis virus, West Nile virus, Kunjin virus, Central European tick borne virus, Far Eastern tick borne virus, Kyasanur forest virus, Louping III virus, Powassan virus, Omsk hemorrhagic fever virus), the genus Rubivirus (Rubella virus), the genus Pestivirus (Mucosal disease virus, Hog cholera virus, Border disease virus); the family Bunyaviridae, including the genus Bunyvirus (Bunyamwera and related viruses, California encephalitis group viruses), the genus Phlebovirus (Sandfly fever Sicilian virus, Rift Valley fever virus), the genus Nairovirus (Crimean-Congo hemorrhagic fever virus, Nairobi sheep disease virus), and the genus Uukuvirus (Uukuniemi and related viruses); the family Orthomyxoviridae, including the genus Influenza virus (Influenza virus type A, many human subtypes); Swine influenza virus, and Avian and Equine Influenza viruses; influenza type B (many human subtypes), and influenza type C (possible separate genus); the family paramyxoviridae, including the genus Paramyxovirus (Parainfluenza virus type 1, Sendai virus, Hemadsorption virus, Parainfluenza viruses types 2 to 5, Newcastle Disease Virus, Mumps virus), the genus Morbillivirus (Measles virus, subacute sclerosing panencephalitis virus, distemper virus, Rinderpest virus), the genus Pneumovirus (respiratory syncytial virus (RSV), Bovine respiratory syncytial virus and Pneumonia virus of mice); forest virus, Sindbis virus, Chikungunya virus, O'Nyong-Nyong virus, Ross river virus, Venezuelan equine encephalitis virus, Western equine encephalitis virus), the genus Flavirius (Mosquito borne yellow fever virus, Dengue virus, Japanese encephalitis virus, St. Louis encephalitis virus, Murray Valley encephalitis virus, West Nile virus, Kunjin virus, Central European tick borne virus, Far Eastern tick borne virus, Kyasanur forest virus, Louping III virus, Powassan virus, Omsk hemorrhagic fever virus), the genus Rubivirus (Rubella virus), the genus Pestivirus (Mucosal disease virus, Hog cholera virus, Border disease virus); the family Bunyaviridae, including the genus Bunyvirus (Bunyamwera and related viruses, California encephalitis group viruses), the genus Phlebovirus (Sandfly fever Sicilian virus, Rift Valley fever virus), the genus Nairovirus (Crimean-Congo hemorrhagic fever virus, Nairobi sheep disease virus), and the genus Uukuvirus (Uukuniemi and related viruses); the family Orthomyxoviridae, including the genus Influenza virus (Influenza virus type A, many human subtypes); Swine influenza virus, and Avian and Equine Influenza viruses; influenza type B (many human subtypes), and influenza type C (possible separate genus); the family paramyxoviridae, including the genus Paramyxovirus (Parainfluenza virus type 1, Sendai virus, Hemadsorption virus, Parainfluenza viruses types 2 to 5, Newcastle Disease Virus, Mumps virus), the genus Morbillivirus (Measles virus, subacute sclerosing panencephalitis virus, distemper virus, Rinderpest virus), the genus Pneumovirus (respiratory syncytial virus (RSV), Bovine respiratory syncytial virus and Pneumonia virus of mice); the family Rhabdoviridae, including the genus Vesiculovirus (VSV), ChanBipura virus, Flanders-Hart Park virus), the genus Lyssavirus (Rabies virus), fish Rhabdoviruses, and two probable Rhabdoviruses (Marburg virus and Ebola virus); the family Arenaviridae, including Lymphocytic choriomeningitis virus (LCM), Tacaribe virus complex, and Lassa virus; the family Coronoaviridae, including Infectious Bronchitis Virus (IBV), Mouse Hepatitis virus, Human enteric corona virus, and Feline infectious peritonitis (Feline coronavirus).

Illustrative DNA viruses that are antigens in vertebrate animals include, but are not limited to: the family Poxviridae, including the genus Orthopoxvirus (Variola major, Variola minor, Monkey pox Vaccinia, Cowpox, Buffalopox, Rabbitpox, Ectromelia), the genus Leporipoxvirus (Myxoma, Fibroma), the genus Avipoxvirus (Fowlpox, other avian poxvirus), the genus Capripoxvirus (sheeppox, goatpox), the genus Suipoxvirus (Swinepox), the genus Parapoxvirus (contagious postular dermatitis virus, pseudocowpox, bovine papular stomatitis virus); the family Iridoviridae (African swine fever virus, Frog viruses 2 and 3, Lymphocystis virus of fish); the family Herpesviridae, including the alpha-Herpesviruses (Herpes Simplex Types 1 and 2, Varicella-Zoster, Equine abortion virus, Equine herpes virus 2 and 3, pseudorabies virus, infectious bovine keratoconjunctivitis virus, infectious bovine rhinotracheitis virus, feline rhinotracheitis virus, infectious laryngotracheitis virus) the Beta-herpesviruses (Human cytomegalovirus and cytomegaloviruses of swine, monkeys and rodents); the gamma-herpesviruses (Epstein-Barr virus (EBV), Marek's disease virus, Herpes saimiri, Herpesvirus ateles, Herpesvirus sylvilagus, guinea pig herpes virus, Lucke tumor virus); the family Adenoviridae, including the genus Mastadenovirus (Human subgroups A,B,C,D,E and ungrouped; simian adenoviruses (at least 23 serotypes), infectious canine hepatitis, and adenoviruses of cattle, pigs, sheep, frogs and many other species, the genus Aviadenovirus (Avian adenoviruses); and non-cultivatable adenoviruses; the family Papoviridae, including the genus Papillomavirus (Human papilloma viruses, bovine papilloma viruses, Shope rabbit papilloma virus, and various pathogenic papilloma viruses of other species), the genus Polyomavirus (polyomavirus, Simian vacuolating agent (SV-40), Rabbit vacuolating agent (RKV), K virus, BK virus, JC virus, and other primate polyoma viruses such as Lymphotrophic papilloma virus); the family Parvoviridae including the genus Adeno-associated viruses, the genus Parvovirus (Feline panleukopenia virus, bovine parvovirus, canine parvovirus, Aleutian mink disease virus, etc). Finally, DNA viruses may include viruses which do not fit into the above families such as Kuru and Creutzfeldt-Jacob disease viruses and chronic infectious neuropathic agents.

Many examples of antiviral compounds that can be used in combination with the complexes of the invention are known in the art and include but are not limited to: rifampicin, nucleoside reverse transcriptase inhibitors (*e.g*., AZT, ddI, ddC, 3TC, d4T), non-nucleoside reverse transcriptase inhibitors (*e.g*., Efavirenz, Nevirapine), protease inhibitors (*e.g*., aprenavir, indinavir, ritonavir, and saquinavir), idoxuridine, cidofovir, acyclovir, ganciclovir, zanamivir, amantadine, and Palivizumab. Other examples of anti-viral agents include but are not limited to Acemannan; Acyclovir; Acyclovir Sodium; Adefovir; Alovudine; Alvircept Sudotox; Amantadine Hydrochloride; Aranotin; Arildone; Atevirdine Mesylate; Avridine; Cidofovir; Cipamfylline; Cytarabine Hydrochloride; Delavirdine Mesylate; Desciclovir; Didanosine; Disoxaril; Edoxudine; Enviradene; Enviroxime; Famciclovir; Famotine Hydrochloride; Fiacitabine; Fialuridine; Fosarilate; Foscamet Sodium; Fosfonet Sodium; Ganciclovir; Ganciclovir Sodium; Idoxuridine; Kethoxal; Lamivudine; Lobucavir; Memotine Hydrochloride; Methisazone; Nevirapine; Penciclovir; Pirodavir; Ribavirin; Rimantadine Hydrochloride; Saquinavir Mesylate; Somantadine Hydrochloride; Sorivudine; Statolon; Stavudine; Tilorone Hydrochloride; Trifluridine; Valacyclovir Hydrochloride; Vidarabine; Vidarabine Phosphate; Vidarabine Sodium Phosphate; Viroxime; Zalcitabine; Zidovudine; Zinviroxime.

Bacterial infections or diseases that can be treated or prevented by the methods disclosed herein are caused by bacteria including, but not limited to, bacteria that have an intracellular stage in its life cycle, such as mycobacteria (*e.g*., Mycobacteria tuberculosis, M. bovis, M. avium, M. leprae, or M. africanum), rickettsia, mycoplasma, chlamydia, and legionella. Other examples of bacterial infections contemplated include but are not limited to infections caused by Gram positive bacillus (*e.g*., Listeria, Bacillus such as Bacillus anthracis, Erysipelothrix species), Gram negative bacillus (*e.g*., Bartonella, Brucella, Campylobacter, Enterobacter, Escherichia, Francisella, Hemophilus, Klebsiella, Morganella, Proteus, Providencia, Pseudomonas, Salmonella, Serratia, Shigella, Vibrio, and Yersinia species), spirochete bacteria (*e.g*., Borrelia species including Borrelia burgdorferi that causes Lyme disease), anaerobic bacteria (*e.g*., Actinomyces and Clostridium species), Gram positive and negative coccal bacteria, Enterococcus species, Streptococcus species, Pneumococcus species, Staphylococcus species, Neisseria species. Specific examples of infectious bacteria include but are not limited to: Helicobacter pyloris, Borelia burgdorferi, Legionella pneumophilia, Mycobacteria tuberculosis, M. avium, M. intracellulare, M. kansaii, M. gordonae, Staphylococcus aureus, Neisseria gonorrhoeae, Neisseria meningitidis, Listeria monocytogenes, Streptococcus pyogenes (Group A Streptococcus), Streptococcus agalactiae (Group B Streptococcus), Streptococcus viridans, Streptococcus faecalis, Streptococcus bovis, Streptococcus pneumoniae, Haemophilus influenzae, Bacillus antracis, corynebacterium Biphtheriae, Erysipelothrix rhusiopathiae, Clostridium perfringers, Clostridium tetani, Enterobacter aerogenes, Klebsiella pneumoniae, Pasturella multocida, Fusobacterium nucleatum, Streptobacillus moniliformis, Treponema pallidium, Treponema pertenue, Leptospira, Rickettsia, and Actinomyces israelli.

Antibacterial agents or antibiotics that can be used in combination with the complexes of the invention include but are not limited to: aminoglycoside antibiotics (*e.g*., apramycin, arbekacin, bambermycins, butirosin, dibekacin, neomycin, neomycin, undecylenate, netilmicin, paromomycin, ribostamycin, sisomicin, and spectinomycin), amphenicol antibiotics (*e.g*., azidamfenicol, chloramphenicol, florfenicol, and thiamphenicol), ansamycin antibiotics (*e.g*., rifamide and rifampin), carbacephems (*e.g*., loracarbef), carbapenems (*e.g*., biapenem and imipenem), cephalosporins (*e.g*., cefaclor, cefadroxil, cefamandole, cefatrizine, cefazedone, cefozopran, cefpimizole, cefpiramide, and cefpirome), cephamycins (*e.g*., cefbuperazone, cefmetazole, and cefininox), monobactams (*e.g*., aztreonam, carumonam, and tigemonam), oxacephems (*e.g*., flomoxef, and moxalactam), penicillins (*e.g*., amdinocillin, amdinocillin pivoxil, amoxicillin, bacampicillin, benzylpenicillinic acid, benzylpenicillin sodium, epicillin, fenbenicillin, floxacillin, penamccillin, penethamate hydriodide, penicillin o-benethamine, penicillin 0, penicillin V, penicillin V benzathine, penicillin V hydrabamine, penimepicycline, and phencihicillin potassium), lincosamides (*e.g*., clindamycin, and lincomycin), macrolides (*e.g*., azithromycin, carbomycin, clarithomycin, dirithromycin, erythromycin, and erythromycin acistrate), amphomycin, bacitracin, capreomycin, colistin, enduracidin, enviomycin, tetracyclines (*e.g*., apicycline, chlortetracycline, clomocycline, and demeclocycline), 2,4-diaminopyrimidines (*e.g*., brodimoprim), nitrofurans (*e.g*., furaltadone, and furazolium chloride), quinolones and analogs thereof (*e.g*., cinoxacin, ciprofloxacin, clinafloxacin, flumequine, and grepagloxacin), sulfonamides (*e.g*., acetyl sulfamethoxypyrazine, benzylsulfamide, noprylsulfamide, phthalylsulfacetamide, sulfachrysoidine, and sulfacytine), sulfones (*e.g*., diathymosulfone, glucosulfone sodium, and solasulfone), cycloserine, mupirocin and tuberin.

Additional examples of antibacterial agents include but are not limited to Acedapsone; Acetosulfone Sodium; Alamecin; Alexidine; Amdinocillin; Amdinocillin Pivoxil; Amicycline; Amifloxacin; Amifloxacin Mesylate; Amikacin; Amikacin Sulfate; Aminosalicylic acid; Aminosalicylate sodium; Amoxicillin; Amphomycin; Ampicillin; Ampicillin Sodium; Apalcillin Sodium; Apramycin; Aspartocin; Astromicin Sulfate; Avilamycin; Avoparcin; Azithromycin; Azlocillin; Azlocillin Sodium; Bacampicillin Hydrochloride; Bacitracin; Bacitracin Methylene Disalicylate; Bacitracin Zinc; Bambermycins; Benzoylpas Calcium; Berythromycin; Betamicin Sulfate; Biapenem; Biniramycin; Biphenamine Hydrochloride; Bispyrithione Magsulfex; Butikacin; Butirosin Sulfate; Capreomycin Sulfate; Carbadox; Carbenicillin Disodium; Carbenicillin Indanyl Sodium; Carbenicillin Phenyl Sodium; Carbenicillin Potassium; Carumonam Sodium; Cefaclor; Cefadroxil; Cefamandole; Cefamandole Nafate; Cefamandole Sodium; Cefaparole; Cefatrizine; Cefazaflur Sodium; Cefazolin; Cefazolin Sodium; Cefbuperazone; Cefdinir; Cefepime; Cefepime Hydrochloride; Cefetecol; Cefixime; Cefmnenoxime Hydrochloride; Cefinetazole; Cefmetazole Sodium; Cefonicid Monosodium; Cefonicid Sodium; Cefoperazone Sodium; Ceforanide; Cefotaxime Sodium; Cefotetan; Cefotetan Disodium; Cefotiam Hydrochloride; Cefoxitin; Cefoxitin Sodium; Cefpimizole; Cefpimizole Sodium; Cefpiramide; Cefpiramide Sodium; Cefpirome Sulfate; Cefpodoxime Proxetil; Cefprozil; Cefroxadine; Cefsulodin Sodium; Ceftazidime; Ceftibuten; Ceftizoxime Sodium; Ceftriaxone Sodium; Cefuroxime; Cefuroxime Axetil; Cefuroxime Pivoxetil; Cefuroxime Sodium; Cephacetrile Sodium; Cephalexin; Cephalexin Hydrochloride; Cephaloglycin; Cephaloridine; Cephalothin Sodium; Cephapirin Sodium; Cephradine; Cetocycline Hydrochloride; Cetophenicol; Chloramphenicol; Chloramphenicol Palmitate; Chloramphenicol Pantothenate Complex; Chloramphenicol Sodium Succinate; Chlorhexidine Phosphanilate; Chloroxylenol; Chlortetracycline Bisulfate; Chlortetracycline Hydrochloride; Cinoxacin; Ciprofloxacin; Ciprofloxacin Hydrochloride; Cirolemycin; Clarithromycin; Clinafloxacin Hydrochloride; Clindamycin; Clindamycin Hydrochloride; Clindamycin Palmitate Hydrochloride; Clindamycin Phosphate; Clofazimine; Cloxacillin Benzathine; Cloxacillin Sodium; Cloxyquin; Colistimethate Sodium; Colistin Sulfate; Coumermycin; Coumermycin Sodium; Cyclacillin; Cycloserine; Dalfopristin; Dapsone; Daptomycin; Demeclocycline; Demeclocycline Hydrochloride; Demecycline; Denofungin; Diaveridine; Dicloxacillin; Dicloxacillin Sodium; Dihydrostreptomycin Sulfate; Bipyrithione; Dirithromycin; Doxycycline; Doxycycline Calcium; Doxycycline Fosfatex; Doxycycline Hyclate; Droxacin Sodium; Enoxacin; Epicillin; Epitetracycline Hydrochloride; Erythromycin; Erythromycin Acistrate; Erythromycin Estolate; Erythromycin Ethylsuccinate; Erythromycin Gluceptate; Erythromycin Lactobionate; Erythromycin Propionate; Erythromycin Stearate; Ethambutol Hydrochloride; Ethionamide; Fleroxacin; Floxacillin; Fludalanine; Flumequine; Fosfomycin; Fosfomycin Tromethamine; Fumoxicillin; Furazolium Chloride; Furazolium Tartrate; Fusidate Sodium; Fusidic Acid; Gentamicin Sulfate; Gloximonam; Gramicidin; Haloprogin; Hetacillin; Hetacillin Potassium; Hexedine; Ibafloxacin; Imipenem; Isoconazole; Isepamicin; Isoniazid; Josamycin; Kanamycin Sulfate; Kitasamycin; Levofuraltadone; Levopropylcillin Potassium; Lexithromycin; Lincomycin; Lincomycin Hydrochloride; Lomefloxacin; Lomefloxacin Hydrochloride; Lomefloxacin Mesylate; Loracarbef; Mafenide; Meclocycline; Meclocycline Sulfosalicylate; Megalomicin Potassium Phosphate; Mequidox; Meropenem; Methacycline; Methacycline Hydrochloride; Methenamine; Methenamine Hippurate; Methenamine Mandelate; Methicillin Sodium; Metioprim; Metronidazole Hydrochloride; Metronidazole Phosphate; Mezlocillin; Mezlocillin Sodium; Minocycline; Minocycline Hydrochloride; Mirincamycin Hydrochloride; Monensin; Monensin Sodium; Nafcillin Sodium; Nalidixate Sodium; Nalidixic Acid; Natamycin; Nebramycin; Neomycin Palmitate; Neomycin Sulfate; Neomycin Undecylenate; Netilmicin Sulfate; Neutramycin; Nifuradene; Nifuraldezone; Nifuratel; Nifuratrone; Nifurdazil; Nifurimide; Nifurpirinol; Nifurquinazol; Nifurthiazole; Nitrocycline; Nitrofurantoin; Nitromide; Norfloxacin; Novobiocin Sodium; Ofloxacin; Ormetoprim; Oxacillin Sodium; Oximonam; Oximonam Sodium; Oxolinic Acid; Oxytetracycline; Oxytetracycline Calcium; Oxytetracycline Hydrochloride; Paldimycin; Parachlorophenol; Paulomycin; Pefloxacin; Pefloxacin Mesylate; Penamecillin; Penicillin G Benzathine; Penicillin G Potassium; Penicillin G Procaine; Penicillin G Sodium; Penicillin V; Penicillin V Benzathine; Penicillin V Hydrabamine; Penicillin V Potassium; Pentizidone Sodium; Phenyl Aminosalicylate; Piperacillin Sodium; Pirbenicillin Sodium; Piridicillin Sodium; Pirlimycin Hydrochloride; Pivampicillin Hydrochloride; Pivampicillin Pamoate; Pivampicillin Probenate; Polymyxin B Sulfate; Porfiromycin; Propikacin; Pyrazinamide; Pyrithione Zinc; Quindecamine Acetate; Quinupristin; Racephenicol; Ramoplanin; Ranimycin; Relomycin; Repromicin; Rifabutin; Rifametane; Rifamexil; Rifamide; Rifampin; Rifapentine; Rifaximin; Rolitetracycline; Rolitetracycline Nitrate; Rosaramicin; Rosaramicin Butyrate; Rosaramicin Propionate; Rosaramicin Sodium Phosphate; Rosaramicin Stearate; Rosoxacin; Roxarsone; Roxithromycin; Sancycline; Sanfetrinem Sodium; Sarmoxicillin; Sarpicillin; Scopafingin; Sisomicin; Sisomicin Sulfate; Sparfloxacin; Spectinomycin Hydrochloride; Spiramycin; Stallimycin Hydrochloride; Steffimycin; Streptomycin Sulfate; Streptonicozid; Sulfabenz; Sulfabenzamide; Sulfacetamide; Sulfacetamide Sodium; Sulfacytine; Sulfadiazine; Sulfadiazine Sodium; Sulfadoxine; Sulfalene; Sulfamerazine; Sulfameter; Sulfamethazine; Sulfamethizole; Sulfamethoxazole; Sulfamonomethoxine; Sulfamoxole; Sulfanilate Zinc; Sulfanitran; Sulfasalazine; Sulfasomizole; Sulfathiazole; Sulfazamet; Sulfisoxazole; Sulfisoxazole Acetyl; Sulfisoxazole Diolamine; Sulfomyxin; Sulopenem; Sultamicillin; Suncillin Sodium; Talampicillin Hydrochloride; Teicoplanin; Temafloxacin Hydrochloride; Temocillin; Tetracycline; Tetracycline Hydrochloride; Tetracycline Phosphate Complex; Tetroxoprim; Thiamphenicol; Thiphencillin Potassium; Ticarcillin Cresyl Sodium; Ticarcillin Disodium; Ticarcillin Monosodium; Ticlatonc; Tiodonium Chloride; Tobramycin; Tobramycin Sulfate; Tosufloxacin; Trimethoprim; Trimethoprim Sulfate; Trisulfapyrimidines; Troleandomycin; Trospectomycin Sulfate; Tyrothricin; Vancomycin; Vancomycin Hydrochloride; Virgimamycin; Zorbamycin.

Fungal diseases that can be treated or prevented by the methods disclosed herein include but not limited to aspergilliosis, crytococcosis, sporotrichosis, coccidioidomycosis, paracoccidioidomycosis, histoplasmosis, blastomycosis, zygomycosis, and candidiasis.

Antifungal compounds that can be used in combination with the complexes of the invention include but are not limited to: polyenes (*e.g.*, amphotericin b, candicidin, mepartricin, natamycin, and nystatin), allylamines (*e.g.*, butenafine, and naftifine), imidazoles (*e.g*., bifonazole, butoconazole, chlordantoin, flutrimazole, isoconazole, ketoconazole, and lanoconazole), thiocarbamates (*e.g*., tolciclate, tolindate, and tolnaftate), triazoles (*e.g*., fluconazole, itraconazole, saperconazole, and terconazole), bromosalicylchloranilide, buclosamide, calcium propionate, chlorphenesin, ciclopirox, azaserine, griseofulvin, oligomycins, neomycin undecylenate, pyrrolnitrin, siccanin, tubercidin, and viridin. Additional examples of antifungal compounds include but are not limited to Acrisorcin; Ambruticin; Amphotericin B; Azaconazole; Azaserine; Basifungin; Bifonazole; Biphenamine Hydrochloride; Bispyrithione Magsulfex; Butoconazole Nitrate; Calcium Undecylenate; Candicidin; Carbol-Fuchsin; Chlordantoin; Ciclopirox; Ciclopirox Olamine; Cilofungin; Cisconazole; Clotrimazole; Cuprimyxin; Denofungin; Bipyritlione; Doconazole; Econazole; Econazole Nitrate; Enilconazole; Ethonarn Nitrate; Fenticonazole Nitrate; Filipin; Fluconazole; Flucytosine; Fungimycin; Griseofulvin; Hamycin; Isoconazole; Itraconazole; Kalafungin; Ketoconazole; Lomofingin; Lydimycin; Mepartricin; Miconazole; Miconazole Nitrate; Monensin; Monensin Sodium; Naftifme Hydrochloride; Neomycin Undecylenate; Nifuratel; Nifurmerone; Nitralamine Hydrochloride; Nystatin; Octanoic Acid; Orconazole Nitrate; Oxiconazole Nitrate; Oxifungin Hydrochloride; Parconazole Hydrochloride; Partricin; Potassium Iodide; Proclonol; Pyrithione Zinc; Pyrrolnitrin; Rutamycin; Sanguinarium Chloride; Saperconazole; Scopafungin; Selenium Sulfide; Sinefungin; Sulconazole Nitrate; Terbinafine; Terconazole; Thiram; Ticlatone; Tioconazole; Tolciclate; Tolindate; Tolnaftate; Triacetin; Triafuigin; Undecylenic Acid; Viridoflilvin; Zinc Undecylenate; and Zinoconazole Hydrochloride.

Parasitic diseases that can be treated or prevented by the methods disclosed herein include , but are not limited to, amebiasis, malaria, leishmania, coccidia, giardiasis, cryptosporidiosis, toxoplasmosis, and trypanosomiasis. Also encompassed are infections by various worms, such as but not limited to ascariasis, ancylostomiasis, trichuriasis, strongyloidiasis, toxoccariasis, trichinosis, onchocerciasis, filaria, and dirofilariasis. Also encompassed are infections by various flukes, such as but not limited to schistosomiasis, paragonimiasis, and clonorchiasis. Parasites that cause these diseases can be classified based on whether they are intracellular or extracellular. An "intracellular parasite" as used herein is a parasite whose entire life cycle is intracellular. Examples of human intracellular parasites include Leishmania spp., Plasmodium spp., Trypanosoma cruzi, Toxoplasma gondii, Babesia spp., and Trichinella spiralis. An "extracellular parasite" as used herein is a parasite whose entire life cycle is extracellular. Extracellular parasites capable of infecting humans include Entamoeba histolytica, Giardia lamblia, Enterocytozoon bieneusi, Naegleria and Acanthamoeba as well as most helminths. Yet another class of parasites is defined as being mainly extracellular but with an obligate intracellular existence at a critical stage in their life cycles. Such parasites are referred to herein as "obligate intracellular parasites". These parasites may exist most of their lives or only a small portion of their lives in an extracellular environment, but they all have at least one obligate intracellular stage in their life cycles. This latter category of parasites includes Trypanosoma rhodesiense and Trypanosoma gambiense, Isospora spp., Cryptosporidium spp, Eimeria spp., Neospora spp., Sarcocystis spp., and Schistosoma spp.

Many examples of antiprotozoal compounds that can be used in combination with the complexes of the invention to treat parasitic diseases are known in the art and include but are not limited to: quinines, chloroquine, mefloquine, proguanil, pyrimethamine, metronidazole, diloxanide furoate, tinidazole, amphotericin, sodium stibogluconate, trimoxazole, and pentamidine isetionate. Many examples of antiparasite drugs that can be used in combination with the complexes of the invention to treat parasitic diseases are known in the art and include but are not limited to: mebendazole, levamisole, niclosamide, praziquantel, albendazole, ivermectin, diethylcarbamazine, and thiabendazole. Further examples of anti-parasitic compounds include but are not limited to Acedapsone; Amodiaquine Hydrochloride; Amquinate; Arteflene; Chloroquine; Chloroquine Hydrochloride; Chloroquine Phosphate; Cycloguanil Pamoate; Enpiroline Phosphate; Halofantrine Hydrochloride; Hydroxychloroquine Sulfate; Mefloquine Hydrochloride; Menoctone; Mirincamycin Hydrochloride; Primaquine Phosphate; Pyrimethamine; Quinine Sulfate; and Tebuquine.

In a less preferred embodiment, the complexes of the invention can be used in combination with a non-HSP and non-α2M-based vaccine composition. Examples of such vaccines for humans are described in The Jordan Report 2000, Accelerated Development of Vaccines, National Institute of Health . Many vaccines for the treatment of non-human vertebrates are disclosed in Bennett, K. Compendium of Veterinary Products, 3rd ed. North American Compendiums, Inc., 1995, which is incorporated herein by reference in its entirety.

### 5.8.3 Targeting Other Diseases

In addition to cancer and infectious diseases, other diseases including, but not limited to, anemia, growth hormone deficiencies, enzyme deficiency diseases, and conditions of immune suppression, can also be treated or prevented by the methods of the present invention.

Anemia may be caused by various reasons, for example, it may be caused by iron deficiency, folic acid deficiency, chronic diseases (*e.g.*, chronic infection or inflammation, cancer, liver diseases, chronic renal failure), chemotherapy, etc.

Growth hormone is secreted by anterior pituitary gland in human. Growth hormone deficiency in adulthood tends to cause mild to moderate obesity, asthenia, and reduced cardiac output. Human growth hormone can be synthesized by recombinant DNA techniques. Patients with hypopituitarism and severe growth hormone deficiency can be treated with human growth hormone.

There are many different kinds of enzyme deficiency diseases. Non-limiting examples are Debrancher enzyme deficiency (also known as Cori's or Forbes' Disease), glycogen storage diseases (*e.g*., glycogen debranching enzyme deficiency), glucose-6-phosphate dehydrogenase(G6PD) deficiency, galactosylcereamidase deficiency (Krabbe disease), etc.

While not limited by any theory, one of the possible explanations of the therapeutic or prophylactic effects of the molecular complexes of the invention or the pharmaceutical compositions comprising the molecular complexes of the invention for treating or preventing diseases such as anemia, growth hormone deficiency, and enzyme deficiency diseases is that oligomerization of an immunologically and/or biologically active glycoprotein, which has therapeutic or prophylactic effect on such diseases, may improve the therapeutic or prophylactic effect of the glycoprotein as compared to the un-oligomerized glycoprotein. For example, oligomerized glycoprotein can be more easily targeted to a desirable site or a desirable cell type, *e.g*., by binding of the lectin in the complex to cell surface glycoprotein receptors. Thus, preferably the lectin is in molar excess in the complex. Alternatively, oligomerized glycoprotein can be more easily taken up by its target cells by either receptor mediated events or non-receptor mediated events.

Hormones and enzymes that are known in the art for treatment or prevention of diseases, such as but not limited to, anemia, hormone deficiencies, or enzyme deficiencies, can be used in accordance with the present invention. Hormones and enzymes that are naturally occurring glycoproteins (*e.g*., erythropoietin) can form oligomers in the presence of a lectin and be used in accordance with the present invention. Hormones and enzymes that are not naturally occurring glycoprotein can be engineered to add one or more carbohydrate groups and used in accordance with the present invention. In one embodiment, the present invention provides a method for treating anemia comprising administering to a subject in need thereof a composition comprising one or more molecular complexes, wherein each complex comprises a lectin and a erythropoietin (EPO). Preferably, the subject is a human, and the EPO administered is a human EPO. EPO is a glycoprotein hormone produced primarily by cells of the peritubular capillary endothelium of the kidney, and is responsible for the regulation of red blood cell production. In another embodiment, the present invention provides a method of treating an enzyme deficiency disease comprising administering a composition comprising a lectin and a glucocerebrosidase. Preferably, the enzyme deficiency disease to be treated is Gaucher disease.

Immune suppression conditions may be caused by variety of reasons, including but not limited to, cancer (*e.g.*, thymoma, Hodgkin's disease), Acquired Immune Deficiency Syndrome (AIDS), sarcoidosis, and chemotherapies. Proteins that are known to stimulate the immune system can be used in accordance with the present invention to treat or prevent an immune suppression condition. In one embodiment, the present invention provides a method of treating or preventing an immune suppression condition comprising administering to a subject in need thereof a composition comprising one or more molecular complexes, wherein each complex comprises a lectin and a granulocyte-macrophage colony stimulating factor (GM-CSF). In another embodiment, the present invention provides a method of treating or preventing an immune suppression condition comprising administering to a subject in need thereof a composition comprising one or more molecular complexes, wherein each complex comprises a lectin and a granulocyte colony stimulating factor (G-CSF).

### 5.8.4 Autologous Embodiment

The specific immunogenicity of HSPs derives not from HSPs *pe*r *se*, but from the antigenic proteins bound to them. In a preferred embodiment of the invention, the complexes in the compositions of the inventions for use as cancer vaccines are autologous complexes, thereby circumventing two of the most intractable hurdles to cancer immunotherapy. First is the possibility that human cancers, like cancers of experimental animals, are antigenically distinct. To circumvent this hurdle, in a preferred embodiment of the present invention, the lectin-HSPs are complexed to antigenic proteins, and the complexes are used to treat the cancers in the same subject from which the proteins are derived. Second, most current approaches to cancer immunotherapy focus on determining the CTL-recognized epitopes of cancer cell lines. This approach requires the availability of cell lines and CTLs against cancers. These reagents are unavailable for an overwhelming proportion of human cancers. In an embodiment of the present invention directed to the use of autologous antigenic proteins, cancer immunotherapy does not depend on the availability of cell lines or CTLs nor does it require definition of the antigenic epitopes of cancer cells. These advantages make complexes of lectin-HSPs bound to autologous antigenic proteins attractive immunogens against cancer.

In some embodiments, the antigenic proteins in the therapeutic or prophylactic complexes can be prepared from cancerous tissue of the same type of cancer from a subject allogeneic to the subject to whom the complexes are administered.

### 5.9. Pharmaceutical Preparations And Methods of Administration

The molecular complexes and pharmaceutical compositions of the invention can be administered to a patient at therapeutically effective doses to treat or ameliorate a disease or disorder (*e.g.*, cancer, infectious disease, anemia, immunosuppressive conditions, enzyme deficiencies or hormone deficiencies). A therapeutically effective dose refers to that amount of the complexes sufficient to result in amelioration of symptoms of such a disorder. The effective dose of the complexes may be different when another treatment modality is being used in combination. The appropriate and recommended dosages, formulation and routes of administration for treatment modalities such as chemotherapeutic agents, radiation therapy and biological/immunotherapeutic agents such as cytokines are known in the art (e.g., as described in such literature as the Physicians' Desk Reference (56th ed., 2002, 57th ed., 2003, and 58th ed., 2004)), or can be used in accordance with manufacturer's instructions or directions.

### 5.9.1 Effective Dose

Toxicity and therapeutic efficacy of the molecular complexes of the invention can be determined by standard pharmaceutical procedures in cell cultures or experimental animals, *e.g.*, for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Complexes that exhibit large therapeutic indices are preferred. While complexes that exhibit toxic side effects may be used, care should be taken to design a delivery system that targets such complexes to the site of affected tissue in order to minimize potential damage to uninfected cells and, thereby, reduce side effects.

In one embodiment, the data obtained from the cell culture assays and animal studies can be used in formulating a range of dosage for use in humans. The dosage of complexes lies preferably within a range of circulating concentrations that include the ED50 with little or no toxicity. The dosage may vary within this range depending upon the dosage form employed and the route of administration utilized. For any complexes used in the method disclosed herein, the therapeutically effective dose can be estimated initially from cell culture assays. A dose may be formulated in animal models to achieve a circulating plasma concentration range that includes the IC50 (*i.e*., the concentration of the test compound that achieves a half maximal inhibition of symptoms) as determined in cell culture. Such information can be used to more accurately determine useful doses in humans. Levels in plasma may be measured, for example, by high performance liquid chromatography.

In another embodiment, the amount of molecular complexes of the invention comprising lectins and Hsp96-Antigenic Molecule complexes that is administered to a subject is in the range of about Inanogram to about 600 micrograms for a human patient. The preferred human dosage is the same as used in a 25g mouse, *i.e*., in the range of about 1-10ng, about 20 ng, about 30ng, about 40ng, about 50ng, about 70ng, about 100ng, about 200ng, about 300ng, about 400ng, about 500ng, about 600ng, about 700ng, about 800ng, about 900ng, about 1µg, about 10µg, about 25µg, about 50µg, about 100µg, about 200µg, about 300µg, about 400µg, about 500µg, or about 600µg. The dosage for molecular complexes of the invention comprising lectin associated with any other HSP complexes in a human patient is in the range of about 5 to 5,000 micrograms, the preferred dosage being 100 microgram. These doses are preferably administered intradermally, subcutaneously, intramuscularly, intravenously, or intraperitoneally. These doses can be given once or repeatedly, such as daily, every other day, weekly, biweekly, or monthly. Preferably, the complexes are given once weekly for a period of about 4-6 weeks, and the mode or site of administration is preferably varied with each administration. Thus, by way of example and not limitation, the first injection may be given subcutaneously on the left arm, the second on the right arm, the third on the left belly, the fourth on the right belly, the fifth on the left thigh, the sixth on the right thigh, etc. The same site may be repeated after a gap of one or more injections. Also, split injections maybe given. Thus, for example, half the dose may be given in one site and the other half on another site on the same day. Alternatively, the mode of administration is sequentially varied, e.g., weekly injections are given in sequence intradermally, intramuscularly, subcutaneously, intravenously or intraperitoneally. Preferably, the once weekly dose is given for a period of 4 weeks. After 4-6 weeks, further injections are preferably given at two-week intervals over a period of time of one or more months, or until supply of complexes is exhausted. The pace of later injections may be modified, depending upon the patient's clinical progress and responsiveness to the immunotherapy. In a preferred example, intradermal administrations are given, with each site of administration varied sequentially.

Accordingly, the description discloses methods of preventing and treating cancer or an infectious disease in a subject comprising administering a composition which stimulates the immunocompetence of the host individual and elicits specific immunity against the preneoplastic and/or neoplastic cells or infected cells.

In a specific embodiment, during combination therapy, the molecular complexes of the invention (e.g., molecular complexes comprising a lectin and an HSP) are administered in a sub-optimal amount, *e.g*., an amount that does not manifest detectable therapeutic benefits when administered in the absence of the therapeutic modality, as determined by methods know in the art. In such methods, the administration of such a sub-optimal amount of a molecular complex of the invention to a subject receiving a therapeutic modality results in an overall improvement in effectiveness of treatment. In another specific embodiment, a therapeutic modality that does not comprise the molecular complexes of the invention is administered in a sub-optimal amount during combination therapy. In such methods, the administration of such a sub-optimal amount of the therapeutic modality to a subject receiving a molecular complex of the invention results in an overall improvement in effectiveness of treatment.

In one embodiment, one or more molecular complexes of the invention are administered in an amount that does not result in tumor regression or cancer remission or an amount wherein the cancer cells have not been significantly reduced or have increased when said molecular complexes is administered in the absence of another therapeutic modality. In another embodiment, the sub-optimal amount of molecular complexes of the invention is administered to a subject receiving a treatment modality whereby the overall effectiveness of treatment is improved. Among these subjects being treated with the molecular complexes of the invention are those receiving chemotherapy or radiation therapy. A sub-optimal amount can be determined by appropriate animal studies. Such a sub-optimal amount in humans can be determined by extrapolation from experiments in animals.

In one embodiment, one or more molecular complexes of the invention comprises lectin associated with a glycoprotein and an Antigenic Molecule, wherein the glycoprotein is not a heat shock protein. For example, the molecular complex of the invention may comprise erythropoietin (EPO). When EPO is used as a single drug, the commonly used initial dosage is 25-30 units per kg injection, twice or three times a week. (On average 5000-6000 units per week). Some patients can manage weekly or even every two weeks with subcutaneous injections. Intravenous EPO needs to be given a minimum of 2 or 3 times weekly. Other dosage regimens can be found in Physician's Desk References (56th ed., 2002, 57th ed., 2003, and 58th ed., 2004).

In another embodiment, the molecular complex of the invention may comprise a tissue plasminogen activator (tPA), such as Alteplase (Activase®, Genentech). Alteplase is a purified glycoprotein of 527 amino acids. Alteplase is used in management and treatment of acute myocardial infarction (AMI), acute ischemic stroke, and pulmonary embolism. Activase is administered intravenously. For management and treatment of AMI, there are two dosing regimens: accelerated infusion and 3-hour infusion. In accelerated infusion, for patients weighing > 67 kg, 100mg as a 15mg intravenous bolus is administered, followed by 50 mg infused over the next 30 minutes, and then 35mg infused over the next 60 minutes. For patients weighing less or equal to 67 kg, the recommended dose is administered as a 15mg intravenous bolus, followed by 0.75mg/kg infused over the next 30 minutes not to exceed 50mg, and then 0.5mg/kg over the next 60 minutes not to exceed 35mg. in the 3-hour infusion, the recommended dose is 100mg administered as 60mg in the first hour, 20mg over the second hour, and 20mg over the third hour. Dosage regimens of Alteplase in treating other disease can also be found in Physician's Desk Reference, (56th ed., 2002, 57th ed., 2003, and 58th ed., 2004) .

In another embodiment, the molecular complex of the invention may comprise a granulocyte-macrophage colony stimulating factor (GM-CSF), such as Leukine® (Berlex). Leukine® can be used, *e.g*., following induction chemotherapy in acute myelogenous leukemia, in mobiliztion and following transplantation of autologous peripheral blood progenitor cells, in myeloid reconstitution after autologous bone marrow tranplantation, in myeloid reconstitution after allogeneic bone marrow transplantation, and in bone marrow transplantation failure or engraftment delay. The dosage regimens for different disease may vary. In one example, when Leukine® is used post peripheral blood progenitor cell transplantation, the recommended dose is 250mcg/m²/day administered IV over 24 hours or SC once daily beginning immediately following infusion of progenitor cells and continuing until an ANC > 1500 cells/mm³ for 3 consecutive days in attained. Other dosage regimens can also be found in Physician's Desk Reference, (56th ed., 2002, 57th ed., 2003, and 58th ed., 2004).

In another embodiment, the molecular complex of the invention may comprise a granulocyte colony-stimulating factor (G-CSF), such Neupogen®. Neupogen® can be used, *e.g*., in cancer patients receiving myelosuppressive chemotherapy, patents with acute myeloid leukemia receiving induction or consolidation chemotherapy, cancer patients receiving bone marrow transplant, patients undergoing peripheral blood progenitor cell collection and therapy, and patients with severe chronic neutropenia. The dosage regiments are different in different disease. In one example, cancer patients receiving bone marrow transplant can be administered 10mcg/kg/day given as an IV infusion of 4 or 24 hours, or as a continuous 24-hour SC infusion. Other regimens can be found in Physician's Desk Reference, (56th ed., 2002, 57th ed., 2003, and 58th ed., 2004).

In another embodiment, the molecular complex of the invention may comprise a enzyme, such as glucocerebrosidase (Cerezyme® by Genzyme), which is used in enzyme deficiency diseases such as Gaucher disease. Cerezyme® is administered by intravenous infusion over 1-2 hours. Dosage should be individualized to each patient. Initial dosages range from 2.5U/kg of body weight 3 times a week to 60U/kg once every 2 weeks. Other regimens can be found in Physician's Desk Reference, (56th ed., 2002, 57th ed., 2003, and 58th ed., 2004).

In various embodiments, the oligomerization with lectin in accordance of the present invention may decrease the effective dosage of the drug mentioned, *e.g*., by 1, 5, 10, 20, 50, 100 fold or more. When the drug dosage is not given in weight unit, it can be converted to weight unit according to manufacturer's specification or any method known in the art, and corresponding amount of lectin present in the molecular complex can be then calculated accordingly.

### 5.9.2 Therapeutic Regimens

For any of the combination therapies described above for treatment or prevention of a disease (*e.g.*, cancer, infectious disease, anemia, immunosuppressive conditions, enzyme deficiencies or hormone deficiencies), the complexes of the invention can be administered prior to, concurrently with, or subsequent to the administration of the non-lectin-glycoprotein based modality. The non-lectin-glycoprotein based modality can be any one of the modalities described above for treatment or prevention of cancer or infectious disease (or any other treatment modality that is desirable for treatment or prevention of the disease in question).

In one embodiment, the complexes of the invention are administered to a subject at reasonably the same time as the other modality. This method provides that the two administrations are performed within a time frame of less than one minute to about five minutes, about sixty minutes, about 2 hours, about 3 hours, about 4 hours, about 6 hours, about 8 hours, or up to 12 hours from each other, for example, at the same doctor's visit.

In another embodiment, the complexes of the invention and a modality are administered at exactly the same time. In yet another embodiment the complexes of the invention and the modality are administered in a sequence and within a time interval such that the complexes of the invention and the modality can act together to provide an increased benefit than if they were administered alone. In another embodiment, the complexes of the invention and a modality are administered sufficiently close in time so as to provide the desired therapeutic or prophylactic outcome. Each can be administered simultaneously or separately, in any appropriate form and by any suitable route. In one embodiment, the complexes of the invention and the modality are administered by different routes of administration. In an alternate embodiment, each is administered by the same route of administration. The complexes of the invention can be administered at the same or different sites, *e.g.* arm and leg. When administered simultaneously, the complexes of the invention and the modality may or may not be administered in admixture or at the same site of administration by the same route of administration.

In a preferred embodiment, the complexes of the invention are administered according to the regimen described in Section 5.9.1. In various embodiments, the complexes of the invention and another modality are administered less than 1 hour apart, at about 1 hour apart, 1 hour to 2 hours apart, 2 hours to 3 hours apart, 3 hours to 4 hours apart, 4 hours to 5 hours apart, 5 hours to 6 hours apart, 6 hours to 7 hours apart, 7 hours to 8 hours apart, 8 hours to 9 hours apart, 9 hours to 10 hours apart, 10 hours to 11 hours apart, 11 hours to 12 hours apart, no more than 24 hours apart or no more than 48 hours apart. In other embodiments, the complexes of the invention and another modality are administered 2 to 4 days apart, 4 to 6 days apart, 1 week a part, 1 to 2 weeks apart, 2 to 4 weeks apart, one moth apart, 1 to 2 months apart, or 2 or more months apart. In preferred embodiments, the complexes of the invention and another modality are administered in a time frame where both are still active. One skilled in the art would be able to determine such a time frame by determining the half life of each administered component.

In one embodiment, the complexes of the invention and another modality are administered within the same patient visit. In a specific preferred embodiment, the complexes of the invention are administered prior to the administration of another modality. In an alternate specific embodiment, the complexes of the invention are administered subsequent to the administration of another modality.

In certain embodiments, the complexes of the invention and one or more other modalities are cyclically administered to a subject. Cycling therapy involves the administration of the complexes of the invention for a period of time, followed by the administration of another modality for a period of time and repeating this sequential administration. Cycling therapy can reduce the development of resistance to one or more of the therapies, avoid or reduce the side effects of one of the therapies, and/or improve the efficacy of the treatment. In such embodiments, the invention contemplates the alternating administration of a complexes of the invention followed by the administration of another modality 4 to 6 days later, preferable 2 to 4 days, later, more preferably 1 to 2 days later, wherein such a cycle may be repeated as many times as desired. In certain embodiments, the complexes of the invention and one or more other modalities are alternately administered in a cycle of less than 3 weeks, once every two weeks, once every 10 days or once every week. In a specific embodiment, complexes of the invention is administered to a subj ect within a time frame of one hour to twenty four hours after the administration of another modality. The time frame can be extended further to a few days or more if a slow-or continuous-release type of modality delivery system is used.

### 5.9.3 Formulations and Use

Pharmaceutical compositions for use in accordance with the present invention may be formulated in conventional manner using one or more physiologically acceptable carriers or excipients.

Thus, the complexes and their physiologically acceptable salts and solvates may be formulated for administration by inhalation or insufflation (either through the mouth or the nose), oral, buccal, parenteral, intradermal, mucosal, subcutaneous, intravenous, rectal, or transdermal administration. Non-invasive methods of administration are also contemplated.

For oral administration, the pharmaceutical compositions may take the form of, for example, tablets or capsules prepared by conventional means with pharmaceutically acceptable excipients such as binding agents (*e.g.*, pregelatinised maize starch, polyvinylpyrrolidone or hydroxypropyl methylcellulose); fillers (*e.g.*, lactose, microcrystalline cellulose or calcium hydrogen phosphate); lubricants (*e.g*., magnesium stearate, talc or silica); disintegrants (*e.g*., potato starch or sodium starch glycolate); or wetting agents (*e.g*., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (*e.g.*, sorbitol syrup, cellulose derivatives or hydrogenated edible fats); emulsifying agents (*e.g.*, lecithin or acacia); non-aqueous vehicles (*e.g.*, almond oil, oily esters, ethyl alcohol or fractionated vegetable oils); and preservatives (*e.g.*, methyl or propyl-p-hydroxybenzoates or sorbic acid). The preparations may also contain buffer salts, flavoring, coloring and sweetening agents as appropriate.

Preparations for oral administration may be suitably formulated to give controlled release of the active complexes.

For buccal administration the compositions may take the form of tablets or lozenges formulated in conventional manner.

For administration by inhalation, the complexes for use according to the present invention are conveniently delivered in the form of an aerosol spray presentation from pressurized packs or a nebuliser, with the use of a suitable propellant, *e.g.*, dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of *e.g.,* gelatin for use in an inhaler or insufflator may be formulated containing a powder mix of the complexes and a suitable powder base such as lactose or starch.

The complexes may be formulated for parenteral administration by injection, *e.g.,* by bolus injection or continuous infusion. Formulations for injection may be presented in unit dosage form, *e.g.,* in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, *e.g.,* sterile pyrogen-free water, before use.

The complexes may also be formulated in rectal compositions such as suppositories or retention enemas, *e.*g., containing conventional suppository bases such as cocoa butter or other glycerides.

In addition to the formulations described previously, the complexes may also be formulated as a depot preparation. Such long acting formulations may be administered by implantation (for example subcutaneously or intramuscularly) or by intramuscular injection. Thus, for example, the complexes may be formulated with suitable polymeric or hydrophobic materials (for example as an emulsion in an acceptable oil) or ion exchange resins, or as sparingly soluble derivatives, for example, as a sparingly soluble salt.

The compositions may, if desired, be presented in a pack or dispenser device that may contain one or more unit dosage forms containing the active ingredient. The pack may for example comprise metal or plastic foil, such as a blister pack. The pack or dispenser device may be accompanied by instructions for administration.

Also encompassed is the use of adjuvants in combination with or in admixture with the complexes of the invention. Adjuvants contemplated include but are not limited to mineral salt adjuvants or mineral salt gel adjuvants, particulate adjuvants, microparticulate adjuvants, mucosal adjuvants, and immunostimulatory adjuvants, such as those described in Section 5.8. Adjuvants can be administered to a subject as a mixture with complexes of the invention, or used in combination with the complexes as described in Section 5.9.2.

Also contemplated is the use of adenosine Biphosphate (ADP) in combination with or in admixture with the complexes of the invention, preferably gp96 complexes.

### 5.9.4 Kits

The invention also provides kits for carrying out the therapeutic regimens of the invention. Such kits comprise in one or more containers therapeutically or prophylactically effective amounts of the molecule complexes of the invention in pharmaceutically acceptable form. The molecule complex in a vial of a kit of the invention may be in the form of a pharmaceutically acceptable solution, *e.g.*, in combination with sterile saline, dextrose solution, or buffered solution, or other pharmaceutically acceptable sterile fluid. Alternatively, the complex may be lyophilized or desiccated; in this instance, the kit optionally further comprises in a container a pharmaceutically acceptable solution (*e.g.*, saline, dextrose solution, etc.), preferably sterile, to reconstitute the complex to form a solution for injection purposes.

In another embodiment, a kit of the invention further comprises a needle or syringe, preferably packaged in sterile form, for injecting the complex, and/or a packaged alcohol pad. Instructions are optionally included for administration of molecule complexes of the invention by a clinician or by the patient.

In some embodiments, the present invention provides kits comprising a plurality of containers each comprising a pharmaceutical formulation or composition comprising a dose of molecular complexes of the invention sufficient for a single therapeutic or prophylactic administration. The invention also provides kits comprising a container comprising an immunologically and/or biologically active glycoprotein or a complex thereof, and a container comprising lectin. Optionally, instructions for formulating the oligomerized complexes according to the methods of the invention can be included in the kits.

In a specific embodiment, a kit comprises a first container containing a purified molecular complex; and a second container containing a different treatment modality in an amount that, when administered before, concurrently with, or after the administration of the molecular complex in the first container, is effective to improve overall treatment effectiveness over the effectiveness of the administration of each component alone, or is effective to decrease side effects of the treatment (e.g., as compared to side effects observed) when each component is used alone. In a preferred specific embodiment, the invention provides a kit comprising in a first container, a purified molecular complex of the invention comprising a population of noncovalent peptide complexes obtained from cancerous tissue of a mammal and oligomerized in the presence of ConA; in a second container, a composition comprising a purified cancer chemotherapeutic agent; and in a third container, a composition comprising a purified cytokine.

### 5.10. Monitoring of Effects During Treatment

The effect of treatment with the molecular complexes of the invention can be monitored by any methods known to one skilled in the art. For example, improvement or worsening of symptoms and/or laboratory results, imaging technologies, or worsening various biochemical assays, can all be used to monitor the treatment effect.

The effect of treatment with the molecule complexes of the invention on development and progression of neoplastic diseases can be monitored by any methods known to one skilled in the art, including but not limited to measuring: a) delayed hypersensitivity as an assessment of cellular immunity; b) activity of cytolytic T-lymphocytes *in vitro;* c) levels of tumor specific antigens, *e.g.*, carcinoembryonic (CEA) antigens; d) changes in the morphology of tumors using techniques such as a computed tomographic (CT) scan; e) changes in levels of putative biomarkers of risk for a particular cancer in subjects at high risk, and f) changes in the morphology of tumors using a sonogram.

### 5.10.1 Delayed Hypersensitivity Skin Test

Delayed hypersensitivity skin tests are of great value in the overall immunocompetence and cellular immunity to an antigen. Inability to react to a battery of common skin antigens is termed anergy (Sato, T., et al, 1995, Clin. Immunol. Pathol., 74:35-43).

Proper technique of skin testing requires that the antigens be stored sterile at 4 C, protected from light and reconstituted shorted before use. A 25- or 27-gauge needle ensures intradermal, rather than subcutaneous, administration of antigen. Twenty-four and 48 hours after intradermal administration of the antigen, the largest dimensions of both erythema and induration are measured with a ruler. Hypoactivity to any given antigen or group of antigens is confirmed by testing with higher concentrations of antigen or, in ambiguous circumstances, by a repeat test with an intermediate test.

### 5.10.2 In Vitro Activation of Cytotoxic T Cells

8x106 peripheral blood derived T lymphocytes isolated by the Ficoll-Hypaque centrifugation gradient technique, are restimulated with 4x104 mitomycin C treated tumor cells in 3ml RPMI medium containing 10% fetal calf serum. In some experiments, 33% secondary mixed lymphocyte culture supernatant or IL-2, is included in the culture medium as a source of T cell growth factors.

In order to measure the primary response of cytolytic T-lymphocytes after immunization, T cells are cultured without the stimulator tumor cells. In other experiments, T cells are restimulated with antigenically distinct cells. After six days, the cultures are tested for cytotoxicity in a 4 hour 51 Cr-release assay. The spontaneous 51 Cr-release of the targets should reach a level less than 20%. For the anti-MHC class I blocking activity, a tenfold concentrated supernatant of W6/32 hybridoma is added to the test at a final concentration of 12.5% (Heike M., et al., J. Immunotherapy, 15:165-174).

### 5.10.3 Levels of Tumor Specific Antigens

Although it may not be possible to detect unique tumor antigens on all tumors, many tumors display antigens that distinguish them from normal cells. The monoclonal antibody reagents have permitted the isolation and biochemical characterization of the antigens and have been invaluable diagnostically for distinction of transformed from nontransformed cells and for definition of the cell lineage of transformed cells. The best-characterized human tumor-associated antigens are the oncofetal antigens. These antigens are expressed during embryogenesis, but are absent or very difficult to detect in normal adult tissue. The prototype antigen is carcinoembryonic antigen (CEA), a glycoprotein found on fetal gut and human colon cancer cells, but not on normal adult colon cells. Since CEA is shed from colon carcinoma cells and found in the serum, it was originally thought that the presence of this antigen in the serum could be used to screen patients for colon cancer. However, patients with other tumors, such as pancreatic and breast cancer, also have elevated serum levels of CEA. Therefore, monitoring the fall and rise of CEA levels in cancer patients undergoing therapy has proven useful for predicting tumor progression and responses to treatment.

Several other oncofetal antigens have been useful for diagnosing and monitoring human tumors, *e.g.,* alpha-fetoprotein, an alpha-globulin normally secreted by fetal liver and yolk sac cells, is found in the serum of patients with liver and germinal cell tumors and can be used as a marker of disease status.

### 5.10.4 Computed Tomographic (CT) Scan

CT remains the choice of techniques for the accurate staging of cancers. CT has proved more sensitive and specific than any other imaging techniques for the detection of metastases.

### 5.10.5 Measurement of Putative Biomarkers

The levels of a putative biomarker for risk of a specific cancer are measured to monitor the effect of the molecular complex of the invention. For example, in subjects at enhanced risk for prostate cancer, serum prostate-specific antigen (PSA) is measured by the procedure described by Brawer, M.K., et. al., 1992, J. Urol., 147:841-845, and Catalona, W.J., et al., 1993, JAMA, 270:948-958; or in subjects at risk for colorectal cancer, CEA is measured as described above in Section 5.10.3; and in subjects at enhanced risk for breast cancer, 16- -hydroxylation of estradiol is measured by the procedure described by Schneider, J. et al., 1982, Proc. Natl. Acad. Sci. USA, 79:3047-3051.

### 5.10.6 Sonogram

A sonogram remains an alternative choice of technique for the accurate staging of cancers.

### 6. EXAMPLE 1: CONSISTENTLY ELEVATED LEVELS OF CON A IN HUMAN GP96-PEPTIDE COMPLEX LOTS

Tissue homogenates from four independent human renal tumor samples (A through D) were prepared and processed through Con A column chromatography. The Con A eluate was divided and half of the material set aside. The remaining sample was buffer exchanged into PBS (PD-10 column) and then both samples further purified over separate DEAE columns producing two homogenate-matched final products - one produced without buffer exchange (no Bx) between Con A and DEAE columns and one produced with a buffer exchange step (Bx) between the two columns. A sensitive ELISA to detect Con A was then used to determine the con A concentration in these separate gp96-peptide samples. Concanavalin A specific ELISA:

Materials: Concanavalin A was from Sigma, Catalog # C7275. Capture antibody: mouse anti Con A Cat# MAB 158 Maine Biotechnology, primary antibody: Rabbit anti Con A Cat# C7401 Sigma; detection antibody: Goat anti Rabbit IgG-HRP Cat# 111-035-144 Jackson ImmunoResearch. 0.1M NaHCO3 pH 9.6. Wash Buffer PBST PBS+0.05% Tween 20. Blocking Buffer: 2% Nonfat Dry milk in Wash Buffer(PBST). Methyl a-D-Mannopyranoside (a-MM), USB Cat# 19115. Sample Diluent: PBS plus 1% BSA and 10% MMP. TMB Microwell Substrate, Cat# 50-76-05 KPL. Stop Solution, Cat # 50-85-05 KPL

Methods: plates were coated with 2µg/mL mouse anti-Con A in 0.1M NaHCO3 pH 9.6 and incubated overnight at 4°C. The plate was washed (PBS-Tween) and subsequently blocked (1% BSA/PBS) at 37°C for 1hr and then washed. Samples and 100µl/well, incubated (37°C for 1 hr) and the plate washed. Rabbit anti-Con A in 1% BSA + 10% a-MM in was added and the plate incubated at 37°C for 1 hr and then washed. The detection antibody goat anti Rabbit IgG-horseradish peroxidase 1: 5000 in blocking buffer was added and the plate incubated at RT for 0.5 hr and then washed. TMB substrate was then added to each well, the plate incubated (RT, 10 min), stop solution added and the plate read plate at 450nm.

Results: gp96 purified from a common homogenate using a process including the buffer exchange step had higher levels of Con A than did the corresponding homogenate-matched gp96 sample produced with the omission of the buffer exchange step (Figure 1).

### 7. EXAMPLE 2: CON A IS PRESENT IN AN OLIGOMERIZED MOLECULAR COMPLEX

A common homogenate from chemically induced murine fibrosarcoma (Meth A) tissue was prepared and processed through Con A column chromatography. The con A eluate was divided and half of the material set aside. The remaining sample was buffer exchanged into PBS and then both samples purified over separate DEAE columns producing two homogenate-matched final products - one produced without buffer exchange (no Bx) and one produced with a buffer exchange step (Bx) between the Con A and DEAE columns. Both samples, along with a sample of free con A (5µg, 50µg/mL) were fractionated by SEC on a Superdex 200 column (Upper, middle, lower respectively). Collected fractions were analyzed for gp96 by SDS-PAGE (Fractions 1 through 8; inset) and the Con A content in the individual fractions evaluated by a direct ELISA against Con A (Fractions 1 through 14; overlay) (For direct ELISA against Con A, see Example 1). Little Con A was found in the no Bx-gp96 preparation while the gp96 produced with Bx was shown to have Con A in fractions 1 through 5. Free Con A eluted much later suggesting the Con A present in the Bx-gp96 sample was not free, but associated with a higher molecular weight species.

To verify gp96 was oligomerized with Con A, a common homogenate from Meth A-induced murine fibrosarcoma tissue was prepared and processed using a process that included Con A and DEAE column chromatography. The final purified gp96 preparation was divided in two. To one half of the material, exogenous con A was added to a final concentration of 50ug/mL; buffer alone was added to the other as a control, both samples incubated at 37°C for 2hr and then fractionated by SEC (Superdex 200). Individual fractions were analyzed by SDS-PAGE, and by gp96- and con A-specific ELISA. Analytical data for material produced without the Buffer exchange step is shown in the left panel; that to which con A was added to the right. In the left panel the peak of gp96 is in fraction 5 and con A levels as detected by specific ELISA are low. In the right panel (con A added) two peaks of gp96 are evident as shown by SDS-PAGE (inset; peak fractions 3 (arrow) and 5) and gp96 ELISA (fractions 3 and 5) as well as a distinct peak of Con A centered on fraction 3. Con A mediated a shift in the elution position of gp96.

### 8. EXAMPLE 3: OLIGOMERIZATION CORRELATES WITH IN VITRO AND IN VIVO POTENCY

### 8.1. Con A Content Correlates with In Vitro Potency for Human gp96 Samples

The gp96 samples from four independent human renal tumor samples (A through D) were prepared as described above (See. Fig. 1) generating four paired samples differing only in the inclusion or omission of a buffer exchange step between Con A and DEAE columns. All eight samples were assayed for con A content (Panel A; also see Fig. 1) along with *in vitro* antigen representation using the CD71 system (Panel B). In each case, material prepared by the process including the buffer exchange step (and containing increased levels of Con A) had higher *in vitro* representation activity than a sample generated from the matching tumor homogenate and prepared by a step in which the buffer exchange step was omitted.

### CD71 in vitro representation assay:

Materials: the antigen presenting cell line, RAW264.7 (ATCC #TIB-71), was used in these experiments. It is an Abelson murine leukemia virus transformed macrophage cell line, which originated from the BALB/c strain (H-2^{d}). A T cell hybridoma was generated by fusion of BALB/c T cells specific for human CD71 9-mer with BWαβ⁻ thymoma cells. T cells were fused with BWαβ⁻ cells by PEG and were selected in HAT medium. Resulting T-T hybridoma cells were then screened for CD71 9-mer specificity by measuring IL-2 production by proliferation of HT-2 cells after antigen stimulation. Medium used was RPMI 1640 (Gibco-BRL). Human gp96 was purified from human renal tumors using both the buffer exchange and non-buffer exchange processes and also a scFv-column method as described (Arnold-Schild *et al., supra*). Murine gp96-CT26, purified from CT26 tumor using the same purification protocol as test sample and murine CD71 9mer peptide (TYEALTQKV) were used as negative antigen controls. Human CD71 9mer (TYKELIERI) was used as a positive antigen control.

Preparation of Human Tumor derived gp96: human derived gp96 was purified from human renal tumors. Briefly, tumors were homogenized and clarified by centrifugation. The cell-free supernatant was subjected to a 50% ammonium sulfate precipitation. The resulting supernatant was further purified using ConA and DEAE chromatography. Protein was filter sterilized (0.22 µm), aliquoted, and stored at -80 ± 20°C until use.

Re-presentation assay: in a 96-well flat bottom plate, human CD71-specific T cell hyridomas (5x10⁴) were co-cultured with RAW264.7 cells (5x10⁴). Desired concentrations of human gp96, starting at 150 µg/ml and titrating down, were added in triplicate. Appropriate positive and negative controls were added, and the volume of medium was adjusted to reach a final volume of 200µl. In addition to the test plate containing both T cell hybridomas and APCs, plates containing T cell hybridomas only, or APCs only, were added as controls. Plates were agitated slightly by tapping before being placed at 37°C in a 5% CO₂ incubator for 20 hours. Following incubation, cells were pelleted at 1000 RPM for 5 minutes at 4°C. Supernatants were transferred to 96-well round bottom plate and tested for IL-2 production by ELISA (R&D).

### 8.2. Con A Content Correlates with In Vivo Potency in the Murine CT 26 System

The gp96 samples from two independent murine CT26 tumor samples (Preps A and B) were prepared as described above for human tumor derived samples (See. Fig. 1) All four samples were assayed for con A content (Panel A) and *in vitro* antigen representation using the CT26 system (Panel B). In each case, material prepared by the process including the buffer exchange step (and having an increased amount of con A) had higher *in vitro* representation activity than a sample generated from the matching tumor homogenate and prepared by a step in which the buffer exchange step was omitted.

### CT26 in vitro antigen representation assay:

Materials: the antigen presenting cell line, RAW264.7 (ATCC #TIB-71), was used in these experiments. It is an Abelson murine leukemia virus transformed macrophage cell line, which originated from the BALB/c strain (H-2d). Cytotoxic T Lymphocyte (CTLs): T cells specific for the CT26 tumor peptide were obtained from the University of Connecticut Medical Center. These T cells are specific for the AH1 epitope, amino acid sequence SPSYVYHQF. These CTLs are re-stimulated on a weekly basis with the AH1 9 mer peptide plus irradiated BALB/c splenocytes. AIM V (Gibco-BRL) tissue culture medium was used. This is a serum free medium that does not contain proteases. Proteases are undesirable in that they may digest elongated peptides to a smaller size able to surface load on MHC molecule inducing a CTL response independent of antigen representation. CT26 derived gp96 was purified from mouse tumors using both the buffer exchange and non-buffer exchange processes. AH1 19mer (RVTYHSPSYVYHQFERRAK) alone was added as a negative control, as well as gp96 derived from normal mouse organs. AH1 9mer (SPSYVYHQF), which can surface load and prime for CTL recognition was used as a positive control. An additional positive control included mouse derived gp96 complexed with AH1 19 mer peptide.

Methods:

Preparation of CT26 Derived gp96: CT26 derived gp96 was purified from solid tumors. Mouse tumor was homogenized and clarified by centrifugation. The cell-free supernatant was subjected to a 50% ammonium sulfate precipitation. The resulting supernatant was further purified using ConA and DEAE chromatography. Protein was filter sterilized (0.22 µm), aliquoted and stored at -80 ± 20°C until use.

Preparation of gp96-AH1 19mer complexes (complex positive control samples): AH1 19mer peptide, dissolved in H₂O, was added to gp96 at a 50:1 molar ratio. Samples were briefly mixed in a 15ml conical tube in a volume of approximately 1 to 2ml, depending on how much protein was being complexed, and placed at 37°C for 0.5 hours. After incubation, samples were washed 4X with 5ml PBS using a 30K MWCO Centricon spin filter unit (Millipore) and analyzed for protein concentration by the Bradford Assay.

Preparation of AH1-9mer (assay positive control samples): AH1 9mer can be loaded directly on MHC I molecules, therefore was used as a positive control to directly stimulate T cells without processing by APCs.

Negative control samples included uncomplexed gp96 or naked 19mer peptide dissolved in PBS at the same molar concentration.

Re-Presentation Assay: AH1 peptide-specific T cells (8 days post-stimulation) were washed 3X to remove APCs, and re-suspended in AIM V media at 2x105 cells/ml. RAW 264.7 cells were used as APCs, and washed once in DMEM plus 10% FCS, before re-suspending at 2x105 cells/ml in AIM V. In a 96-well round bottom plate CT26 derived gp96 samples were added in quadruplicate and a two fold serial dilution was done (200µg/ml - 6.25ug/ml). Appropriate positive and negative controls were added, as well as the amount of AIM V needed to reach a final volume of 250µl. In each well 1x104 T cells were co-cultured with an equal number of APCs. Plates containing only T cells were used as controls. The plates were incubated at 37°C and 5% CO₂ for 18 hours.

After incubation, cells were pelleted by centrifugation at 1000rpm for 5 minutes at 4°C. Supernatants were transferred to 96-well flat bottom plates for ELISA and storage at -20°C. IFN-γ levels were measured by ELISA (R&D Systems).

### 8.3. Con A Content Correlates with Both in vitro in the Meth A

### Representation Assay and In Vivo Potency in the Murine Meth A Tumor Protection Model

Two separate Meth A gp96 preparations were prepared from a common tumor homogenate (described above) generating a paired sample differing only in the inclusion or omission of a buffer exchange step between Con A and DEAE columns. These samples were assayed by Con A ELISA for con A content (Panel A), *in vitro* activity in the Meth A representation assay (Panel B) and *in vivo* in the meth A tumor protection assay at a dose of 10µg (Panel C). Meth A gp96 prepared by a process including a buffer exchange step between Con A and DEAE columns had increased Con A content, higher *in vitro* antigen representation and higher *in vivo* tumor protection activity over that prepared by a process in which the buffer exchange step was omitted.

### Meth A in vitro antigen representation:

Materials: irradiated BALB/c splenocytes were used as the Antigen Presenting Cells (APCs). The Meth A-specific CD4+ T cell clone, 24D3, was used. They are restricted by the MHCII molecule I-Ed, and are specific for the antigenic peptide contained within the sequence EYELRKHNFSDTG. The medium used for this assay was RPMI 1640 (Gibco-BRL). The gp96 was purified from mouse Meth A tumors using both the buffer exchange and non-buffer exchange processes. The wild-type form of the L11 peptide (EYELRKNNFSDTG) was used as a negative control, and the mutated form of the L11 peptide (EYELRKHNFSDTG) was used as the positive control.

Methods:

Preparation of Meth A Derived gp96: Meth A derived gp96 was purified from solid tumors. Mouse tumor was homogenized and clarified by centrifugation. The cell-free supernatant was subjected to a 50% ammonium sulfate precipitation. The resulting supernatant was further purified using Con A and DEAE chromatography. Protein was filter sterilized (0.22µm), aliquoted and stored at -80 ± 20°C until use.

Re-Presentation Assay: 2x10⁴ of Meth A specific CD4+T cell clones (24D3) were incubated with 5x10⁵ of irradiated BALB/c splenic APCs in the presence of various concentration of gp96 derived from Meth A or other sources (100, 50, 25, 12.5, 6.25 µg/ml final concentration) for 48-72hrs in the 96 well flat bottom plate in 200 µl of final volume. Wild type ribosomal protein L1 peptide and mutated ribosomal protein L11 peptide were added in replicate wells and used as a negative control or positive control respectively. After 48-72 hours of incubation at 37°C, 5% CO₂, 100 µl of supernatant was taken and IL-5 production was measured by ELISA.

### In Vivo Meth A Tumor Inhibition Assay:

*In vivo* Meth A growth inhibition assay: BALB/c mice (n=30 / group) were immunized on day 0 and day 7 with 10 or 50 µg of gp96 intradermally in the flank. On study day 14, mice were challenged with 1X10⁵ Meth A cells intradermally in a total volume of 100 µl. Growing tumors were monitored during the subsequent four weeks. All animals were euthanized on study day 41 after a final tumor measurement. Data was reported as percentage of animals that are tumor-free on study day 41. Control groups included un-immunized (diluent - negative control) and immunized with irradiated Meth A cells (positive control).

### 9. EXAMPLE 4: EXOGENOUS CON A INCREASES GP96 ACTIVITY IN THE CD71 IN VITRO REPRESENTATION ASSAY

Human liver tissue was homogenized and centrifuged producing an 11K supernatant that was divided into 3 identical samples and processed by different methods.

Two samples were processed through Con A column chromatography (see Section 5.13), the Con A eluate was divided and half of the material set aside. The remaining sample was buffer exchanged (see Section 5.13) into PBS and then both samples purified over separate DEAE columns producing two homogenate-matched final products one produced without buffer exchange (NO Bx - sample A) and one produced with a buffer exchange step (Bx - sample B) between the Con A and DEAE columns (Figure 7).

The remaining 11K supernatant was used to purify gp96 by using gp96-specific scFv column as described in Arnold-Schild et al., Cancer Research, 2000, 60(15):4175-4178 (referred as "Arnold-Schild" herein after) . The purification was done as follows: five mg of scFv anti-gp96 were coupled to 0.5 mg of CNBr-activated Sepharose (Pharmacia) (For production of scFv anti-gp96, see Arnold-Schild, or it can be made by any method well-known in the art.). The 11K supernatants were applied to the scFv anti-gp96 column. After extensive washing with PBS, gp96 was eluted with PBS, 1.3 M NaCl, 10 mM sodium acetate (pH 7.2).

All samples were analyzed for con A concentration by a con A specific ELISA (exogenous con A (7.5ng con A/µg total protein) was added to both samples A and D to levels equivalent to that in sample B) and for *in vitro* antigen representation in the CD71 system ata protein concentration of 75µg/mL. For matched sample pairs, material produced by the process including the buffer exchange step (sample #1; con A content 7.5ng/ug total protein) was more active *in vitro* than material produced by a process in which the buffer exchange step was omitted (sample A; con A content 0.43ng/ug total protein) (Figure 7). Material produced by a single-step method that did not utilize a con A column purification step (scFv gp96; 0 ng/ug) had low *in vitro* activity similar to sample A (Figure 7). Addition of exogenous con A to samples A and C to levels equivalent to that in sample B (7.5ng Con A/µg total protein), increased the specific *in vitro* antigen representation activity to a level similar to that present in sample B (Figure 7). This level of Con A had no effect on T cells alone.

### 10. EXAMPLE 5: THE OLIGOMERIC SPECIES IS METHYL ALPHA-D-MANNOPYRANOSIDE (ALPHA-MM) SENSITIVE

A meth A gp96 sample was purified by the standard purification process including Con A and DEAE chromatography and the protein analyzed by analytical SEC using a superose 6 column (Pharmacia) which showed the protein preparation contained primarily dimeric gp96 (gp96 T=0). Con A was added (50ug/mL final) to an aliquot of this gp96 sample (conc. 500ug/ml), the sample incubated at RT and hourly samples were taken (T=1 through T=5) and analyzed by SEC. A sample comprising con A alone was also run. The addition of con A mediated a shift in the elution position of the gp96 dimer peak which changed only slightly following the first time point. gp96 alone did not change over this time period (gp96 T=5). Following the final time point, two separate aliquots of the final 5hr sample were taken and either an equal volume of PBS or PBS containing 10% α-MM added. Each sample was then re-analyzed by SEC. No change was evident in the sample to which PBS was added (not shown). The addition of α-MM dissociated the high molecular weight complex (gp96 +con A T=5 +α-MM) resulting in the SEC profile resembling that of the original gp96 sample (gp96 T=0 or T=5).

### 11. EXAMPLE 6: LOW CON A:GP96 STOICHIOMETRIES MEDIATE AN SEC SENSITIVE SHIFT IN THE GP96 ELUTION POSITION

Human renal tumor gp96 was purified by the standard purification process including Con A and DEAE chromatography and the protein analyzed by analytical SEC using a superose 6 column (Pharmacia). Con A was added to final concentration of 0.005 - 50µg/mL to gp96 (180ug/mL), the sample incubated at room temperature for 1hour and analyzed by SEC. Stiochiometries of about 1Con A : 10 gp96 are able to generate an SEC sensitive shift in gp96 elution position.

### 12. EXAMPLE 7: ADDITTION OF METHYL ALPHA-D-MANNOPYRANOSIDE CAUSESA CONCENTRATION DEPENDENT DECREASE IN CT26 IN VITRO ANTIGEN REPRESENTATION ACTIVITY

A sample of CT26-derived gp96 (prepared by the Bx process) along with a positive control 9mer peptide (SPSYVYHQF) were incubated for 30 minutes in the presence of 50, 100 or 400 mM α-MM prior to being diluted (1 in 5) into a microtiter plate well containing RAW264.7 APC cells and AH1 specific T-cells. The samples were incubated overnight and the resulting supernatants analyzed by an INF-γ specific ELISA. α-MM caused a dose-dependent decrease in CT26 antigen representation and was without effect on T-cell recognition of the positive control 9mer peptide. This level of α-MM does not affect the viabilities of APC or T cells.

### 13. EXAMPLE 8: CON A INCREASES TUMOR REJECTION ACTIVITY OF HSPPC-96

### 13.1. Con A Added During the Purification of gp96 Increases the Activity of the Purified Final Product

Meth A derived gp96 was prepared with different levels of Con A in the final product by addition of Con A during the purification process. All samples were assayed by Con A ELISA for Con A content (the measured Con A concentration in ng Con A/µg for each sample is indicated in the figure) and *in vivo* tumor rejection. Figure 11 shows the results of the Meth A *in vivo* tumor rejection assay. The in-process addition of Con A caused a titratable increase in the tumor rejection activity of HSPPC-96.

Methods:

Preparation of Meth A derived gp96 and in process addition of Con A: A sample of Meth A tumor was homogenized (30mM sodium phosphate buffer pH 7.2 containing 2mM MgCl2 and 2mM AEBSF), clarified by centrifugation, solid ammonium sulfate added to 50%, the sample centrifuged again and the supernatant applied directly to a Con A column. At this stage, the Con A eluate was buffer exchanged into phosphate buffered saline ("PBS") and divided into 6 identical samples. One sample was processed immediately by loading onto a diethylaminoethyl ("DEAE") column, washing this sample with 10mM sodium phosphate, 260mM NaCl and eluting with 10mM sodium phosphate, 700mM NaCl (Standard procedure). To the remaining five, exogenous Con A was added (Levels one through five of 3ug/ml, 10ug/ml, 30ug/ml, 100ug/ml and 300ug/ml respectively). All samples were then purified over DEAE column as described above. All DEAE eluates were subsequently buffer exchange into 9% sucrose-potassium phosphate buffer pH 7.4.

In vivo Meth A growth inhibition assay: BALB/c mice (n=10/group) were immunized on day 0 and day 7 with 10 µg of gp96 intradermally in the flank. On study day 14, mice were challenged with 1X105 Meth A cells intradermally in a total volume of 100 µl. Growing tumors were monitored during the subsequent four weeks. All animals were euthanized on study day 41 after a final tumor measurement. Data was reported as percentage of animals that are tumor-free on study day 41. Control groups included un-immunized (diluent - negative control) and immunized with irradiated Meth A cells (positive control).

### 13.2. Con A Added During the Purification of gp96 or Subsequent to the Purification of gp96 Increases the Activity of the Purified Final Product

Meth A derived gp96 was prepared with different levels of Con A in the final product by either the addition of Con A during the purification process (at levels of 30µg/ml and 300µg/ml) or following the purification (at a level of 30µg/ml). All samples were assayed by Con A ELISA for Con A content (the measured Con A concentration in ng Con A/µg for each sample is indicated in the figure) and *in vivo* tumor rejection. Figure 12(A) shows the results of the Meth A in vivo tumor rejection assay for in process Con A addition, and Figure 12(B) shows the results for Con A addition to the final product. Addition of Con A, either in process or following purification of gp96 resulted in an increase in the tumor rejection activity of HSPPC-96.

Methods:

Preparation of Meth A derived gp96 and addition of Con A: A sample of Meth A tumor was homogenized (30mM sodium phosphate buffer pH 7.2 containing 2mM MgCl2 and 2mM AEBSF), clarified by centrifugation, solid ammonium sulfate added to 50%, the sample centrifuged again and the supernatant applied directly to a Con A column. At this stage, the Con A eluate was buffer exchanged into PBS and divided into 3 samples. One sample was processed immediately through the DEAE column to obtain gp96 (Standard procedure). To the remaining two, exogenous Con A was added (30 and 300ug/mL respectively) and the samples were then purified over DEAE column. All DEAE eluted samples were subject to final buffer exchange into 9% sucrose-potassium phosphate buffer pH 7.4. Con A was added (30ug/mL) to an aliquot of the gp96 purified by the standard procedure. Samples were analyzed for Con A content and in vivo tumor rejection. Con A caused an increase in the tumor rejection activity of gp96 when added either in process (Figure 12 (A)) or to the final purified gp96 protein (Figure 12 (B)).

In vivo Meth A growth inhibition assay: BALB/c mice (n=10/group) were immunized on day 0 and day 7 with 10 µg of gp96 intradermally in the flank. On study day 14, mice were challenged with 1X105 Meth A cells intradermally in a total volume of 100 µl. Growing tumors were monitored during the subsequent four weeks. All animals were euthanized on study day 41 after a final tumor measurement. Data was reported as percentage of animals that are tumor-free on study day 41. Control groups included un-immunized (diluent - negative control) and immunized with irradiated Meth A cells (positive control).

In another experiment, Meth A derived gp96 was prepared using an anti-gp96 scFv immunoaffinity column. The purified gp96 was buffer exchanged into PBS and divided in to several aliquots. To one aliquot, Con A was added. Samples were analyzed by Con A ELISA (the measured Con A concentration in ng Con A/µg for each sample is indicated in the figure) and for in vivo tumor rejection. The results are shown in Figure 13. In each case, the addition of Con A increased the tumor rejection activity of gp96.

Method:

Preparation of Meth A derived gp96 and addition of Con A: A sample of Meth A tumor was homogenized (30mM sodium phosphate buffer pH 7.2 containing 2mM MgCl₂ and 2mM AEBSF), clarified by centrifugation and filtered (0.45µM). The resulting clarified homogenate was and applied to a 1mL anti-gp96 scFv immunoaffinitiy column. The column was washed with 10 column volumes of PBS and gp96 subsequently eluted with 5 column volumes of PBS containing 1.3M NaCl. The column eluate was buffer exchanged into PBS.

In vivo Meth A growth inhibition assay: BALB/c mice (n=10/group) were immunized on day 0 and day 7 with 0.3 or 3 µg of gp96 alone or in combination with Con A intradermally in the flank. On study day 14, mice were challenged with 1X105 Meth A cells intradermally in a total volume of 100 µl. Growing tumors were monitored during the subsequent four weeks. All animals were euthanized on study day 41 after a final tumor measurement. Data was reported as percentage of animals that are tumor-free on study day 41. Control groups included un-immunized (diluent - negative control) and immunized with irradiated Meth A cells (positive control).

### 14. EXAMPLE 9: IMMUNOTHERAPY OF HSV-2 INFECTION

Herpes Simples Virus type 2 ("HSV-2") is grown and viral particles isolated by any number of methods known in the art (see e.g., Principles of Virology, Molecular Biology, Pathgenesis, and Control, Flint et al., ed., ASM Press, (2000)). Viral particles are extracted by one of several common methods and the resulting sample centrifuged and filtered to obtain a viral protein-enriched fraction. The initial pool of solublized viral proteins can be quantified and a stoichiometric excess of Con A (or other lectin) added. Alternatively, the soluble protein extract can also be further enriched for virus-specific glycoprotein by chromatography on a Concacavalin A (or other immobilized lectin) column. Glycoproteins are specifically eluted from the column using a lectin-specific inhibitor (such as methyl alpha-manopyranoside for Con A). The inhibitor can be removed using a number of buffer exchange methods. The enriched glycoprotein sample can then be quantified and a stoichiometric excess of Con A added. Alternatively, the enriched glycoprotein sample can be further processed using enzymatic or chemical methods to generate smaller peptide-fragments of the glycoproteins. These smaller fragments can be mixed directly with a stoichiometric excess of Con A (or other lectin), or further purified by chromatography and elution from a Con A column (or other immobilized lectin column). The enriched glycopeptide fraction can be quantified and a stoichiometric excess of Con A (or other lectin) added. Samples, and appropriate controls are evaluated for biological activity using either prophylactic (murine HSV-2) or therapeutic (guinea-pig HSV-2) models.

Similar methodologies can be performed to generate test material for evaluation in cancer immunotherapy.

### Equivalence :

Many modifications and variations of this invention can be made without departing from its spirit and scope, as will be apparent to those skilled in the art. The specific embodiments described herein are offered by way of example only.

## Claims

1. One or more noncovalent complexes, each complex comprising a heat shock protein, an antigenic molecule, and a lectin, wherein said heat shock protein is glycosylated, and wherein the amount of lectin present in said complexes relative to the amount of heat shock protein is greater than or equal to 50 nanograms lectin per microgram of heat shock protein or less than or equal to 5 nanogram lectin per microgram of heat shock protein.

2. The complexes of claim 1, wherein the lectin present in said complexes relative to the amount of heat shock protein or glycoprotein is 50 to 1000 nanograms lectin per microgram of heat shock protein or glycoprotein.

3. The complexes of claim 1, wherein the lectin present in said complexes relative to the amount of heat shock protein is 100 to 500 nanograms lectin per microgram of heat shock protein.

4. The complexes of claim 1, wherein the lectin present in said complexes relative to the amount of heat shock protein or glycoprotein is 0.1 to 1 nanograms lectin per microgram of heat shock protein or glycoprotein.

5. The complexes of claim 1, wherein the lectin present in said complexes relative to the amount of heat shock protein is 0.5 to 1 nanograms lectin per microgram of heat shock protein.

6. The complexes of claim 1, wherein the molar ratio between the heat shock protein and lectin is 3:1, 2:1, or 1:1.

7. The complexes of any of claims 1 to 6, wherein said lectin is in the form of a dimer or an oligomer.

8. The complexes of any of claims 1 to 7, wherein said lectin is a mannose binding lectin.

9. The complexes of claim 8, wherein said mannose-binding lectin is Concanavalin A (Con A).

10. The complexes of any one of claims 1-9, wherein said heat shock protein is selected from the group consisting of gp96, Calreticulin, and GRP170.

11. The complexes of any one of claims 1-9, wherein said heat shock protein is gp96.

12. The complexes of any one of claims 1-11, wherein said complexes are purified.

13. The complexes of any one of claims 1-12, wherein said antigenic molecule is an antigenic molecule that displays one or more antigenic determinants against which an immune response is desired in a subject.

14. The complexes of any one of claims 1-13, wherein a complex of said heat shock protein and said antigenic molecule is isolated from cancerous tissue.

15. The complexes of any one of claims 1-13, wherein said antigenic molecule is a tumor specific antigen or a tumor-associated antigen.

16. The complexes of any one of claims 1-13, wherein said antigenic molecule displays the antigenicity of an antigen of an infectious agent.

17. A method of making the population of one or more noncovalent complexes according to any one of claims 1-16, said method comprising the steps of:
a) binding said lectin to said heat shock protein; and
b) complexing said heat shock protein to said antigenic molecule.

18. A method of making the population of one or more noncovalent complexes according to any one of claims 1-16, said method comprising binding a lectin to one or more complexes, each complex comprising a glycosylated heat shock protein and an antigenic molecule, wherein said lectin is not bound to a solid phase.

19. The method of claim 18, further comprising isolating said complexes of heat shock protein and antigenic molecule by lectin-based affinity chromatography prior to binding said complexes to lectins.

20. The method of claim 18, further comprising isolating said complexes of heat shock protein and antigenic molecule by non-lectin based chromatography prior to binding said complexes to said lectin.

21. The method of claim 20, wherein said non-lectin based chromatography is antibody-based affinity chromatography.

22. The method of claim 18, wherein the lectin is added during the process of purifying the one or more noncovalent complexes.

23. The method of claim 19 , wherein the lectin is added during the process of purifying the one or more noncovalent complexes by using a gp96-specific scFv column, wherein the heat shock protein in the one or more noncovalent complexes is gp96.

24. A pharmaceutical composition comprising a pharmaceutically acceptable carrier, and one or more noncovalent complexes according to any one of claims 1-13.

25. The pharmaceutical composition of claim 24 , for use in a method for the treatment or prevention of a cancer or an infectious disease in a subject.

26. The pharmaceutical composition of claim 24, for use as a medicament.

27. The pharmaceutical composition of claim 26, wherein a complex of said heat shock protein and said antigenic molecule is isolated from cancerous tissue.

28. The pharmaceutical composition of claim 26, wherein said antigenic molecule is a tumor specific antigen or a tumor-associated antigen.

29. The pharmaceutical composition of claim 26, wherein said antigenic molecule
displays the antigenicity of an antigen of an infectious agent.

30. The pharmaceutical composition of claim 27 or 28, for use in a method for the treatment of a cancer in a subject having cancer.

31. The pharmaceutical composition of claim 29, for use in a method for the treatment of an infectious disease in a subject having an infectious disease.

32. The pharmaceutical composition of any one of claims 25, 30, or 31, wherein the subject is a mammal.

33. The pharmaceutical composition of claim 32, wherein the mammal is a human.

34. Use of the one or more complexes of claim 14 or 15, for the manufacture of a medicament for use in the treatment of a cancer in a subject having cancer.

35. Use of the one or more complexes of claim 16, for the manufacture of a medicament for use in the treatment of an infectious disease in a subject having an infectious disease.

36. A composition comprising a population of noncovalent complexes, wherein said noncovalent complexes are according to any one of claims 1-13.

37. The composition of claim 36 for use as a medicament.

38. The composition of claim 37, wherein a complex of said heat shock protein and said antigenic molecule is isolated from cancerous tissue.

39. The composition of claim 37, wherein said antigenic molecule is a tumor specific antigen or a tumor-associated antigen.

40. The pharmaceutical composition of claim 37, wherein said antigenic molecule displays that antigenicity of an antigen of an infectious agent.

41. The composition of claim 38 or 39, for use in a method for the treatment of a type of cancer in
a subject having cancer.

42. The composition of claim 40, for use in a method for the treatment of an infectious disease in a subject having an infectious disease.

43. The composition of claim 36, for use in a method for the treatment or prevention of cancer or an infectious disease.

44. Use of the composition of claim 38 or 39 , for the manufacture of a medicament for use in the treatment of a cancer in a subject having cancer.

45. Use of the composition of claim 40 for the manufacture of a medicament for use in the treatment of an infectious disease in a subject having an infectious disease.

46. Use of the composition of claim 36 for the manufacture of a medicament for treatment or prevention of cancer or an infectious disease.

47. A kit comprising:
a) a first container containing a composition comprising a population of noncovalent complexes according to any one of claims 1-13,
b) a second container containing a purified lectin.

48. The kit of claim 47, wherein the antigenic molecule displays the antigenicity of an antigen of a cancer or of an antigen of an agent of an infectious disease.

49. The kit of claim 47, wherein the lectin is a mannose-binding lectin.

50. The kit of claim 47, wherein the mannose-binding lectin is Concanavalin A (Con A).

51. The kit of any one of claims 47-50, wherein the heat shock protein is selected from the group consisting of gp96, Calreticulin, and GRP170.

52. The kit of any one of claims 47-50, wherein the heat shock protein is gp96.

## Patentansprüche

1. Ein oder mehrere nichtkovalente Komplexe, wobei jeder Komplex ein Hitzeschockprotein, ein antigenes Molekül und ein Lektin umfasst, wobei das Hitzeschockprotein glycosyliert ist und wobei die Menge des in den Komplexen enthaltenen Lektins bezogen auf die Menge des Hitzeschockproteins größer als oder gleich 50 Nanogramm Lektin pro Mikrogramm des Hitzeschockproteins oder weniger als oder gleich 5 Nanogramm Lektin pro Mikrogramm des Hitzeschockproteins ist.

2. Komplexe nach Anspruch 1, wobei das in den Komplexen enthaltene Lektin bezogen auf die Menge des Hitzeschockproteins oder Glycoproteins 50 bis 1000 Nanogramm Lektin pro Mikrogramm des Hitzeschockproteins oder Glycoproteins beträgt.

3. Komplexe nach Anspruch 1, wobei das in den Komplexen enthaltene Lektin bezogen auf die Menge des Hitzeschockproteins 100 bis 500 Nanogramm Lektin pro Mikrogramm des Hitzeschockproteins beträgt.

4. Komplexe nach Anspruch 1, wobei das in den Komplexen enthaltene Lektin bezogen auf die Menge des Hitzeschockproteins oder Glycoproteins 0,1 bis 1 Nanogramm Lektin pro Mikrogramm des Hitzeschockproteins oder Glycoproteins beträgt.

5. Komplexe nach Anspruch 1, wobei das in den Komplexen enthaltene Lektin bezogen auf die Menge des Hitzeschockproteins 0,5 bis 1 Nanogramm Lektin pro Mikrogramm des Hitzeschockproteins beträgt.

6. Komplexe nach Anspruch 1, wobei das Molverhältnis zwischen dem Hitzeschockprotein und Lektin 3:1, 2:1 oder 1:1 beträgt.

7. Komplexe nach einem der Ansprüche 1 bis 6, wobei das Lektin in der Form eines Dimers oder eines Oligomers vorliegt.

8. Komplexe nach einem der Ansprüche 1 bis 7, wobei das Lektin ein Mannose-bindendes Lektin ist.

9. Komplexe nach Anspruch 8, wobei das Mannose-bindende Lektin Concanavalin A (Con A) ist.

10. Komplexe nach einem der Ansprüche 1 bis 9, wobei das Hitzeschockprotein ausgewählt ist aus der Gruppe, bestehend aus gp96, Calreticulin und GRP170.

11. Komplexe nach einem der Ansprüche 1 bis 9, wobei das Hitzeschockprotein gp96 ist.

12. Komplexe nach einem der Ansprüche 1 bis 11, wobei die Komplexe gereinigt sind.

13. Komplexe nach einem der Ansprüche 1 bis 12, wobei das antigene Molekül ein antigenes Molekül ist, das eine oder mehrere antigenen Determinanten, gegen die eine Immunantwort in einem Subjekt gewünscht ist, zeigt.

14. Komplexe nach einem der Ansprüche 1 bis 13, wobei ein Komplex des Hitzeschockproteins und des antigenen Moleküls aus Krebsgewebe isoliert ist.

15. Komplexe nach einem der Ansprüche 1 bis 13, wobei das antigene Molekül ein Tumor-spezifisches Antigen oder ein Tumor-assoziiertes Antigen ist.

16. Komplexe nach einem der Ansprüche 1 bis 13, wobei das antigene Molekül die Antigenität eines Antigens eines infektiösen Mittels zeigt.

17. Verfahren zur Herstellung eines Bestandes von einem oder mehreren nichtkovalenten Komplexen gemäß einem der Ansprüche 1 bis 16, wobei das Verfahren die folgenden Stufen umfasst:
a) das Binden des Lektins an das Hitzeschockprotein; und
b) das Komplexieren des Hitzeschockproteins an das antigene Molekül.

18. Verfahren zur Herstellung eines Bestandes von einem oder mehreren nichtkovalenten Komplexen gemäß einem der Ansprüche 1 bis 16, wobei das Verfahren das Binden eines Lektins an einen oder mehrere Komplexe umfasst, wobei jeder Komplex ein glycosyliertes Hitzeschockprotein und ein antigenes Molekül umfasst, wobei das Lektin nicht an eine feste Phase gebunden ist.

19. Verfahren nach Anspruch 18, das ferner das Isolieren der Komplexe des Hitzeschockproteins und des antigenen Moleküls durch Lektin-basierte Affinitätschromatographie vor dem Binden der Komplexe an die Lektine umfasst.

20. Verfahren nach Anspruch 18, das ferner das Isolieren der Komplexe des Hitzeschockproteins und des antigenen Moleküls durch nicht-Lektin-basierte Chromatographie vor dem Binden der Komplexe an das Lektin umfasst.

21. Verfahren nach Anspruch 20, wobei die nicht-Lektin-basierte Chromatographie eine Antikörper-basierte Affinitätschromatographie ist.

22. Verfahren nach Anspruch 18, wobei das Lektin während des Vorgangs der Aufreinigung der einen oder mehreren nichtkovalenten Komplexe hinzugegeben wird.

23. Verfahren nach Anspruch 19, wobei das Lektin während des Vorgangs der Aufreinigung des einen oder der mehreren nichtkovalenten Komplexe unter Verwendung einer gp96-spezifischen scFv-Säule hinzugegeben wird, wobei das Hitzeschockprotein in dem einen oder mehreren nichtkovalenten Komplexen gp96 ist.

24. Pharmazeutische Zusammensetzung, die einen pharmazeutisch akzeptablen Träger und einen oder mehrere nichtkovalente Komplexe gemäß einem der Ansprüche 1 bis 13 umfasst.

25. Pharmazeutische Zusammensetzung nach Anspruch 24 zur Verwendung bei einem Verfahren zur Behandlung oder Vorbeugung von Krebs oder einer infektiösen Erkrankung in einem Subjekt.

26. Pharmazeutische Zusammensetzung nach Anspruch 24 zur Verwendung als ein Medikament.

27. Pharmazeutische Zusammensetzung nach Anspruch 26, wobei ein Komplex des Hitzeschockproteins und des antigenen Moleküls aus Krebsgewebe isoliert ist.

28. Pharmazeutische Zusammensetzung nach Anspruch 26, wobei das antigene Molekül ein Tumor-spezifisches Antigen oder ein Tumor-assoziertes Antigen ist.

29. Pharmazeutische Zusammensetzung nach Anspruch 26, wobei das antigene Molekül die Antigenität eines Antigens eines infektiösen Mittels zeigt.

30. Pharmazeutische Zusammensetzung nach Anspruch 27 oder 28 zur Verwendung bei einem Verfahren zur Behandlung von Krebs in einem Krebs aufweisenden Subjekt.

31. Pharmazeutische Zusammensetzung nach Anspruch 29 zur Verwendung bei einem Verfahren zur Behandlung einer infektiösen Erkrankung in einem eine infektiöse Erkrankung aufweisenden Subjekt.

32. Pharmazeutische Zusammensetzung nach einem der Ansprüche 25, 30 oder 31, wobei das Subjekt ein Säuger ist.

33. Pharmazeutische Zusammensetzung nach Anspruch 32, wobei der Säuger ein Mensch ist.

34. Verwendung des einen oder der mehreren Komplexe gemäß Anspruch 14 oder 15 zur Herstellung eines Medikaments zur Verwendung bei der Behandlung von Krebs in einem Krebs aufweisenden Subjekt.

35. Verwendung des einen oder der mehreren Komplexe gemäß Anspruch 16 zur Herstellung eines Medikaments zur Verwendung bei der Behandlung einer infektiösen Erkrankung in einem eine infektiöse Erkrankung aufweisenden Subjekt.

36. Zusammensetzung, umfassend einen Bestand an nichtkovalenten Komplexen, wobei die nichtkovalenten Komplexe gemäß einem der Ansprüche 1 bis 13 sind.

37. Zusammensetzung nach Anspruch 36 zur Verwendung als ein Medikament.

38. Zusammensetzung nach Anspruch 37, wobei ein Komplex des Hitzeschockproteins und des antigenen Moleküls aus Krebsgewebe isoliert ist.

39. Zusammensetzung nach Anspruch 37, wobei das antigene Molekül ein Tumor-spezifisches Antigen oder ein Tumor-assoziiertes Antigen ist.

40. Pharmazeutische Zusammensetzung nach Anspruch 37, wobei das antigene Molekül die Antigenität eines Antigens eines infektiösen Mittels zeigt.

41. Zusammensetzung nach Anspruch 38 oder 39 zur Verwendung bei einem Verfahren zur Behandlung einer Art von Krebs in einem Krebs aufweisenden Subjekt.

42. Zusammensetzung nach Anspruch 40 zur Verwendung bei einem Verfahren zur Behandlung einer infektiösen Erkrankung in einem eine infektiöse Erkrankung aufweisenden Subjekt.

43. Zusammensetzung nach Anspruch 36 zur Verwendung bei einem Verfahren zur Behandlung oder Vorbeugung von Krebs oder einer infektiösen Erkrankung.

44. Verwendung einer Zusammensetzung gemäß Anspruch 38 oder 39 zur Herstellung eines Medikaments zur Verwendung bei der Behandlung von Krebs in einem Krebs aufweisenden Subjekt.

45. Verwendung der Zusammensetzung gemäß Anspruch 40 zur Herstellung eines Medikaments zur Verwendung bei der Behandlung einer infektiösen Erkrankung in einem eine infektiöse Erkrankung aufweisenden Subjekt.

46. Verwendung der Zusammensetzung gemäß Anspruch 36 zur Herstellung eines Medikaments für die Behandlung oder Vorbeugung von Krebs oder einer infektiösen Erkrankung.

47. Kit umfassend:
a) einen ersten Behälter, der eine Zusammensetzung enthält, die ein Bestand an nichtkovalenten Komplexen gemäß einem der Ansprüche 1 bis 13 umfasst,
b) einen zweiten Behälter, der ein gereinigtes Lektin enthält.

48. Kit nach Anspruch 47, wobei das antigene Molekül die Antigenität eines Antigens von einem Krebs oder die Antigenität eines Antigens eines Mittels einer infektiösen Erkrankung zeigt.

49. Kit nach Anspruch 47, wobei das Lektin ein Mannose-bindendes Lektin ist.

50. Kit nach Anspruch 47, wobei das Mannose-bindende Lektin Concanavalin A (Con A) ist.

51. Kit nach einem der Ansprüche 47 bis 50, wobei das Hitzeschockprotein ausgewählt ist aus der Gruppe, bestehend aus gp96, Calreticulin und GLP170.

52. Kit nach einem der Ansprüche 47 bis 50, wobei das Hitzeschockprotein gp96 ist.

## Revendications

1. Un ou plusieurs complexes non covalents, chaque complexe comprenant une protéine de choc thermique, une molécule antigénique et une lectine, dans lesquels ladite protéine de choc thermique est glycosylée, et dans lesquels la quantité de lectine présente dans lesdits complexes par rapport à la quantité de protéine de choc thermique est supérieure ou égale à 50 nanogrammes de lectine par microgramme de protéine de choc thermique ou inférieure ou égale à 5 nanogrammes de lectine par microgramme de protéine de choc thermique.

2. Complexes selon la revendication 1, dans lesquels la lectine présente dans lesdits complexes par rapport à la quantité de protéine de choc thermique ou de glycoprotéine est de 50 à 1 000 nanogrammes de lectine par microgramme de protéine de choc thermique ou de glycoprotéine.

3. Complexes selon la revendication 1, dans lesquels la lectine présente dans lesdits complexes par rapport à la quantité de protéine de choc thermique est de 100 à 500 nanogrammes de lectine par microgramme de protéine de choc thermique.

4. Complexes selon la revendication 1, dans lesquels la lectine présente dans lesdits complexes par rapport à la quantité de protéine de choc thermique ou de glycoprotéine est de 0,1 à 1 nanogramme de lectine par microgramme de protéine de choc thermique ou de glycoprotéine.

5. Complexes selon la revendication 1, dans lesquels la lectine présente dans lesdits complexes par rapport à la quantité de protéine de choc thermique est de 0,5 à 1 nanogramme de lectine par microgramme de protéine de choc thermique.

6. Complexes selon la revendication 1, dans lesquels le rapport molaire entre la protéine de choc thermique et la lectine est de 3 : 1, 2 : 1 ou 1 : 1.

7. Complexes selon l'une quelconque des revendications 1 à 6, dans lesquels ladite lectine se présente sous la forme d'un dimère ou d'un oligomère.

8. Complexes selon l'une quelconque des revendications 1 à 7, dans lesquels ladite lectine est une lectine liant le mannose.

9. Complexes selon la revendication 8, dans lesquels ladite lectine liant le mannose est la Concanavaline A (Con A).

10. Complexes selon l'une quelconque des revendications 1 à 9, dans lesquels ladite protéine de choc thermique est choisie dans le groupe constitué par gp96, Calréticuline et GRP170.

11. Complexes selon l'une quelconque des revendications 1 à 9, dans lesquels ladite protéine de choc thermique est gp96.

12. Complexes selon l'une quelconque des revendications 1 à 11, dans lesquels lesdits complexes sont purifiés.

13. Complexes selon l'une quelconque des revendications 1 à 12, dans lesquels ladite molécule antigénique est une molécule antigénique qui affiche un ou plusieurs déterminants antigéniques contre lesquels une réponse immunitaire est souhaitée chez un sujet.

14. Complexes selon l'une quelconque des revendications 1 à 13, dans lesquels un complexe de ladite protéine de choc thermique et de ladite molécule antigénique est isolé d'un tissu cancéreux.

15. Complexes selon l'une quelconque des revendications 1 à 13, dans lesquels ladite molécule antigénique est un antigène spécifique d'une tumeur ou un antigène associé à une tumeur.

16. Complexes selon l'une quelconque des revendications 1 à 13, dans lesquels ladite molécule antigénique affiche l'antigénicité d'un antigène d'un agent infectieux.

17. Procédé de préparation de la population d'un ou plusieurs complexes non covalents selon l'une quelconque des revendications 1 à 16, ledit procédé comprenant les étapes consistant à :
a) lier ladite lectine à ladite protéine de choc thermique ; et
b) complexer ladite protéine de choc thermique à ladite molécule antigénique.

18. Procédé de préparation de la population d'un ou plusieurs complexes non covalents selon l'une quelconque des revendications 1 à 16, ledit procédé comprenant la liaison d'une lectine à un ou plusieurs complexes, chaque complexe comprenant une protéine de choc thermique glycosylée et une molécule antigénique, dans lesquels ladite lectine n'est pas liée à une phase solide.

19. Procédé selon la revendication 18, comprenant en outre l'isolement desdits complexes de protéine de choc thermique et de molécule antigénique par chromatographie d'affinité à base de lectine avant la liaison desdits complexes aux lectines.

20. Procédé selon la revendication 18, comprenant en outre l'isolement desdits complexes de protéine de choc thermique et de molécule antigénique par chromatographie non à base de lectine avant la liaison desdits complexes à ladite lectine.

21. Procédé selon la revendication 20, dans lequel ladite chromatographie non à base de lectine est une chromatographie d'affinité à base d'anticorps.

22. Procédé selon la revendication 18, dans lequel la lectine est ajoutée pendant le processus de purification des un ou plusieurs complexes non covalents.

23. Procédé selon la revendication 19, dans lequel la lectine est ajoutée pendant le processus de purification des un ou plusieurs complexes non covalents en utilisant une colonne scFv spécifique de gp96, dans lequel la protéine de choc thermique dans les un ou plusieurs complexes non covalents est gp96.

24. Composition pharmaceutique comprenant un vecteur pharmaceutiquement acceptable et un ou plusieurs complexes non covalents selon l'une quelconque des revendications 1 à 13.

25. Composition pharmaceutique selon la revendication 24, pour utilisation dans un procédé de traitement ou de prévention d'un cancer ou d'une maladie infectieuse chez un sujet.

26. Composition pharmaceutique selon la revendication 24, pour utilisation comme médicament.

27. Composition pharmaceutique selon la revendication 26, dans laquelle un complexe de ladite protéine de choc thermique et de ladite molécule antigénique est isolé d'un tissu cancéreux.

28. Composition pharmaceutique selon la revendication 26, dans laquelle ladite molécule antigénique est un antigène spécifique d'une tumeur ou un antigène associé à une tumeur.

29. Composition pharmaceutique selon la revendication 26, dans laquelle ladite molécule antigénique affiche l'antigénicité d'un antigène d'un agent infectieux.

30. Composition pharmaceutique selon la revendication 27 ou 28, pour utilisation dans un procédé de traitement d'un cancer chez un sujet atteint d'un cancer.

31. Composition pharmaceutique selon la revendication 29, pour utilisation dans un procédé de traitement d'une maladie infectieuse chez un sujet atteint d'une maladie infectieuse.

32. Composition pharmaceutique selon l'une quelconque des revendications 25, 30 ou 31, dans laquelle le sujet est un mammifère.

33. Composition pharmaceutique selon la revendication 32, dans laquelle le mammifère est un humain.

34. Utilisation des un ou plusieurs complexes de la revendication 14 ou 15, pour la fabrication d'un médicament pour utilisation dans le traitement d'un cancer chez un sujet atteint d'un cancer.

35. Utilisation des un ou plusieurs complexes de la revendication 16, pour la fabrication d'un médicament pour utilisation dans le traitement d'une maladie infectieuse chez un sujet atteint d'une maladie infectieuse.

36. Composition comprenant une population de complexes non covalents, dans laquelle lesdits complexes non covalents sont selon l'une quelconque des revendications 1 à 13.

37. Composition selon la revendication 36, pour utilisation comme médicament.

38. Composition selon la revendication 37, dans laquelle un complexe de ladite protéine de choc thermique et de ladite molécule antigénique est isolé d'un tissu cancéreux.

39. Composition selon la revendication 37, dans laquelle ladite molécule antigénique est un antigène spécifique d'une tumeur ou un antigène associé à une tumeur.

40. Composition pharmaceutique selon la revendication 37, dans laquelle ladite molécule antigénique affiche cette antigénicité d'un antigène d'un agent infectieux.

41. Composition selon la revendication 38 ou 39, pour utilisation dans un procédé de traitement d'un type de cancer chez un sujet atteint d'un cancer.

42. Composition selon la revendication 40, pour utilisation dans un procédé de traitement d'une maladie infectieuse chez un sujet atteint d'une maladie infectieuse.

43. Composition selon la revendication 36, pour utilisation dans un procédé de traitement ou de prévention d'un cancer ou d'une maladie infectieuse.

44. Utilisation de la composition de la revendication 38 ou 39, pour la fabrication d'un médicament pour utilisation dans le traitement d'un cancer chez un sujet atteint d'un cancer.

45. Utilisation de la composition de la revendication 40, pour la fabrication d'un médicament pour utilisation dans le traitement d'une maladie infectieuse chez un sujet atteint d'une maladie infectieuse.

46. Utilisation de la composition de la revendication 36, pour la fabrication d'un médicament destiné au traitement ou à la prévention d'un cancer ou d'une maladie infectieuse.

47. Kit comprenant :
a) un premier conteneur contenant une composition comprenant une population de complexes non covalents selon l'une quelconque des revendications 1 à 13,
b) un second conteneur contenant une lectine purifiée.

48. Nécessaire selon la revendication 47, dans lequel la molécule antigénique affiche l'antigénicité d'un antigène d'un cancer ou d'un antigène d'un agent d'une maladie infectieuse.

49. Nécessaire selon la revendication 47, dans lequel la lectine est une lectine liant le mannose.

50. Nécessaire selon la revendication 47, dans lequel la lectine liant le mannose est la Concanavaline A (Con A).

51. Nécessaire selon l'une quelconque des revendications 47 à 50, dans lequel la protéine de choc thermique est choisie dans le groupe constitué par gp96, Calréticuline et GRP170.

52. Nécessaire selon l'une quelconque des revendications 47 à 50, dans lequel la protéine de choc thermique est gp96.


### SEQUENCE LISTING

<110> Antigenics, Inc.
<120> Use of Lectins to Promote Oligomerization of Glycoproteins
   and Antigenic Molecules
<130> 8449-330-228
<150> 60/450,721
   <151> 2003-02-28
<160> 6
<170> PatentIn version 3.2
<210> 1
   <211> 9
   <212> PRT
   <213> murine leukemia virus
<400> 1
   Ser Pro Ser Tyr Val Tyr His Gln Phe
   1               5
<210> 2
   <211> 9
   <212> PRT
   <213> Mus Musculus
<400> 2
   Thr Tyr Glu Ala Leu Thr Gln Lys Val
   1               5
<210> 3
   <211> 9
   <212> PRT
   <213> Homo sapiens
<400> 3
   Thr Tyr Lys Glu Leu Ile Glu Arg Ile
   1               5
<210> 4
   <211> 19
   <212> PRT
   <213> murine leukemia virus
<400> 4
   Arg Val Thr Tyr His Ser Pro Ser Tyr Val Tyr His Gln Phe Glu Arg
   1 5 10 15
   Arg Ala Lys
<210> 5
   <211> 13
   <212> PRT
   <213> Mus Musculus
<400> 5
   Glu Tyr Glu Leu Arg Lys His Asn Phe Ser Asp Thr Gly
   1 5 10
<210> 6
   <211> 13
   <212> PRT
   <213> Mus Musculus
<400> 6
   Glu Tyr Glu Leu Arg Lys Asn Asn Phe Ser Asp Thr Gly
   1 5 10